# EUROPEAN PATENT APPLICATION

(11) **EP 4 467 157 A1**
(43) Date of publication of application: **27.11.2024**
(21) Application number: 23743377.6
(22) Date of filing: 23.01.2023
(51) Int. Cl.: A61K 45/00, A61K 39/395, A61P 1/04, A61P 35/00, C07K 16/28

(54) **PHARMACEUTICAL COMPOSITION FOR TREATING OR PREVENTING AT LEAST ONE DISEASE SELECTED FROM GROUP CONSISTING OF INFLAMMATORY BOWEL DISEASE AND INTESTINAL TUMOR**

(30) Priority: 21.01.2022 JP 2022008129
(71) Applicant: Louis Pasteur Center for Medical Research, Kyoto 606-8225 (JP)
(72) Inventor: IWAKURA, Yoichiro, Kyoto-shi, Kyoto 606-8225 (JP); CHUNG, Soo-Hyun, Kyoto-shi, Kyoto 606-8225 (JP); KUBO, Masato, Kyoto-shi, Kyoto 606-8225 (JP); SUN, Haiyang, Kyoto-shi, Kyoto 606-8225 (JP); YABE, Rikio, Kyoto-shi, Kyoto 606-8225 (JP)
(74) Representative: Cabinet Laurent & Charras
(86) International application number: PCT/JP2023/001986
(87) International publication number: WO 2023/140385

(57) **Abstract**

A pharmaceutical composition for treating or preventing at least one disease selected from the group consisting of inflammatory bowel disease and intestinal tumor, the pharmaceutical composition comprising a dendritic cell immunoreceptor inhibitor or a binding inhibitor between a dendritic cell immunoreceptor and an asialo-biantennary N-glycan, and a pharmaceutically acceptable carrier.

## Description

### Technical Field

The present disclosure relates to a pharmaceutical composition for treating or preventing at least one disease selected from the group consisting of inflammatory bowel disease and intestinal tumor.

### Background Art

Inflammatory bowel disease (IBD) including Crohn's disease (CD) and ulcerative colitis (UC) is a chronic inflammatory disease of the intestinal tract that ultimately causes the development of colorectal tumors. Innate immune receptors such as C-type lectin receptors (CLRs) and Toll-like receptors (TLRs) expressed in the intestine are suggested to play important roles not only for the host defense against pathogens but also for the development of IBD by recognizing commensal microbiota and food components to induce reactive oxygen species (ROS), antimicrobial proteins (AMPs), cytokines, chemokines, and the like. Therefore, a fine balance between the commensal microbiota and mucosal immune system is needed for the homeostasis of the intestine.

In this context, the inventors previously showed that Dectin-1, a CLR that recognizes β-glucans, induces antimicrobial protein S100A8 in the intestine by recognizing β-glucans in foods and inhibits growth of Treg-inducing bacteria *Lactobacillus murinus* (*L. murinus*). Thus, blocking Dectin-1 can suppress development of colitis by expanding *L. murinus* in the intestine and increasing Treg cells. MGL1, another CLR expressed in colonic lamina propria (cLP) macrophages, also ameliorates chemical-induced colitis through induction of IL-10 by binding *Streptococcus* and *Lactobacillus* species.

Dendritic cell immunoreceptor (DCIR; gene symbol Clec4a2) is another CLR family member, and predominantly expressed in myeloid-derived innate immune cells such as dendritic cells (DCs) and osteoclasts. Different from Dectin-1, which has a hemi-immunoreceptor tyrosine-based activation motif (hemi-ITAM), DCIR has an immunoreceptor tyrosine-based inhibitory motif (ITIM) in the cytoplasmic domain to negatively control multiple signaling pathways by recruiting protein tyrosine phosphatase SHP-1.

The inventors previously reported that aged Clec4a2^{-/-} mice spontaneously develop autoimmune sialadenitis and enthesitis (see Non-Patent Literatures 1 to 3).

### Prior Art Literature

### Non-Patent Literature

Non-Patent Literature 1: Fujikado N et al. Dcir deficiency causes development of autoimmune diseases in mice due to excess expansion of dendritic cells. Nat. Med. 14, 176-180 (2008).
Non-Patent Literature 2: Maruhashi T. et al. DCIR Maintains Bone Homeostasis by Regulating IFN-g Production in T Cells. J. Immunol. 194, 5681-5691 (2015).
Non-Patent Literature 3: Kaifu, T. et al., DCIR and its ligand asialo-biantennary N-glycan regulate DC function and osteoclastogenesis. J. Exp. Med., 218 (12), e20210435 (2021).

### SUMMARY OF INVENTION

### Technical Problem

Clec4a2^{-/-} mice are highly susceptible to autoimmune diseases such as collagen-induced arthritis (CIA) and experimental autoimmune encephalomyelitis (EAE). DC population is expanded and antigen presenting ability of DC is enhanced in Clec4a2^{-/-} mice, and this enhanced antigen presenting ability is suggested to cause autoimmunity and overresponses to antigen stimulation in Clec4a2^{-/-} mice. Further, DC differentiation from Clec4a2^{-/-} mouse bone marrow cells (BMCs) by granulocyte-macrophage colony-stimulating factor (GM-CSF) is enhanced in BMCs of Clec4a2^{-/-} mice, and excessive phosphorylation of signal transducer and activation of transcription-5 (Stat-5), an intracellular signaling molecule, was observed. It is suggested that DCIR regulates the signaling downstream of GM-CSF by suppressing phosphorylation of downstream Stat-5 though recruitment of SHP-1, a dephosphorylation enzyme, to ITIM. Moreover, DCIR suppresses signals such as TLR4, 7, 8, and 9 thereby suppressing the production of inflammatory cytokines such as IL-1β and IL-23 from myeloid cells such as macrophages. As a result, it was found that, since the expression of IL-1β and IL-23 is strongly induced by components such as LPS and DNA of intestinal bacteria in DCIR deficient mice, the expression of GM-CSF is significantly enhanced by these cytokines. Thus, it seems likely that DCIR is also involved in intestinal inflammation and anti-tumor immunity in the intestine. However, the involvement of DCIR in the development of intestinal inflammation and tumor remains to be elucidated.

In light of the foregoing background, it is useful to elucidate the role of DCIR in intestinal inflammation and tumors and utilize it for the treatment or prevention of such diseases. The present disclosure relates to providing a novel pharmaceutical composition for treating or preventing at least one disease selected from the group consisting of inflammatory bowel disease and intestinal tumor.

### Solution to Problem

Means to solve the foregoing problem include the following aspects.
(1) A pharmaceutical composition for treating or preventing at least one disease selected from the group consisting of inflammatory bowel disease and intestinal tumor, the pharmaceutical composition containing a dendritic cell immunoreceptor inhibitor or a binding inhibitor between a dendritic cell immunoreceptor and an asialo-biantennary N-glycan, and a pharmaceutically acceptable carrier.
(2) The pharmaceutical composition according to (1), wherein the dendritic cell immunoreceptor inhibitor or the binding inhibitor between a dendritic cell immunoreceptor and an asialo-biantennary N-glycan is an antibody.
(3) The pharmaceutical composition according to (1) or (2), wherein the binding inhibitor between a dendritic cell immunoreceptor and an asialo-biantennary N-glycan is an anti-asialo-biantennary N-glycan antibody.
(4) The pharmaceutical composition according to (1) or (2), wherein the dendritic cell immunoreceptor inhibitor or the binding inhibitor between a dendritic cell immunoreceptor and an asialo-biantennary N-glycan is an anti-dendritic cell immunoreceptor antibody.
(5) The pharmaceutical composition according to (2), wherein the antibody is a mouse antibody, a chimeric antibody, a humanized antibody, or a human antibody.
(6) The pharmaceutical composition according to (2), wherein the antibody is a humanized antibody.
(7) The pharmaceutical composition according to (2), wherein the antibody is a full-length antibody, or a low-molecular antibody selected from the group consisting of Fab, Fab', F (ab')2, scFv, and diabody.
(8) The pharmaceutical composition according to any one of (1) to (7), wherein the intestinal tumor includes at least one selected from the group consisting of colorectal cancer, rectal cancer, and colon cancer.
(9) The pharmaceutical composition according to any one of (1) to (8), wherein the inflammatory bowel disease includes at least one selected from the group consisting of Crohn's disease and ulcerative colitis.

### Advantageous Effects of Invention

According to the present disclosure, a novel pharmaceutical composition for treating or preventing at least one disease selected from the group consisting of inflammatory bowel disease and intestinal tumor is provided.

### BRIEF DESCRIPTION OF DRAWINGS

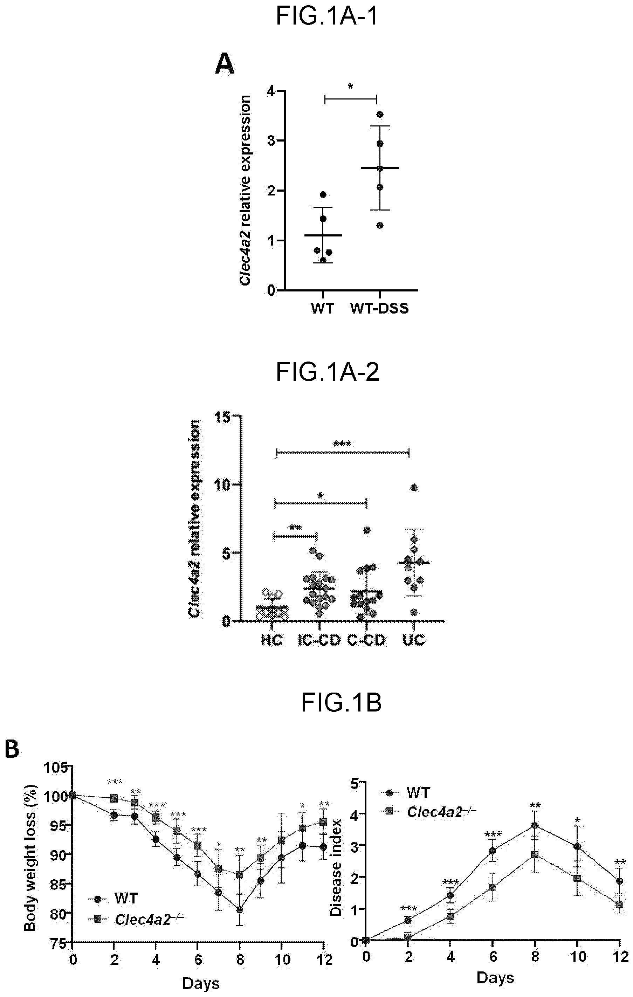

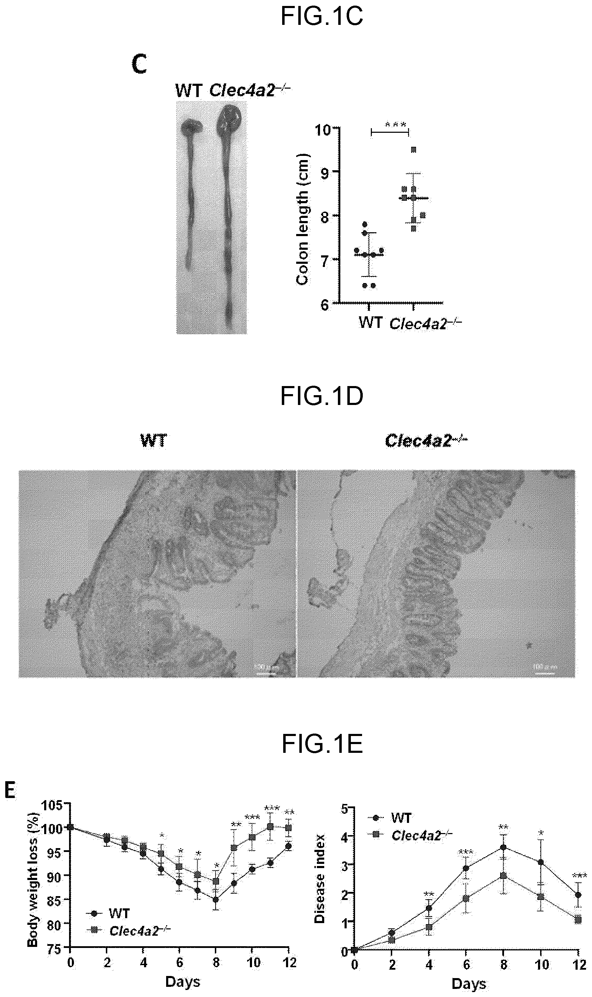

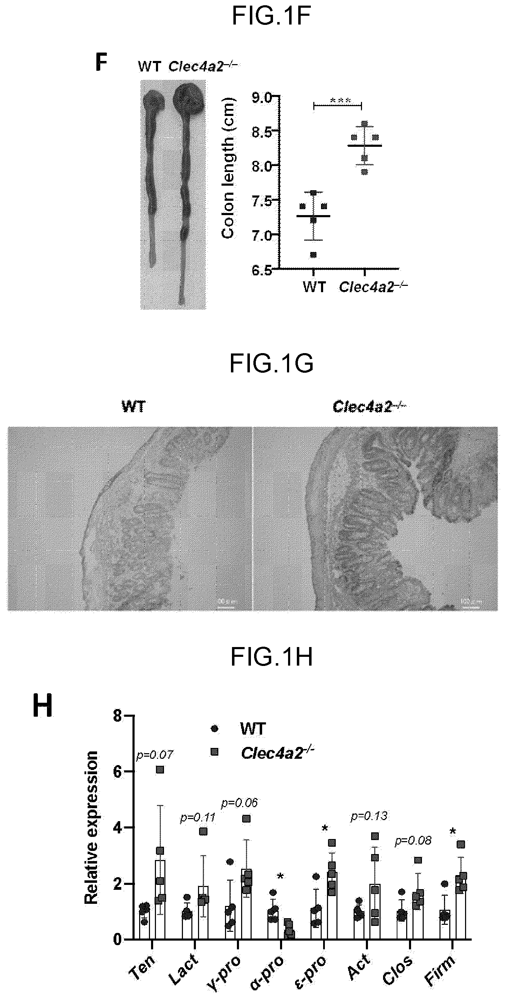

FIG. 1A-1: FIG. 1A-1 to FIG. 1I are figures showing that Clec4a gene expression is upregulated topically in the inflammatory bowel and that Clec4a2^{-/-} mice are refractory against DSS-induced colitis independently of intestinal commensal microbiota. Data in FIGs. 1B to D are representatives of four independent experiments, and data in FIGs. 1E to G are representatives of two independent experiments. Data in FIGs. 1B, C, E, and F are presented as means ± SD. *P < 0.05; **P < 0.05 ***P<0.001. (FIG. A-1) Wild type (WT) mice were fed with 4% dextran sulfate sodium (DSS)-containing drinking water for 7 days, followed by normal drinking water for another 5 days. On day 12, total RNA was extracted from the colon, and Clec4a2 expression intensity was examined by real-time PCR (n = 5/group).
FIG.1A-2: FIG.1A-2 shows expression of human DCIR gene at an affected site of Crohn's disease (colon type (C-CD): n = 15, rectal type (IC-CD): n = 18) and ulcerative colitis (UC, n = 10), compared to healthy people (HC, n = 11), using the data (GSE 10616) of human colitis gene expression in the NCBI GEO database.
FIG. 1B: (FIG. 1B to FIG. 1D) WT and Clec4a2^{-/-} mice were raised in separate cages and were administrated with 4% DSS for 7 days and with normal water for another 5 days (n = 8/group). (FIG. 1B) Body weight was measured every day, and disease severity index was evaluated once every two days.
FIG. 1C: On day 12 after the start of the DSS treatment, mice were sacrificed and examined for gross pathology of the colon, and the length of the colon was measured.
FIG. 1D: On day 12 after the start of the DSS treatment, mice were sacrificed, colon tissue was harvested, and histology of the distal colon was analyzed (H&E staining, 40x).
FIG. 1E: (FIGs. 1E to I) After weaning, WT and Clec4a2^{-/-} mice were first co-housed for 4 weeks, administrated with 4% DSS for 7 days followed by normal water for another 5 days in a same cage (n = 5/group). (FIG. 1E) Body weight was measured every day, and disease severity index was evaluated once every two days.
FIG. 1F: On day 12 after the start of the DSS treatment, mice were sacrificed and examined for gross pathology of the colon, and the length of the colon was measured.
FIG. 1G: On day 12 after the start of the DSS treatment, mice were sacrificed and histology of the distal colon was analyzed (H&E staining, 40x).
FIG. 1H: Nucleic acid was extracted from feces of the mice before co-housing, and the composition of the representative bacterial species of the intestinal microbiota was examined by real-time PCR of 16S rRNA sequences (n = 5/group).
FIG. 1I: After four weeks of co-housing, nucleic acid was extracted from the feces of the mice, and the composition of the representative bacterial species of the intestinal microbiota was examined by real-time PCR of 16S rRNA sequences (n = 5/group).

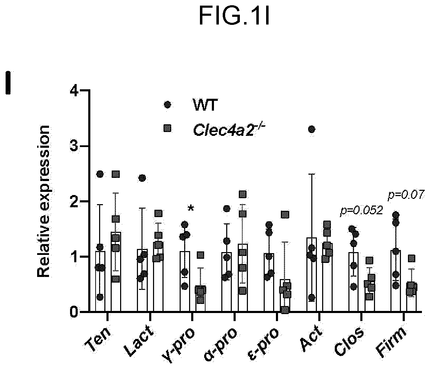

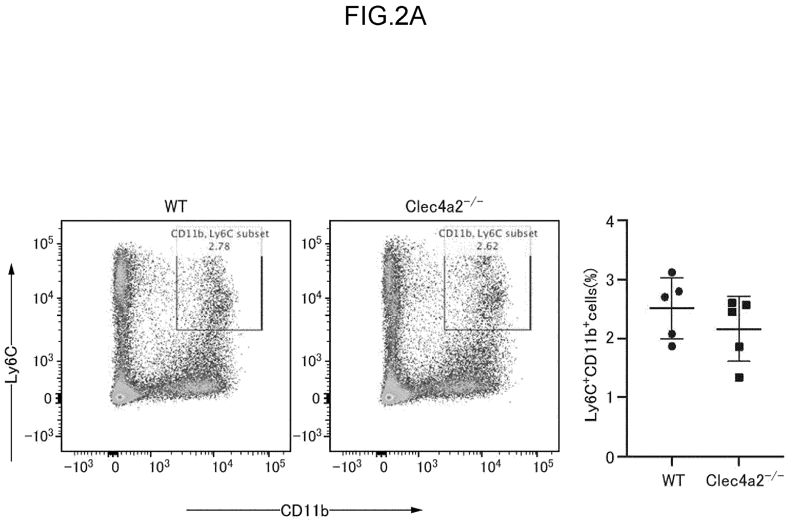

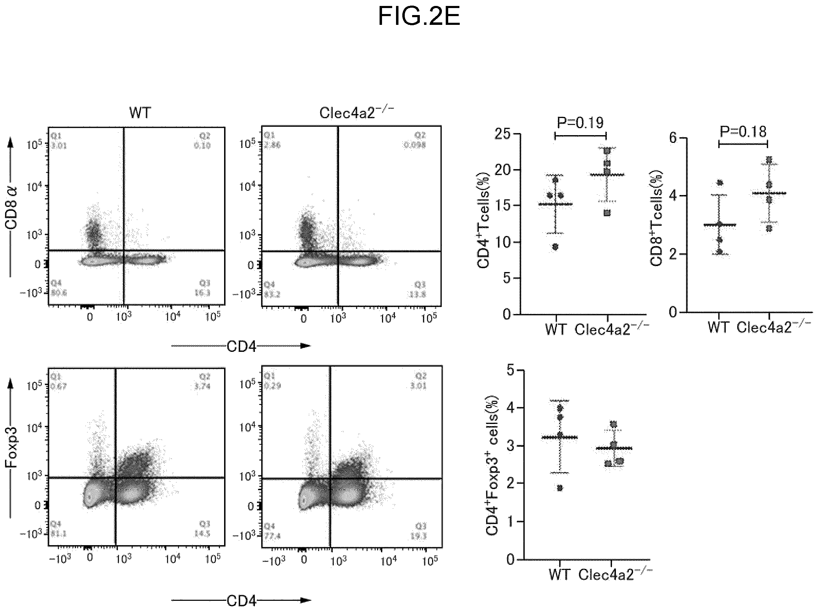

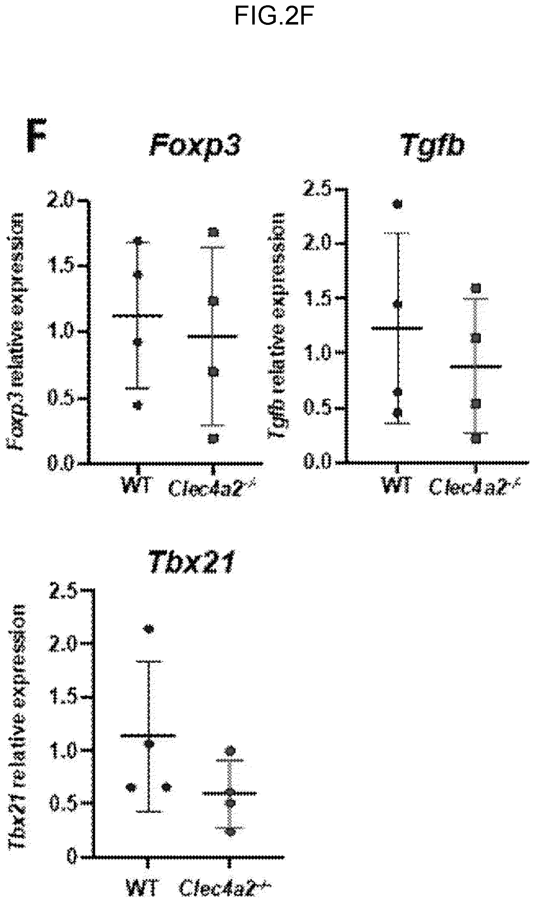

FIG. 2A: FIGs. 2A to F are figures showing that infiltration of monocytes and neutrophils, but not Treg cells, are reduced in colonic lamina propria (cLP) of Clec4a2^{-/-} mice after induction of DSS-induced colitis. Data in FIGs. 2A to D are representatives of three independent experiments, and data in FIGs. 2E and F are representatives of two independent experiments. Data are presented as means ± SD. **P < 0.01. (FIG. 2A) As described in FIG. 1, separately-housed WT and Clec4a2^{-/-} mice were sacrificed on day 12 without DSS treatment (n = 5/group), and the proportion of Ly6C⁺CD11b⁺ monocytes in CD45⁺ cells of the cLP was determined by flow cytometry.
FIG. 2B: As described in FIG. 1, separately-housed WT and Clec4a2^{-/-} mice were sacrificed on day 12 without DSS treatment (n = 5/group), and the proportion of Ly6G⁺CD11b⁺ neutrophils in CD45⁺ cells of the cLP was determined by flow cytometry.
FIG. 2C: As described in FIG. 1, separately-housed WT and Clec4a2^{-/-} mice were sacrificed on day 12 with DSS treatment (n = 4/group), and the proportion of Ly6C⁺CD11b⁺ monocytes in CD45⁺ cells of the cLP was determined by flow cytometry.
FIG. 2D: As described in FIG. 1, separately-housed WT and Clec4a2^{-/-} mice were sacrificed on day 12 with DSS treatment (n = 4/group), and the proportion of Ly6C⁺CD11b⁺ neutrophils in CD45⁺ cells of the cLP was determined by flow cytometry.
FIG. 2E: As described in FIG. 1, separately-housed WT and Clec4a2^{-/-} mice were sacrificed on day 12 with DSS treatment (n = 4/group), and the proportions of CD4⁺ and CD8⁺ T cells and CD4⁺Fox3⁺ Treg cells in CD45⁺ cells of the cLP were determined by flow cytometry.
FIG. 2F: The colon was removed from these mice on day 12 after DSS treatment (n = 4/group), and the expression of Foxp3, Tgfb and Tbx21 genes was examined by real-time qPCR.
FIG. 3A: FIGs. 3A to L are figures showing that the expression of the genes of inflammatory cytokines and chemokines such as Il17a and Cxcl2 is reduced in Clec4a2^{-/-} mice after the induction of DSS-induced colitis. FIGs. 3C to H are representatives of three independent experiments, and FIGs. 3K and L are representatives of two independent experiments. Data in FIGs. 3C to Hand L are presented as means ± SD. *P<0.05, ***P<0.001. (FIG. 3A) Gene expression in the colon was analyzed by RNA-seq in untreated or DSS-treated WT and Clec4a2^{-/-} mice. The expression levels are shown as a heatmap.
FIG. 3B: FIG.3B shows the result of pathway analysis in the transcripts whose expression was changed after the DSS treatment in FIG. 3A. The analysis was performed by pooling n = 5 mice samples for each group. (FIGs. 3C to J) Total RNA was extracted from the colon of separately housed WT and Clec4a2^{-/-} mice on day 12 after the start of the DSS treatment, and the expression of each gene was analyzed by real-time PCR.
FIG. 3C: FIG. 3C shows the expression of Tnfa gene in WT and Clec4a2^{-/-} mice after DSS treatment (n = 4).
FIG. 3D: FIG. 3D shows the expression of Il1b in WT and Clec4a2^{-/-} mice after DSS treatment (n = 4).
FIG. 3E: FIG. 3E shows the expression of Il6 in WT and Clec4a2^{-/-} mice after the DSS treatment (n = 4).
FIG. 3F: FIG. 3F shows the expression of Il17a in WT and Clec4a2^{-/-} mice after the DSS treatment (n = 8).
FIG. 3G: FIG. 3G shows the expression of Il17f in WT and Clec4a2^{-/-} mice after the DSS treatment (n = 4).
FIG. 3H: FIG. 3H shows the expression of Cxcl2 in WT and Clec4a2^{-/-} mice after the DSS treatment (n = 8).
FIG. 3I: The Cxcl2 expression in the colon was compared between untreated WT and Clec4a2^{-/-} mice (n = 5).
FIG. 3J: The Cxcl2 expression in the colon was compared between untreated and DSS-treated WT mice (n = 5).
FIG. 3K: cLP cells harvested from separately housed WT and Clec4a2^{-/-} mice on day 12 after the DSS treatment were stimulated with PMA + Ionomycin, and the proportions of IL-17- and IFN-γ-producing CD4⁺ or CD8⁺ T cells were analyzed by flow cytometry (n = 5/group).
FIG. 3L: cLP cells harvested from separately housed WT and Clec4a2^{-/-} mice on day 12 after the DSS treatment were stimulated with PMA + Ionomycin, and the proportions of IL-17- and IFN-γ-producing CD4⁺ T cells, CD8⁺ T cells, and γδT cells were analyzed by flow cytometry (n = 5/group).
FIG. 4A: FIGs. 4A to J are figures showing that the GM-SCF-Stat5 axis is activated in Clec4a^{-/-} mice after DSS treatment. (FIG. 4A) The expression of Csf2 gene was examined by real-time PCR after treating WT and Clec4a2^{-/-} mice with DSS.
FIG. 4B: The expression of Csf2 gene was examined by real-time PCR after treating or not treating WT mice with DSS.
FIG. 4C: The expression of Csf2 mRNA was examined by real-time PCR in DSS-untreated WT or Clec4a2^{-/-} mice.
FIG. 4D: The proportions of GATA⁺ ILC2 cells and RORγt⁺ ILC3 cells in cLP CD45⁺CD90.2⁺CD3⁻CD19⁻CD11b⁻Gr1⁺ cells were analyzed by flow cytometry after DSS treatment on day 12 (n = 5/group).
FIG. 4E: The proportion of GM-CSF⁺ in RORγt⁺ ILC3 cells in cLP CD45⁺CD90.2⁺CD3⁻CD19⁻CD11b⁻Gr1⁺ cells was analyzed by flow cytometry after DSS treatment on day 12 (n = 5/group).
FIG. 4F: CD11b⁺ cells were sorted by MACS from the colon the DSS-untreated mice or DSS-treated mice on day 7 and 12. 5 ×10⁵ cells were stimulated with TLR8 (R848, 100 ng/ml) and TLR9 agonist (CpG-ODN, 100 ng/ml) for 4 hours, and the expression of Il1b was examined by real-time PCR (n = 3/group).
FIG. 4G: Total protein was extracted from the colon of WT and Clec4a2^{-/-} mice, and the expression of phosphorylated Stat5 (pStat5) (90 kDa) and total StatS was examined by western blot analysis (n = 3/group)
FIG. 4H: FIG. 4H shows the ratio of pStat5/Stat5 in FIG. 4G.
FIG. 4I: The proportions of M2 macrophages (CD206⁺CD11b⁺) and M1 macrophages (Nos2⁺CD11b⁺) in CD11b⁺ cells of cLP after DSS treatment were analyzed by flow cytometry (WT: n = 3, Clec4a2^{-/-:} n = 3).
FIG. 4J: Cells (1 × 10⁶) from the colon of WT and Clec4a2^{-/-} mice were treated with rmGM-CSF (0, 1, or 10 ng/ml) for 1 hour, and then protein was extracted and the expression of pStatS and total StatS was analyzed by western blot analysis.
FIG. 5A: FIGs. 5A to I are figures showing that GM-CSF suppresses the development of DSS-induced colitis. Data are presented as means ± SD. *P<0.05, **P < 0.01, ***P<0.001. (FIGs. 5A to C) WT mice were treated with 4% DSS for 7 days, followed by normal water for 5 days. PBS or rmGM-CSF (1 mg/mouse) was administrated intraperitoneally every day until day 6 during the DSS treatment. (FIG. 5A) Body weight was measured every day.
FIG. 5B: On day 12 after the start of the DSS treatment, mice were sacrificed and examined for gross pathology of the colon, and the length of the colon was measured (n = 5/group).
FIG. 5C: Total RNA was extracted from the colon of the PBS or rmGM-CSF-treated mice on day 12 after the start of the DSS treatment, and the expression of Cxcl2, Tnf, Il17a, Il1b, Il6, Stat5a, Stat5b (n = 5), Occludin, Zo1, and Claudin (n = 4) was examined by real-time PCR.
FIG. 5D: Cells (5 × 10⁵) from the colon of WT and Clec4a2^{-/-} mice were treated with rmGM-CSF (10 ng/ml) for 12 hours, or untreated, and the expression of Occludin, Zo1 and Claudin was examined by real-time PCR (n = 4/group).
FIG. 5E: (FIGs. 5E to H) WT and Clec4a2^{-/-} mice were treated with 4% DSS for 7 days, followed by normal water for 5 days. An isotype antibody or an anti-GM-CSF neutralizing antibody (100 µg) (Bio X Cell, U.S., Clone: MP1-22E9) was administrated intraperitoneally at days -1, 1, 3, and 5 during the DSS treatment. Body weight and survival of the WT and Clec4a2^{-/-}mice were examined every day (n = 5/group). FIG. 5E shows the body weight.
FIG. 5F: FIG. 5F shows the survival.
FIG. 5G: On day 12 after the start of the DSS treatment, mice were sacrificed and examined for gross pathology of the colon, and the length of the colon was measured.
FIG. 5H: Total RNA was extracted from the colon of the WT mice treated with the isotype IgG or the anti-GM-CSF antibody on day 12 after the start of the DSS treatment, and the expression of Il17a, Il6, Tnf, Il1b, Ifnb, StatS, Il1a, Il10, and Cxcl2 (n = 4/group) was examined by real-time PCR.
FIG. 5I: Total RNA was extracted from the colon of the WT mice treated with the isotype IgG or the anti-GM-CSF antibody on day 12 after the start of the DSS treatment, and the expression of Zol1, Occludin, and Claudin (n = 4/group) was examined by real-time qPCR.
FIG. 6A: FIG. 6A is a figure showing that the colorectal tumor development in AOM-DSS mouse model is suppressed in Clec4a2^{-/-} mice in a commensal bacterium-independent manner. Data in FIGs. 6A to H are representatives of four independent experiments. Data in FIGs. 6A, B, D, E, F, and H are presented as means ± SD. *P < 0.05; **P < 0.01 ***P<0.001. (FIGs. 6A to H) WT and Clec4a2^{-/-} mice were raised separately and were subjected to AOM-DSS mouse model. (FIG. 6A) Body weight was measured every three days. The measured body weight is shown (WT: n = 6, Clec4a2^{-/-:} n = 8).
FIG. 6B: FIG. 6B shows the survival (WT: n = 6, Clec4a2^{-/-}: n = 8).
FIG. 6C: FIG. 6C shows images of the colons (WT: n = 3, Clec4a2^{-/-}: n = 5).
FIG. 6D: FIG. 6D shows the size and the number of the colon tumors (WT: n = 3, Clec4a2^{-/-}: n = 5).
FIG. 6E: (FIGs. 6E to H) WT and Clec4a2^{-/-} mice were co-housed for 4 weeks and were subject to AOM-DSS mouse model. (FIG. 6E) Body weight was measured every three days. FIG. 6E shows the measured body weight (WT: n = 5 Clec4a2-1-: n = 5).
FIG. 6F: FIG. 6F shows the survival (WT: n = 5, Clec4a2^{-/-}: n = 5).
FIG. 6G: FIG. 6G shows images of the colons (WT: n = 3, Clec4a2^{-/-}: n = 5).
FIG. 6H: FIG. 6H shows the number of colon tumors (WT: n = 3, Clec4a2^{-/-}: n = 5).

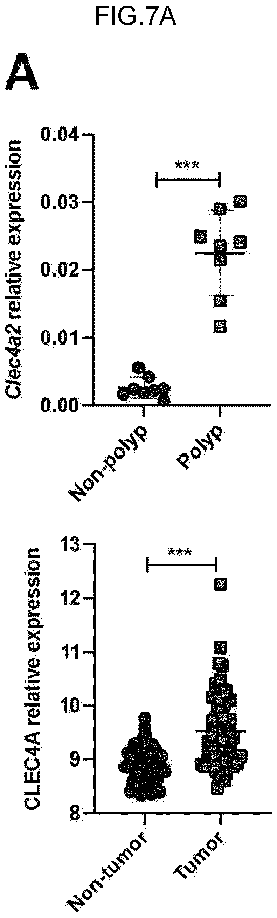

FIG. 7A: FIGs. 7A to J show the expression of Clec4a2 and cytokines in polyps and non-polyp portions, and the abundance of MDSC. Data are representatives of three independent experiments, and are presented as means ± SD. *P<0.05, **P < 0.01, ***P < 0.001. Total RNA was extracted from polyps and non-polyp portions of the colon harvested from separately housed WT and Clec4a2^{-/-} mice after AOM-DSS treatment. (FIG. 7A) The expression of Clec4a2 in the colon polyp portions and non-polyp portions of WT mice (n = 8/group) is shown in the upper figure, and the expression of CLEC4A at tumor and non-tumor sites from colon cancer patients obtained from public database (GSE20842, n = 65) is shown in the lower figure.
FIG. 7B: The expression of Il17a in polyps (p) and non-polyp portions (np) of WT and Clec4a2^{-/-} mouse colon was examined by real-time PCR (n = 8/group).
FIG. 7C: The expression of Il17f in polyps (p) and non-polyp portions (np) of WT and Clec4a2^{-/-} mouse colon was examined by real-time PCR (n = 8/group).
FIG. 7D: The expression of Cxcl2 in polyps (p) and non-polyp portions (np) of WT and Clec4a2^{-/-} mouse colon was examined by real-time PCR (n = 8/group).
FIG. 7E: The gene expression levels analyzed by RNA-seq in polyps and non-polyp portions of WT or Clec4a2^{-/-} mouse colons are shown as a heatmap (n = 5/group, 5 samples were pooled in each group).
FIG. 7F: The abundances of monocyte (CD11b⁺Ly6C⁺) and granulocyte (CD11b⁺Ly6G⁺) myeloid-derived suppressor cells (MDSCs) in polyps and non-polyp portions from WT and Clec4a2^{-/-} mice after AOM-DSS treatment were measured, and are shown in the graphs (WT: n = 3, Clec4a2^{-/-:} n = 5).
FIG. 7G: The abundances of monocyte (CD11⁺Ly6C⁺) and granulocyte (CD11⁺Ly6G⁺) myeloid-derived suppressor cells (MDSCs) in polyps (p) and non-polyp portions (np) of WT and Clec4a2^{-/-} mice after AOM-DSS treatment were measured by flow cytometry, and are shown by dot blot (WT: n = 3, Clec4a2^{-/-}: n = 5).
FIG. 7H: The expression intensity of Csf2 gene in colon polyps (p) and non-polyp portions (np) of the WT and Clec4a2^{-/-} mice was examined by real-time PCR (WT: n = 3, Clec4a2^{-/-}: n = 4).
FIG. 7I: The mRNA expression of Stat5a and Stat5b in colon non-polyp (np) and polyp (p) portions of the WT and Clec4a2^{-/-} mice was examined by real-time PCR (WT: n = 3, Clec4a2^{-/-}: n = 4).
FIG. 7J: Protein from non-polyp and polyp portions of WT and Clec4a2^{-/-} mice was extracted, and pStatSa and total Stat5 (90 kDa) protein expression was determined by western blot analysis.
FIG. 8A: FIGs. 8A to F are figures showing that an anti-NA2 antibody administration ameliorates the development of colitis. Data are presented as means ± SD. *P<0.05, **P < 0.01. (FIGs. 8A to E) WT mice were treated with 4% DSS for 7 days, followed by normal water for 4 days. An anti-NA2 antibody (IgM, 200 µg /mouse) and an isotype immunoglobulin (IgM, 200 µg /mouse) were administered intraperitoneally to WT mice every 3 days (days -1, 2, and 5) during the DSS treatment (n = 5/group). Fig 8A shows changes in the body weight.
FIG. 8B: FIG. 8B shows disease severity.
FIG. 8C: On day 11 after the start of the DSS treatment, mice were sacrificed and examined for gross pathology of the colon, and the length of the colon was measured.
FIG. 8D: On day 11 after the start of the DSS treatment, total RNA was prepared from the colon of the mice treated with an anti-NA2 antibody and an isotype immunoglobulin, and the expression of Cxcl2, Il17a, Il6, Csf2, StatS and Il1b was examined by real-time PCR (n = 5/group).
FIG. 8E: On day 11 after the start of the DSS treatment, the proportion of Ly6G⁺CD11b⁺ neutrophils in CD45⁺ cells from cLP was examined by flow cytometry (n = 5/group).
FIG. 8F: WT mice were administrated with AOM-DSS and an anti-NA2 antibody or an isotype. The number of colon tumors in each size is shown.

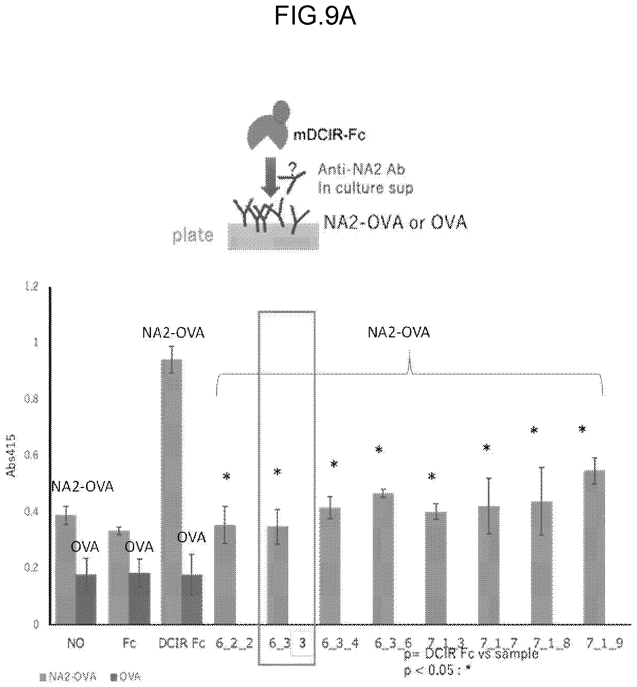

FIG. 9A: FIG. 9A shows data indicating that an anti-NA2 antibody inhibits the binding between DCIR and NA2. 6_2_2, 6_3_3, and the like are the names of different anti-NA2 monoclonal antibody clones. In wells to which only DCIR-Fc is added, binding to NA2-OVA is observed, whereas this binding significantly decreases in wells to which an anti-NA2 antibody is added. This suggests that the anti-NA2 antibody inhibits the binding between DCIR and NA2. In the Examples, 633_1G3 used for the treatment of colitis and intestinal tumor was cloned again from a 6_3_3 clone. It has been confirmed that the VDJ sequences of the 633_1G3 clone and the 6_3_3 clone match.
FIG. 9B: FIG. 9B is a result of ELISA indicating that the anti-NA2 antibody (633_1G3) binds to NA2.
FIG. 9C: FIG. 9C shows that the anti-NA2 antibody (633_1G3) is selectively associated with a cell line that express asialoglycans out of N-glycans. As compared to wild type CHO cells (predominantly expressing glycans having sialic acid at the non-reduction terminal), Led cells (high amount of mannose at the terminal), and Lec8 (agalactoglycan), the 633_1G3 antibody strongly bound to Lec2 cells (asialoglycan).
FIG. 10: FIG. 10 shows an example of the amino acid sequence and the DNA sequence of the heavy chain variable region and the light chain variable region of an anti-NA2 antibody.

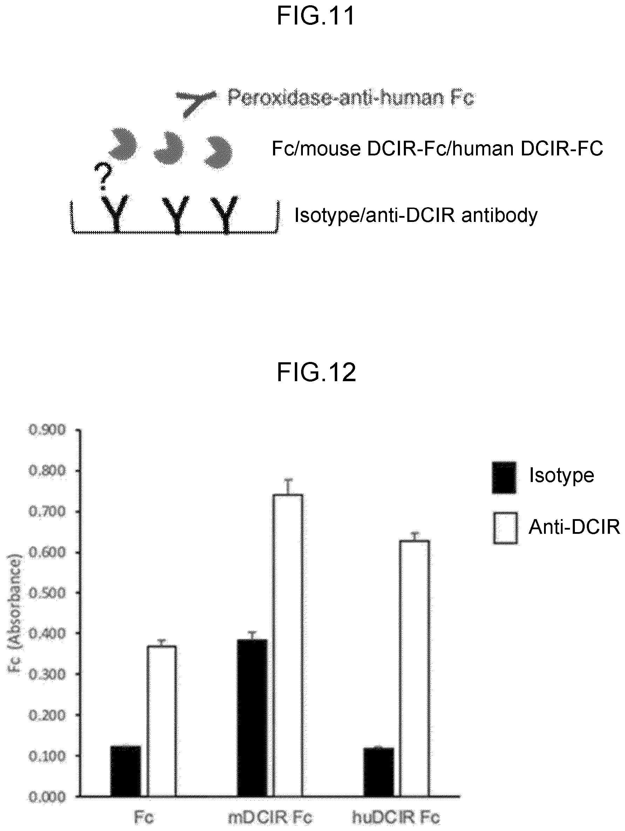

FIG. 11: FIG. 11 shows a schematic diagram of a binding test of an anti-DCIR antibody to mouse and human DCIRs. An isotype or an anti-DCIR antibody is added to a plate and allowed to stand at 4°C overnight to bind to the plate. The plate is blocked with 10% (w/v) FCS-containing PBS, and Fc, mouse DCIR-Fc, or human DCIR-Fc (5 µg/ml) is added. After allowing the plate to stand for 1 hour at room temperature and washing the plate, the Fc or DCIR-Fc associated with the plate is detected by Peroxidase-conjugated anti-human Fc (Jackson Immunoresearch Inc.). Since the detected Fc protein is one that is bound by the antibody used for the initial plate coating, the binding activity of the anti-DCIR antibody to mouse and human DCIRs can eventually be evaluated.
FIG. 12: FIG. 12 shows the binding activity of the anti-DCIR antibody against the mouse and human DCIRs. In the method of FIG. 11, the anti-DCIR antibody showed a binding activity to mouse DCIR-Fc and human DCIR-Fc.

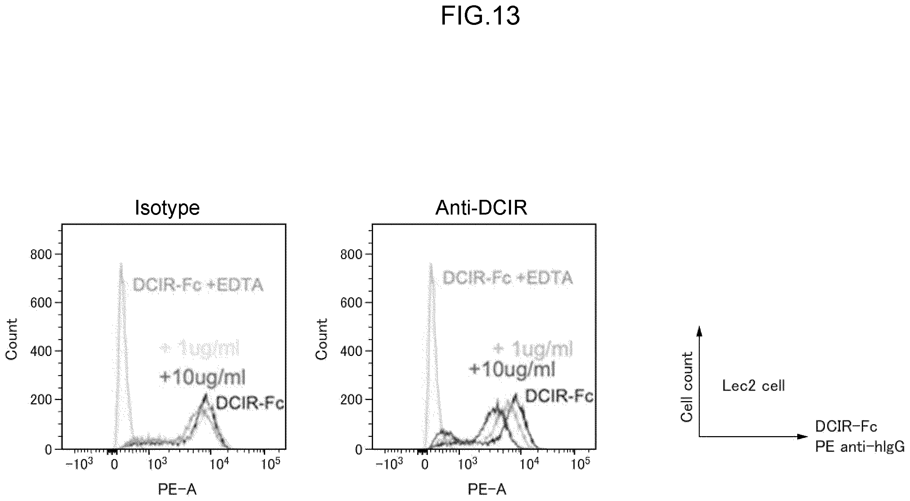

FIG. 13: FIG. 13 shows an activity of the anti-DCIR antibody to inhibit the binding between NA2-expressing cells (Lec2) and DCIR. DCIR-Fc and an isotype or an anti-DCIR antibody were added to Lec2 cells (asialoglycan), and the effect on the binding of DCIR-Fc to Lec2 cells was evaluated. The binding of DCIR-Fc to Lec2 cells was not changed by the addition of the isotype antibody, whereas the addition of the anti-DCIR antibody decreased the binding of DCIR-Fc to Lec2 cells in a concentration-dependent manner. This indicates that the anti-DCIR antibody exhibits an activity to inhibit the binding between NA2-expressing cells (Lec2) and DCIR.
FIG. 14A: FIG. 14A shows an example of the base sequence of an anti-DCIR antagonist antibody.
FIG. 14B: FIG. 14B shows an example of the amino acid sequence of an anti-DCIR antagonist antibody.

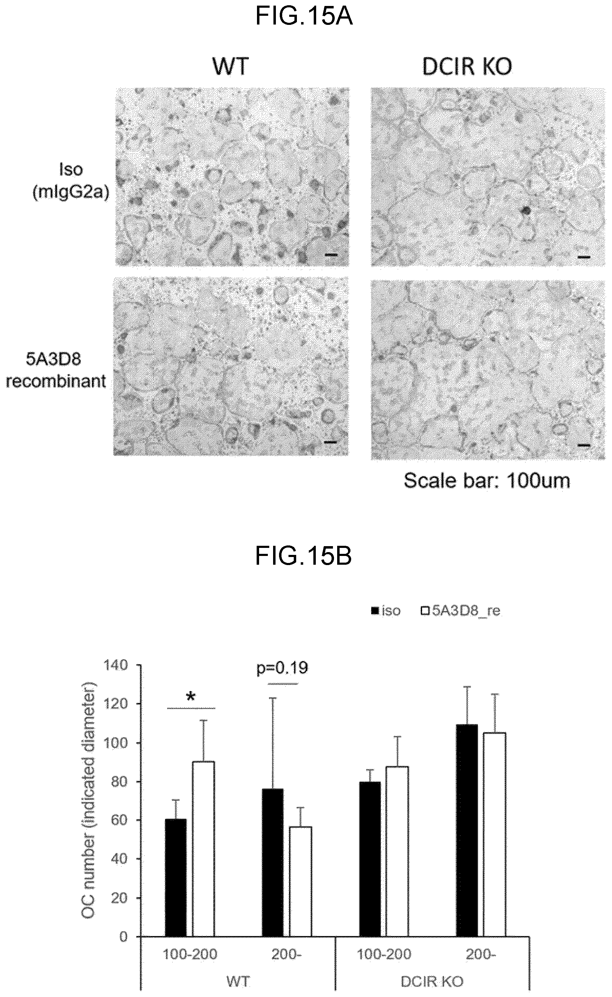

FIG. 15A: Wild type or DCIR-deficient mouse bone marrow cells were cultured with M-CSF (20 ng/mL) for two days, followed by M-CSF (20 ng/mL) and RANKL (100 ng/mL) for four days. An isotype or a recombinant anti-DCIR antagonist antibody (5A3D8_recombinant) is added at 1 µg/mL at the beginning of the culturing and at the time of changing the culture medium. Polynuclear cells that are TRAP positive after TRAP staining and that have a cell diameter of from 100 to 200 µm and 200 µm or more are counted as osteoclasts. FIG. 15A is a microscopic image after the TRAP staining of the osteoclasts of the wild type and DCIR-deficient mice after adding the isotype or the anti-DCIR antibody.
FIG. 15B: FIG. 15B shows the number of osteoclasts having the indicated cell diameter (µm) in the wild type and DCIR-deficient mice. The graph is shown by the mean and standard deviation of the number of osteoclasts in four wells, and student's t-test was performed. *; p < 0.05, iso VS anti-DCIR antibody.

### DESCRIPTION OF EMBODIMENTS

Hereinafter, embodiments of the present disclosure will be described in detail. However, the embodiments of the present disclosure are not limited to the following embodiments. In the following embodiments, constituent elements (including element steps and the like) are not essential unless otherwise specified. The same applies to numerical values and ranges thereof, and the embodiments of present disclosure are not limited thereto.

In the present disclosure, a numerical range indicated using "to" includes numerical values described before and after "to" as a lower limit value and an upper limit value, respectively.

In the numerical ranges described in a stepwise manner in the present disclosure, an upper limit value or a lower limit value of one numerical range may be replaced with an upper limit value or a lower limit value of another numerical range described in a stepwise manner. Further, in the numerical ranges described in the present disclosure, an upper limit value or a lower limit value in a numerical range may be replaced with another value shown in the Examples.

In the present disclosure, each component may include plural corresponding substances. In a case in which plural substances corresponding to a component are present in a composition, the content or amount of the component refers to the total content or amount of the plural substances present in the composition, unless otherwise specified.

In the present disclosure, even in a case in which an element is described in a singular form, it does not exclude the presence of plural substances as long as there is no technical contradiction, unless otherwise specified.

In the present disclosure, "treatment" of a disease encompasses, for example, eliminating or improving a disease or a symptom associated with a disease. Further, "prevention" of a disease encompasses, for example, preventing the expression of a disease or a symptom associated with a disease, delaying the expression of a disease or a symptom associated with a disease, and alleviating a disease or a symptom associated with a disease before the expression thereof.

### <Pharmaceutical Composition>

In the present study, the inventors have found that the development and progression of inflammatory bowel disease and intestinal tumor are promoted by the action of dendritic cell immune receptor (DCIR). Therefore, a pharmaceutical composition for treating or preventing these diseases, containing a binding inhibitor between a dendritic cell immunoreceptor (DCIR) and an asialo-biantennary N-glycan (NA2), or a DCIR inhibitor, and a pharmaceutically acceptable carrier, is proposed.

DCIR (gene symbol: Clec4a2) is a member of myeloid C-type lectin receptors containing ITIM in its cytoplasmic domain. The inventors' study group previously reported that DCIR recognizes asialo-biantennary N-glycan (NA2) to suppress differentiation and activation of dendritic cells (Kaifu et al., J. Exp. Med., 218(12), e20210435, 2021). Inventors found that blockade of DCIR signaling enhances the GM-CSF-Stat5 signaling pathway to improve diseases such as inflammatory bowel disease and intestinal tumor.

The inventors examined the roles of DCIR in the development of dextran sodium sulfate (DSS)-induced colitis and azoxymethane (AOM)-induced colon tumor in mice. DSS-induced colitis is an animal disease model for ulcerative colitis and Crohn's disease in humans. AOM-DSS-induced tumor is a model for colorectal cancer, in which polyps are induced in the colon by inducing mutations in protooncogenes such as the K-ras and β-Catenin genes, and in which tumorigenesis is promoted by DSS-induced inflammation. As a result, it was found that Clec4a2^{-/-} mice are less susceptible to DSS-induced colitis compared with wild type (WT) mice. Further, development of AOM-DSS-induced intestinal tumors, which is a colorectal cancer model, was also reduced in Clec4a2^{-/-} mice. Since the phenotype of Clec4a2^{-/-} mice was mild even under the same condition with WT mice, the phenotype of Clec4a2^{-/-} mice was thought to be independent of intestinal commensal microbiota. The expression of GM-CSF and its downstream StatS phosphorylation were enhanced in the Clec4a2^{-/-} mouse colon, while the expression of 1117 and Cxcl2 was suppressed, associated with reduced infiltration of neutrophils and myeloid-derived suppressor cells. Administration of recombinant GM-CSF to mice efficiently suppressed Il17 expression and ameliorated DSS-induced colitis. On the other hand, administration of anti-GM-CSF antibody significantly exacerbated the symptoms. Furthermore, an anti-NA2 antibody against NA2, a ligand for DCIR, suppressed development of colitis and colorectal tumors by inhibiting the action of NA2 on DCIR. These observations indicate that the function of DCIR exacerbates diseases such as colitis and colorectal tumor, and that a DCIR-NA2 binding inhibitor is useful for treating or preventing the development of these diseases caused by DCIR. Further, it is suggested that a DCIR inhibitor that inactivates DCIR is useful for treating or preventing these diseases.

The inventors have found that DCIR-NA2 axis plays a role to regulate immune responses induced by innate immune receptors such as TLR8 and TLR9 in the intestine. Under physiological conditions, DCIR regulates excess differentiation and activation of DCs to prevent autoimmunity. However, in the intestine, DCIR signaling regulates innate immune signaling and suppresses induction of GM-CSF, which makes intestine vulnerable to inflammation and tumor development. Thus, inhibition of DCIR activation can suppress inflammation and tumor development in the intestine through innate immune mechanisms.

The DCIR-NA2 binding inhibitor is a substance that inhibits binding between DCIR and NA2 (including reducing binding). The DCIR inhibitor is a substance that inactivates DCIR. In one aspect, the DCIR-NA2 binding inhibitor or the DCIR inhibitor may be an antibody. In one aspect, the DCIR-NA2 binding inhibitor may be an anti-NA2 antibody. In one aspect, the DCIR-NA2 binding inhibitor or the DCIR inhibitor may be an anti-DCIR antibody.

For the DCIR-NA2 binding inhibitor, the inhibition of the binding between DCIR and NA2 can be confirmed by a method according to the method described in detail in the sections of the anti-NA2 antibody and the anti-DCIR antibody described later.

For the DCIR inhibitor, inactivation of DCIR can be confirmed by a method according to the methods described in detail in the section of the anti-DCIR antibody.

Hereinafter, an anti-NA2 antibody and an anti-DCIR antibody that may be contained in the pharmaceutical composition according to the present disclosure will be described in detail.

### - Anti NA2 Antibody -

An anti-NA2 antibody is an antibody that specifically binds to NA2. The anti-NA2 antibody may be any antibody that inhibits binding between DCIR and asialo-biantennary N-glycan. NA2 is an asialo-biantennary N-glycan, which is a ligand for DCIR. Examples of NA2 include a glycan having a structure represented by the following formula (here, no sialic acid is present at the two non-reducing terminals of the structure represented by the following formula (A), and each of x and y independently represents 3 or 4) as a basic structure.

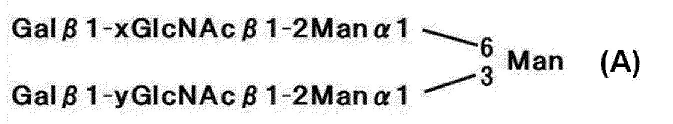

The NA2 that is specifically bound by the anti-NA2 antibody may be one having a glycan having the structure represented by the foregoing formula as a basic structure, and having one branched chain at a certain position of the foregoing basic structure, or having a fucose added thereto. However, there is no sialic acid at the two non-reducing terminals of the foregoing structure. Examples of such a glycan structure include the following glycans.

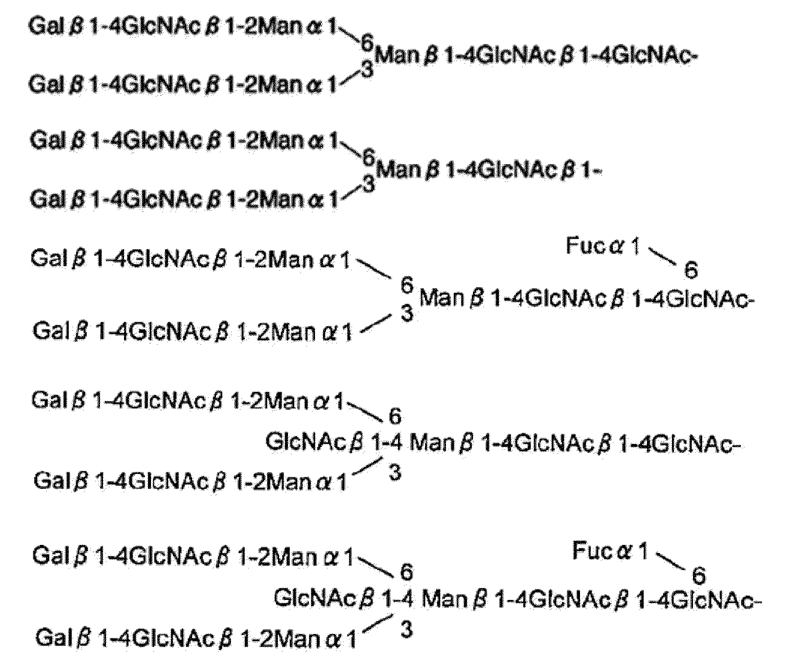

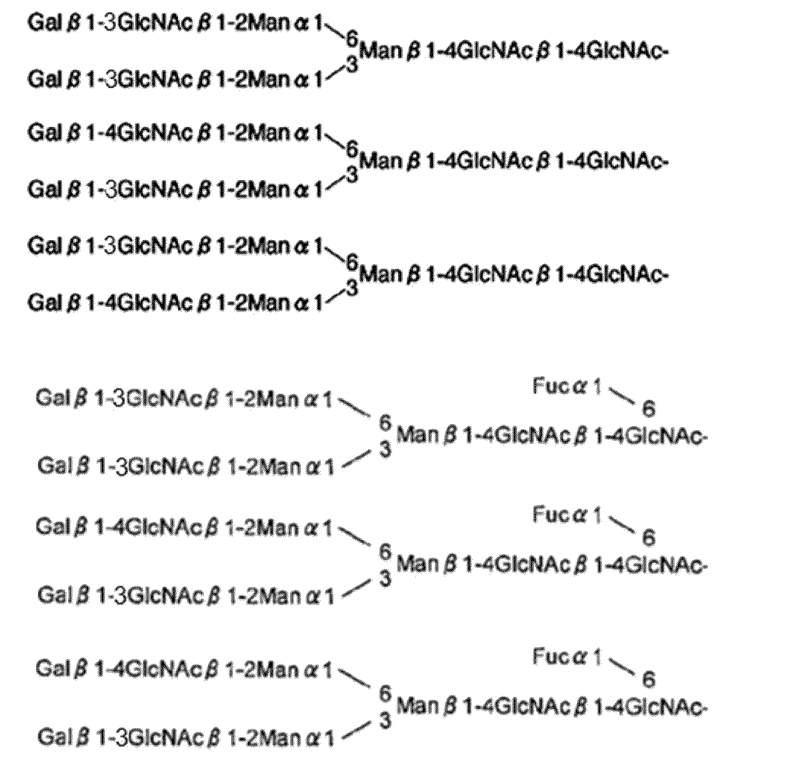

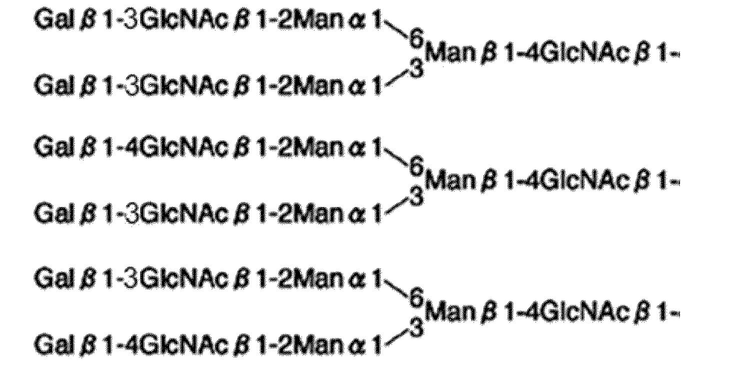

All the NA2s represented by the foregoing formulae are ligands for DCIR, and it is considered that an antibody that binds to any of these NA2s inhibits the binding between DCIR and these NA2s.

The anti-NA2 antibody may be an antibody that recognizes a protein (including a peptide) modified with NA2 or recognizes another polymer compound modified with NA. Examples of the protein modified with NA2 include bovine serum albumin (BSA), ovalbumin (OVA), transferin, and the like, modified with NA2. Examples of the polymer compound modified with NA2 include polyallylamine (PAA) modified with NA. One in which the reducing terminal of the glycan structure of NA2 is linked to any of these proteins or other polymer compounds may be used.

NA2 can be obtained, for example, by collecting NA2 from a living body, producing NA2 by a genetic engineering method, or producing NA2 by an organic synthetic chemical method. Further, NA2 can be produced from commercially available materials. For example, in the case of a PAA-modified derivative, a company name: GlycoTech Co., a product name: Multivalent Biotinylated Polymer (structure: (SA2-6G-GN-M)₂-3,6-M-GN-GNβ-PAA-biotin, in which SA = NeuSAc, G = Gal, GN = GlcNAc, M = Man, sp = -NHCOCH₂NH-, may be treated with neuraminidase to obtain NA2. Therefore, NA2 may have a sugar chain structure represented by (G-GN-M) ₂-3, 6-M-GN-GNβ-.

NA2 may be derived from a living body and may be derived from a non-living body. When NA2 is derived from a living body, NA2 may be derived from a glycoprotein (including a glycopeptide) of a living body. Examples of the glycoprotein include the examples described above. For details of the NA2, for example, International Publication No. 2016/006700 can be referred to.

In one aspect, the anti-NA2 antibody has at least one selected from the group consisting of the heavy chain CDRs 1 to 3, light chain CDRs 1 to 3, heavy chain FRs 1 to 4, and light chain FRs 1 to 4 of the following sequences.

In one aspect, the anti-NA2 antibody has all of the heavy chain CDRs 1 to 3 of the following sequences.

In one aspect, the anti-NA2 antibody has all of the light chain CDRs 1 to 3 of the following sequences.

In one aspect, the anti-NA2 antibody has all of the heavy chain FRs 1 to 4 of the following sequences.

In one aspect, the anti-NA2 antibody has all of the light chain FRs 1 to 4 of the following sequences.

In one aspect, the anti-NA2 antibody has all of the heavy chain CDRs 1 to 3 and the light chain CDRs 1 to 3 of the following sequences.

In one aspect, the anti-NA2 antibody has all of the heavy chain FRs 1 to 4 and the light chain FRs 1 to 4 of the following sequences.

In one aspect, the anti-NA2 antibody has all of the heavy chain CDRs 1 to 3, the light chain CDRs 1 to 3, the heavy chain frameworks (FRs) 1 to 4, and the light chain FRs 1 to 4 of the following sequences.

The base sequence and corresponding amino acid sequence of each region of the anti-NA2 antibody in one aspect are shown below.
Heavy chain FR1 (SEQ ID NO: 51)
   gaggtgaagcttctcgagtctggaggtggcctggtgcagcctggaggatccctgaaactctcctgtgcagcctca
Heavy chain CDR1 (SEQ ID NO: 52)
   ggattcgattttagtagatactgg
Heavy chain FR2 (SEQ ID NO: 53)
   atgagttgggtccggcaggctccagggaaagggctagaatggattggagaa
Heavy chain CDR2 (SEQ ID NO: 54)
   attaatccagatagcagtacgata
Heavy chain FR3 (SEQ ID NO: 55) 
aactatacgccatctctaaaggataaattcatcatctccagagacaacgccaaaaatacgctgtacctgcaaatgagcaaagtgagatct
gaggacacagccctttattactgt
Heavy chain CDR3 (SEQ ID NO: 56)
   gcaagacctgatgggacgatttattactacggttgctggtacttcgatgtc
Heavy chain FR4 (SEQ ID NO: 57)
   tggggcgcagggaccacggtc
Light chain FR1 (SEQ ID NO: 58) gatgttgtgatgacccaaactccactctccctgcctgtcagtctgggagatcaagcctccatctcttgcagatctagt
Light chain CDR1 (SEQ ID NO: 59)
   cagagtcttgcacacagtaatggaaacacctat
Light chain FR2 (SEQ ID NO: 60)
   ttacattggtacctgcagaagccaggccagtctccaaagctcctgatctac
Light chain CDR2
   aaaatttcc
Light chain FR3 (SEQ ID NO: 61) 
aaccgattttctggggtcccagacaggttcagtggcagtggatcagggacagatttcacactcaagatcagcagagtggaggctgagg
atctgggagtttatttctgc
Light chain CDR3 (SEQ ID NO: 62)
   tctcaaagtacacatgttccgtggacg
Light chain FR4 (SEQ ID NO: 63)
   ttcggtggaggcaccaagctggaaatcaaa
Heavy chain FR1 (SEQ ID NO: 64)
   EVKLLESGGGLVQPGGSLKLSCAAS
Heavy chain CDR1 (SEQ ID NO: 65)
   GFDFSRYW
Heavy chain FR2 (SEQ ID NO: 66)
   MSWVRQAPGKGLEWIGE
Heavy chain CDR2 (SEQ ID NO: 67)
   INPDSSTI
Heavy chain FR3 (SEQ ID NO: 68)
   NYTPSLKDKFIISRDNAKNTLYLQMSKVRSEDTALYYC
Heavy chain CDR3 (SEQ ID NO: 69)
   ARPDGTIYYYGCWYFDV
Heavy chain FR4 (SEQ ID NO: 70)
   WGAGTTV
Light chain FR1 (SEQ ID NO: 71)
   DVVMTQTPLSLPVSLGDQASISCRSS
Light chain CDR1 (SEQ ID NO: 72)
   QSLAHSNGNTY
Light chain FR2 (SEQ ID NO: 73)
   LHWYLQKPGQSPKLLIY
Light chain CDR2
   KIS
Light chain FR3 (SEQ ID NO: 74)
   NRFSGVPDRFSGSGSGTDFTLKISRVEAEDLGVYFC
Light chain CDR3 (SEQ ID NO: 75)
   SQSTHVPWT
Light chain FR4 (SEQ ID NO: 76)
   FGGGTKLEIK

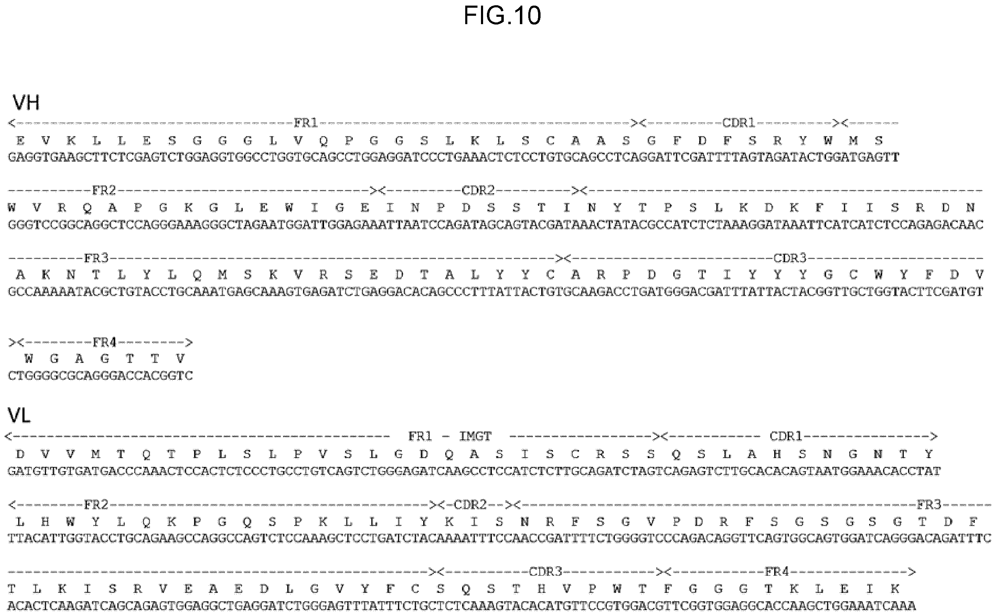

FIG. 10 shows an example of the DNA sequences (SEQ ID NO: 77 and SEQ ID NO: 78, respectively) and the amino acid sequences (SEQ ID NO: 79 and SEQ ID NO: 80, respectively) of the heavy chain variable region and the light chain variable region of an anti-NA2 antibody.
Heavy chain variable region (SEQ ID NO: 77) 
gaggtgaagcttctcgagtctggaggtggcctggtgcagcctggaggatccctgaaactctcctgtgcagcctcaggattcgattttagt
agatactggatgagttgggtccggcaggctccagggaaagggctagaatggattggagaaattaatccagatagcagtacgataaact
atacgccatctctaaaggataaattcatcatctccagagacaacgccaaaaatacgctgtacctgcaaatgagcaaagtgagatctgag
gacacagccctttattactgtgcaagacctgatgggacgatttattactacggttgctggtacttcgatgtctggggcgcagggaccacg
gtc
Light chain variable region (SEQ ID NO: 78) 
gatgttgtgatgacccaaactccactctccctgcctgtcagtctgggagatcaagcctccatctcttgcagatctagtcagagtcttgcaca
cagtaatggaaacacctatttacattggtacctgcagaagccaggccagtctccaaagctcctgatctacaaaatttccaaccgattttctg
gggtcccagacaggttcagtggcagtggatcagggacagatttcacactcaagatcagcagagtggaggctgaggatctgggagttt
atttctgctctcaaagtacacatgttccgtggacgttcggtggaggcaccaagctggaaatcaaa
Heavy chain variable region (SEQ ID NO: 79) 
EVKLLESGGGLVQPGGSLKLSCAASGFDFSRYWMSWVRQAPGKGLEWIGEINPDSST
INYTPSLKDKFIISRDNAKNTLYLQMSKVRSEDTALYYCARPDGTIYYYGCWYFDVW
GAGTTV
Light chain variable region (SEQ ID NO: 80)

DVVMTQTPLSLPVSLGDQASISCRSSQSLAHSNGNTYLHWYLQKPGQSPKLLIYKISN
RFSGVPDRFSGSGSGTDFTLKISRVEAEDLGVYFCSQSTHVPWTFGGGTKLEIK

Note that the anti-NA2 antibody is not limited to the antibody having the sequence of FIG. 10, and may be any antibody that exhibits a binding activity to NA2 and has the ability to inhibit the binding between NA2 and DCIR. Various antibodies that exhibit a binding activity to NA2 and have the ability to inhibit the binding between NA2 and DCIR can be produced by those having ordinary skill the art based on the methods described in the present disclosure and known antibody acquisition techniques. It is known that the human has NA2 having the same glycan as mouse NA2, and a mouse anti-NA2 antibody is also effective for humans.

For example, the anti-NA2 antibody may be an antibody having a heavy chain variable region having 80% or more, 85% or more, 90% or more, or 95% or more amino acid sequence identity to SEQ ID NO: 79. The anti-NA2 antibody may be an antibody having a light chain variable region having 80% or more, 85% or more, 90% or more, or 95% or more amino acid sequence identity to SEQ ID NO: 80. The anti-NA2 antibody may be an antibody having heavy chain CDRs 1 to 3, light chain CDRs 1 to 3, or a combination thereof of the foregoing sequences, having a heavy chain variable region having 80% or more, 85% or more, 90% or more, or 95% or more amino acid sequence identity to SEQ ID NO: 79, and having a light chain variable region having 80% or more, 85% or more, 90% or more, or 95% or more amino acid sequence identity to SEQ ID NO: 80. The anti-NA2 antibody may be an antibody having heavy chain CDRs 1 to 3, light chain CDRs 1 to 3, or a combination thereof of the foregoing sequences, having a heavy chain variable region having 90% or more amino acid sequence identity to SEQ ID NO: 79, and having a light chain variable region having 90% or more amino acid sequence identity to SEQ ID NO: 80.

In the present disclosure, the "identity" of an amino acid sequence refers to the ratio of the number of matching amino acid residues to the total number of amino acid residues when two amino acid sequences to be compared are aligned such that the number of matching amino acid residues is maximized, by inserting a gap into one or both of the amino acid sequences as needed. The alignment can be performed using a known alignment tool such as BLAST, FASTA, CLUSTAL W, or the like. For example, the alignment can be evaluated with a default parameter of BLAST.

The binding activity of the anti-NA2 antibody can be confirmed by the following procedure. Ovalbumin (OVA) or NA2-OVA (10 µg/ml) is coated on a 96 well plate, and allowed to stand overnight at 4°C to coat the plate with the antigen. Thereafter, the plate is washed and blocked by 10% (w/v) FCS-containing PBS at room temperature for 1 hour. Subsequently, the plate is washed, an isotype antibody or an anti-NA2 antibody is added thereto, and the plate is allowed to stand at room temperature for 1 hour. After the plate is washed, the isotype antibody is detected using a labeled antibody. Specifically, the binding activity of the anti-NA2 antibody can be confirmed by the method described in the Examples.

The ability of an anti-NA2 antibody to inhibit the binding between NA2 and DCIR can be confirmed by the following procedure.

NA2-OVA or OVA (10 µg/ml) is added to the bottom of a 96-well plate and allowed to bind to the plate by standing overnight at 4°C. The plate is blocked by 10% (w/v) FCS-containing PBS, and Fc or DCIR-Fc (5 µg/ml) and an anti-NA2 antibody are added thereto. After the plate was allowed to stand at room temperature for 1 hour and washed, the Fc or DCIR-Fc associated with the plate is detected by Peroxidase-conjugated anti-human Fc (Jackson Immunoresearch Inc.). Specifically, the ability of an anti-NA2 antibody to inhibit the binding between NA2 and DCIR can be confirmed by the method described in the Examples section (FIG. 9A).

The anti-NA2 antibody can be produced by any suitable method. For example, an anti-NA2 antibody can be produced, for example, *in vivo,* using cultured cells, by an *in vitro* translation reaction, or using a recombinant DNA expression system. Alternatively, an anti-NA2 antibody can be produced by immunizing a nude mouse with a monoclonal antibody-producing hybridoma and purifying the antibody from peritoneal water.

The isotype of the anti-NA2 antibody may be, for example, IgG, IgM, IgA, IgD, or IgE, and IgM is most preferable.

The anti-NA2 antibody may be a full-length antibody and may be a low-molecular antibody such as an antibody fragment that specifically binds to NA2. Examples of the antibody fragment include Fab, Fab', F (ab')₂, scFv, and diabody. As a method for obtaining these antibody fragments, gene encoding any of these antibody fragments may be constructed and introduced into an expression vector, and then expressed in suitable host cells.

Further, the anti-NA2 antibody may be a modified antibody. As the modified antibody, an antibody bound to any of various kinds of molecules, such as polyethylene glycol (PEG) or biotin, may be used. Such a modified antibody may be obtained by chemically modifying the obtained antibody. As the method for modifying the antibody, any method known in the art may be used.

The anti-NA2 antibody may be a polyclonal antibody, may be a monoclonal antibody, or may be a derivative of an antibody such as an antibody that retains the ability to specifically bind to an antigen-determining group.

A polyclonal antibody can be obtained by isolating antiserum containing antibodies from a GalT-deficient mouse immunized with an NA2 derivative, and screening for the presence of an antibody having a desired specificity using a known method, such as NA2 glycan ELISA assay or staining of asialoglycan-expressing cells.

As a method for producing a monoclonal antibody and a hybridoma, a method devised for producing the present antibody based on known methods. For example, using a NA2-BSA derivative in which 10 or more molecules of NA2 are bound to BSA as an immunogen, a mouse deficient in galactosyl transferase glycan transferase and thereby devoid of asialoglycan in the body (GalT1-deficient mouse) may be immunized by subcutaneous injection of the NA2-BSA complex (GT-111157, manufactured by GlyTech, Inc.). A wild type animal may also be immunized. An adjuvant (Titer max gold; manufactured by TiterMax USA, Inc.) may be used for the immunization. For the adjuvant, adjuvants known in the art may be used.

A monoclonal antibody can be obtained by resecting spleen cells from an immunized animal and fusing the cells with myeloma cells to produce hybridoma cells that produce a monoclonal antibody. Hybridoma cells that produce an antibody that recognizes a target NA2 glycan are screened by using a known method, such as an ELISA assay using a 96-well plate bound by OVA-bound NA2 glycan, or detection of the binding to an asialoglycan-expressing cell line (Lec2 cells) by staining. A hybridoma that secretes a desired antibody may be cloned and cultured under a suitable condition, and the secreted antibody may be recovered and purified by a known method such as with an ion exchange column, affinity chromatography, or protein L-associated beads. In addition, a hybridoma may be transplanted into an immunodeficient mouse, and peritoneal fluid containing the secreted antibody may be recovered and purified in a similar manner. Production of an antibody in cultured cells based on the obtained sequence of the BCR VDJ region, production of a human monoclonal antibody using XenoMouse (see Green, J. Immunol. Methods 231:11-23, 1999; Wells, Eek, Chem Biol 2000 Aug;7(8): R185-6), and production of a monoclonal antibody based on phage display that does not involve immunization may also be employed.

As the anti-NA2 antibody, a mouse antibody, a human antibody, a rat antibody, a rabbit antibody, a sheep antibody, a camelid antibody, an avian antibody or the like may be used, and a genetically modified antibody that has been artificially modified for the purpose of, for example, reducing heteroantigenicity in humans (e.g., a chimeric antibody, a humanized antibody etc.) may also be used. As the anti-NA2 antibody, a mouse antibody, a chimeric antibody, a humanized antibody, and a human antibody are preferred, and a humanized antibody is more preferable.

A chimeric antibody is an antibody that include the variable regions of the heavy and light chains of an antibody of a mammal other than human, such as a mouse antibody, and the constant regions of the heavy and light chains of a human antibody. A chimeric antibody can be obtained by linking the DNA encoding the variable regions of the mouse antibody to the DNA encoding the constant regions of the human antibody, incorporating the DNA into an expression vector, and introducing the vector into a host for production.

A humanized antibody is obtained by transplanting the complementarity determining regions (CDRs) of an antibody of a mammal other than human, such as a mouse antibody, into the complementarity determining regions of a human antibody, and general genetic recombination methods thereof are also known. Specifically, a DNA sequence designed to link the CDR of a mouse antibody and the framework region (FR) of a human antibody is synthesized by PCR from several oligonucleotides prepared such that the oligonucleotides have overlapping portions at their ends. The obtained DNA is linked to DNA encoding the constant region of a human antibody, and then incorporated into an expression vector, which is introduced into a host for production. The FRs of the human antibody linked via the CDRs are selected such that the complementary determining regions form a suitable antigen-binding site. If necessary, amino acid(s) in the framework regions of the variable region of the antibody may be substituted such that the complementarity determining regions of the humanized antibody form a suitable antigen-binding site (Sato, K. et al., Cancer Res, 1993, 53, 851-856).

A human antibody can also be produced using known methods. For example, human lymphocytes may be sensitized *in vitro* with a desired antigen or cells expressing the desired antigen, and the sensitized lymphocytes may be fused with human myeloma cells, such as U266, to obtain a desired human antibody having binding activity to the antigen. Further, a desired human antibody may be obtained by immunizing a transgenic animal having a full repertoire of human antibody genes with a desired antigen. Furthermore, a technique for obtaining a human antibody by panning using a human antibody library is also known. For example, the variable region of a human antibody can be expressed on the surface of a phage as a single chain antibody (scFv) by a phage display method, and a phage that binds to the antigen may be selected. By analyzing the gene of the selected phage, the DNA sequence encoding the variable region of a human antibody that binds to the antigen can be determined. Since the DNA sequence of the scFv that binds to the antigen is known (see, for example, FIG. 10), a suitable expression vector can be prepared using the sequence to obtain a human antibody. Methods for producing antibodies based on DNA sequences are well known.

### -Anti-DCIR antibody-

An anti-DCIR antibody is an antibody that specifically binds to DCIR. DCIR is a member of the CLR family, and is a membrane protein that has a glycan recognition domain outside the cell and an immunoreceptor tyrosine-based inhibition motif (ITIM) inside the cell. The anti-DCIR antibody may be an antibody that inhibits the binding between DCIR and NA2, or an inhibitory antibody that attenuates or inactivates the action of DCIR. As an example, an anti-DCIR antagonist antibody is preferable. The anti-DCIR antagonist antibody may be a competitive antagonist antibody that competes with NA2, or a non-competitive antagonist antibody.

The binding activity of the anti-DCIR antibody can be confirmed by the following procedure.

The binding activity to a DCIR-expressing cell line is measured as follows. An isotype or anti-DCIR antibody (10 µg/ml) is added to a DCIR-expressing cell line or blank cells (HEK293 cells), which is suspended, and allowed to stand on ice for 30 minutes. The cells are washed, and a fluorescent substance-labeled anti-mouse IgM antibody (0.5 µg/ml) is added and allowed to stand on ice for 30 minutes. The cells are washed, and the binding of the antibody to the cells is measured by flow cytometry. Alternatively, the binding activity to the DCIR protein is measured as follows. An isotype antibody or anti-DCIR antibody (5 µg/ml) is coated on a 96-well plate and allowed to stand overnight at 4°C to coat the plate. The plate is then washed, and blocked with PBS containing 10% (w/v) FCS at room temperature for 1 hour. The plate is then washed, and Fc or DCIR-Fc protein (5 µg/ml) is added and allowed to stand at room temperature for 1 hour. After washing the plate, the DCIR-Fc protein bound to the coated antibody is detected using an HRP-labeled anti-human Fc antibody.

The ability of an anti-DCIR antibody that is a DCIR-NA2 binding inhibitor to inhibit the binding between NA2 and DCIR can be confirmed by the following procedure:
DCIR-Fc and an anti-DCIR antibody or an isotype antibody (10 µg/ml) are added to Lec2 cells, which express asialoglycans (mainly NA2), and are suspended and allowed to stand on ice for 30 minutes. The cells are washed, and the binding of the DCIR-Fc protein to Lec2 cells is detected using a fluorescently labeled anti-human Fc antibody.

The inactivation of DCIR by the anti-DCIR antibody that is a DCIR inhibitor can be confirmed by the following procedure. M-CSF and sRANKL are added to bone marrow cells of a wild type mouse, and the cells are cultured for about one week. The degree of osteoclast differentiation can be evaluated by TRAP staining and counting TRAP-positive multinucleated cells (50). The anti-DCIR antibody is added to this differentiation induction system, the degree of osteoclast differentiation is measured, and the effect on osteoclast differentiation is evaluated by comparing with the case in which an isotype antibody is added. Since it has been found that osteoclast differentiation is suppressed by the activation of DCIR, if osteoclast differentiation is promoted by the anti-DCIR antibody, it is considered that DCIR is inactivated and the suppression of the differentiation is released. A similar experiment using bone marrow cells of a DCIR-deficient mouse is performed in parallel to confirm that the anti-DCIR antibody does not inhibit osteoclast formation in a non-specific manner.

The anti-DCIR antibody can be produced by any suitable method. For example, the anti-DCIR antibody may be produced by cultured cells. More specifically, for example, the anti-DCIR antibody may be produced by the following method.

The antibody is established by the following procedure. A DCIR-expressing cell line (a cell line obtained by introducing a DCIR expression vector into HEK293 cells to express DCIR on the cell surface) is mixed with an immune adjuvant (Titer Max), and a DCIR-deficient mouse is immunized twice. When an increase in the antibody titer against DCIR is observed, the mouse is sacrificed. Spleen cells are collected and fused with myeloma cells according to a standard method to produce hybridoma cells, and a monoclonal library is produced by limiting dilution. Next, a DCIR-specific antibody is identified based on the binding activity to DCIR-expressing cells and a DCIR protein. The established monoclonal antibody-producing hybridoma is adapted to serum-free medium, and finally, the antibody is purified from the cell culture medium using Protein L-associated beads. For the purpose of administration to humans, the antibody may be humanized.

With regard to the embodiments of the antibody in the anti-DCIR antibody (an isotype, a full-length antibody or a low-molecular antibody, a modified antibody, a polyclonal antibody and a monoclonal antibody, the origin of the antibody, and the method for producing the antibody), the matters described in the section of the anti-NA2 antibody are applicable, except for matters specific to the anti-NA2 antibody.

In one aspect, the anti-DCIR antibody has at least one selected from the group consisting of the heavy chain CDRs 1 to 3, light chain CDRs 1 to 3, heavy chain FRs 1 to 4, and light chain FRs 1 to 4 of the following sequences.

In one aspect, the anti-DCIR antibody has all of the heavy chain CDRs 1 to 3 of the following sequences.

In one aspect, the anti-DCIR antibody has all of the light chain CDRs 1 to 3 of the following sequences.

In one aspect, the anti-DCIR antibody has all of the heavy chain FRs 1 to 4 of the following sequences.

In one aspect, the anti-DCIR antibody has all of the light chain FRs 1 to 4 of the following sequences.

In one aspect, the anti-DCIR antibody has all of the heavy chain CDRs 1 to 3 and the light chain CDRs 1 to 3 of the following sequences.

In one aspect, the anti-DCIR antibody has all of the heavy chain FRs 1 to 4 and the light chain FRs 1 to 4 of the following sequences.

In one aspect, the anti-DCIR antibody has all of the heavy chain CDRs 1 to 3, the light chain CDRs 1 to 3, the heavy chain frameworks (FRs) 1 to 4, and the light chain FRs 1 to 4 of the following sequences.

The base sequence and corresponding amino acid sequence of each region of the anti-DCIR antibody (anti-DCIR antagonist antibody (SA3D8) BCR VDJ region sequence) in one aspect are shown below.
Heavy chain FR1 (SEQ ID NO: 81)
   gaggtccagctgcagcagtctggacctgagctggtaaagcctggggcttcagtgaagatgtcctgcaaggcttct
Heavy chain CDR1 (SEQ ID NO: 82)
   ggatacacattcactagctatgtt
Heavy chain FR2 (SEQ ID NO: 83)
   atgcactgggtgaagcagaagcctgggcagggccttgagtggattggatat
Heavy chain CDR2 (SEQ ID NO: 84)
   attaatccttacaatgatggtact
Heavy chain FR3 (SEQ ID NO: 85) 
aagtacaatgagaagttcaaaggcaaggccacactgacttcagacaaatcctccagcaca
gcctacatggagctcagcagcctgacctctgaggactatgcggtctattactgt
Heavy chain CDR3 (SEQ ID NO: 86) gcaagatcctacaagaggtacgactggtacccaaga
Heavy chain FR4 (SEQ ID NO: 87) tggggcgcagggaccacggtc
Light chain FR1 (SEQ ID NO: 88) 
gacattgtgatgacccagtctcaaaaattcatgtccacatcagtaggagacagggtcagc
gtcacctgcaaggccagt
Light chain CDR1 (SEQ ID NO: 89)
   cagaatgtgggtactaat
Light chain FR2 (SEQ ID NO: 90)
   gtagcctggtatcaacagaaaccagggcaatctcctaaagcactgatttac
Light chain CDR2 (SEQ ID NO: 91)
   tcggcatcc
Light chain FR3 (SEQ ID NO: 92) 
taccggtacagtggagtccctgatcgcttcacaggcagtggatctgggacagatttcact
ctcaccatcagcaatgtgcagtctgaagacttggcagagtatttctgt
Light chain CDR3 (SEQ ID NO: 93)
   cagcaatataacagctatccgctcacg
Light chain FR4 (SEQ ID NO: 94)
   ttcggtggaggcaccaagctggaaatcaaa
Heavy chain FR1 (SEQ ID NO: 95)
   EVQLQQSGPELVKPGASVKMSCKAS
Heavy chain CDR1 (SEQ ID NO: 96)
   GYTFTSYV
Heavy chain FR2 (SEQ ID NO: 97)
   MHWVKQKPGQGLEWIGY
Heavy chain CDR2 (SEQ ID NO: 98)
   INPYNDGT
Heavy chain FR3 (SEQ ID NO: 99)
   KYNEKFKGKATLTSDKS S STAYMELS SLTSEDYAVYYC
Heavy chain CDR3 (SEQ ID NO: 100)
   ARSYKRYDWYPR
Heavy chain FR4 (SEQ ID NO: 101)
   WGAGTTV
Light chain FR1 (SEQ ID NO: 102)
   DIVMTQSQKFMSTSVGDRVSVTCKAS
Light chain CDR1 (SEQ ID NO: 103)
   QNVGTN
Light chain FR2 (SEQ ID NO: 104)
   VAWYQQKPGQSPKALIY
Light chain CDR2 (SEQ ID NO: 105)
   SAS
Light chain FR3 (SEQ ID NO: 106)
   YRYSGVPDRFTGSGSGTDFTLTISNVQSEDLAEYFC
Light chain CDR3 (SEQ ID NO: 107)
   QQYNSYPLT
Light chain FR4 (SEQ ID NO: 108)
   FGGGTKLEIK

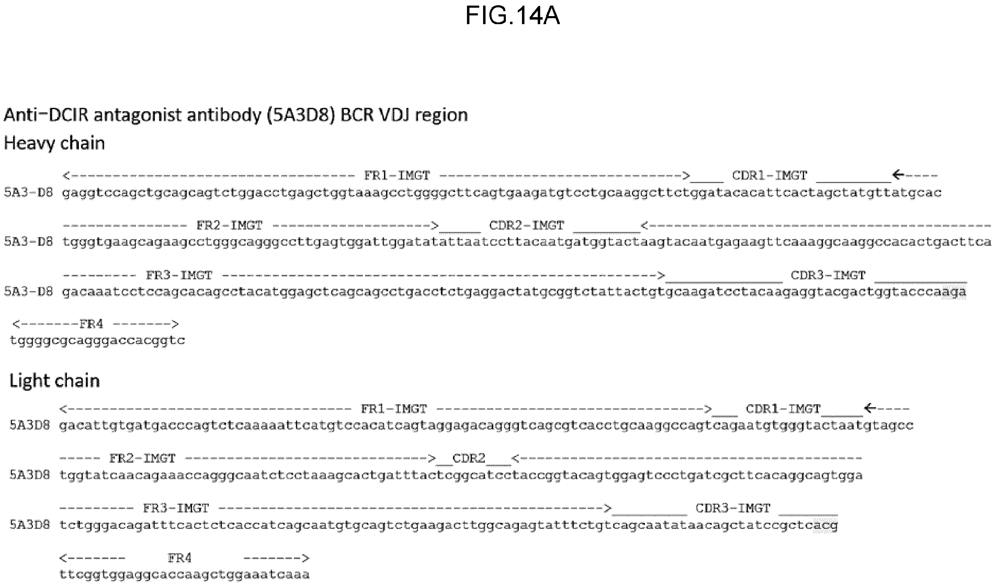

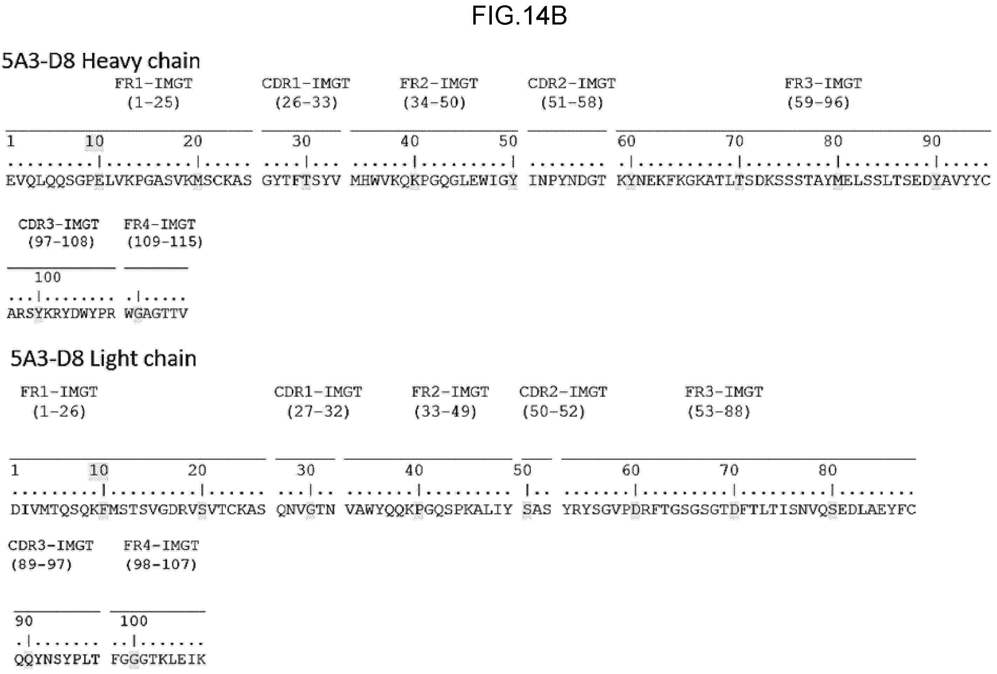

FIGs. 14A and 14B show an example of the DNA sequences (SEQ ID NO: 109 and SEQ ID NO: 110, respectively) and the amino acid sequences (SEQ ID NO: 111 and SEQ ID NO: 112, respectively) of the heavy chain variable region and the light chain variable region of the anti-DCIR antagonist antibody.
Heavy chain variable region (SEQ ID NO: 109) 
gaggtccagctgcagcagtctggacctgagctggtaaagcctggggcttcagtgaagatgtcctgcaaggcttctggatacacattcac
tagctatgttatgcactgggtgaagcagaagcctgggcagggccttgagtggattggatatattaatccttacaatgatggtactaagtac
aatgagaagttcaaaggcaaggccacactgacttcagacaaatcctccagcacagcctacatggagctcagcagcctgacctctgag
gactatgcggtctattactgtgcaagatcctacaagaggtacgactggtacccaagatggggcgcagggaccacggtc
Light chain variable region (SEQ ID NO: 110) 
gacattgtgatgacccagtctcaaaaattcatgtccacatcagtaggagacagggtcagcgtcacctgcaaggccagtcagaatgtgg
gtactaatgtagcctggtatcaacagaaaccagggcaatctcctaaagcactgatttactcggcatcctaccggtacagtggagtccctg
atcgcttcacaggcagtggatctgggacagatttcactctcaccatcagcaatgtgcagtctgaagacttggcagagtatttctgtcagca
 atataacagctatccgctcacg,ttcggtggaggcaccaagctggaaatcaaa
Heavy chain variable region (SEQ ID NO: 111) 
EVQLQQSGPELVKPGASVKMSCKAS GYTFTSYV MHWVKQKPGQGLEWIGY
INPYNDGTKY NEKFKGKATLTSDKSSSTAYMELSSLTSEDYAVYYC
ARSYKRYDWYPR WGAGTTV
Light chain variable region (SEQ ID NO: 112)

DIVMTQSQKFMSTSVGDRVSVTCKAS QNVGTN VAWYQQKPGQSPKALIY SAS
YRYSGVPDRFTGSGSGTDFTLTISNVQSEDLAEYFC QQYNSYPLT FGGGTKLEIK

Note that the anti-DCIR antibody is not limited to the antibody having the sequences of FIG. 14A and FIG. 14B, and may be any antibody that exhibits a binding activity to DCIR and has the ability to inhibit DCIR or inhibit the binding between NA2 and DCIR. Various antibodies that exhibit a binding activity to DCIR and have the ability to inhibit DCIR or inhibit the binding between NA2 and DCIR can be produced by those skilled in the art based on the methods described in the present disclosure and known antibody acquisition techniques.

For example, the anti-DCIR antibody may be an antibody having a heavy chain variable region having 80% or more, 85% or more, 90% or more, or 95% or more amino acid sequence identity to SEQ ID NO: 111. The anti-DCIR antibody may be an antibody having a light chain variable region having 80% or more, 85% or more, 90% or more, or 95% or more amino acid sequence identity to SEQ ID NO: 112. The anti-DCIR antibody may be an antibody having heavy chain CDRs 1 to 3, light chain CDRs 1 to 3, or a combination thereof of the foregoing sequences, having a heavy chain variable region having 80% or more, 85% or more, 90% or more, or 95% or more amino acid sequence identity to SEQ ID NO: 111, and having a light chain variable region having 80% or more, 85% or more, 90% or more, or 95% or more amino acid sequence identity to SEQ ID NO: 112. The anti-DCIR antibody may be an antibody having heavy chain CDRs 1 to 3, light chain CDRs 1 to 3, or a combination thereof of the foregoing sequences, having a heavy chain variable region having 90% or more amino acid sequence identity to SEQ ID NO: 111, and having a light chain variable region having 90% or more amino acid sequence identity to SEQ ID NO: 112.

The pharmaceutical composition according to the present disclosure is used for treating or preventing at least one disease selected from the group consisting of inflammatory bowel disease and intestinal tumor. Examples of the inflammatory disease include Crohn's disease and ulcerative colitis. Examples of the intestinal tumor include colorectal cancer, rectal cancer, and small intestinal cancer. In autoimmune diseases such as collagen-induced arthritis (CIA) and experimental autoimmune encephalomyelitis (EAE), inhibition of the binding between DCIR and NA2 or inhibition of DCIR is thought to exacerbate the disease. However, the present invention has demonstrated that inhibition of the binding between DCIR and NA2 or inhibition of DCIR suppresses inflammatory bowel disease and intestinal tumor in the large intestine. This is because innate lymphocyte 3 (ILC3) cells, which are not present in other organs, are abundant in the intestine.

Examples of the pharmaceutically acceptable carrier used in the pharmaceutical composition according to the present disclosure include various organic or inorganic carrier substances that are commonly used, such as an excipient, a lubricant, a binder, and a disintegrant in a solid preparation; and a solvent, a solubilizing agent, a suspending agent, an isotonicity agent, a buffer, and a soothing agent in a liquid preparation. In addition, an additive, such as a preservative, an antioxidant, a colorant, a sweetener, an adsorbent, or a wetting agent, may also be used as necessary.

The form of the pharmaceutical composition according to the present disclosure is not particularly limited. The pharmaceutical composition according to the present disclosure may be formulated, for example, by mixing, dissolving, granulating, tableting, emulsifying, encapsulating, or lyophilizing the components used in the pharmaceutical composition.

For example, the pharmaceutical composition according to the present disclosure may be formulated for oral administration, more specifically, in the form of a tablet, a pill, a sugarcoated tablet, a soft capsule, a hard capsule, solution, suspension, emulsion, gel, syrup, slurry, or the like.

The pharmaceutical composition according to the present disclosure may also be formulated for parenteral administration, more specifically, in the form of injection solution, suspension, emulsion, cream, ointment, inhalant, suppository, or the like.

The route of administration of the pharmaceutical composition is not particularly limited. Examples of the route of administration include oral administration, subcutaneous administration, intraperitoneal administration, intramuscular administration, eye drop, ear drop, nasal administration, inhalation administration, transdermal administration, rectal administration, intrathecal administration, intravenous administration, and surgical procedures such as placement.

The dosage and administration interval of the pharmaceutical composition are appropriately set depending on the conditions such as the route of administration; symptoms, body weight, physique, age, and DCIR activity state of the subject to be administered; and types and amounts of components to be used in combination. In one aspect, the pharmaceutical composition according to the present disclosure is administered to a subject having at least one disease selected from the group consisting of inflammatory diseases and intestinal tumors at a dosage and interval that provides a therapeutically or prophylactically effective amount of a DCIR-NA2 binding inhibitor or a DCIR inhibitor. The administration interval may be shorter than one day, may be once a day, or may be longer than one day.

According to one aspect of the present disclosure, a method for treating or preventing at least one disease selected from the group consisting of inflammatory bowel disease and intestinal tumor is provided, the method including administering to a subject an effective amount of a DCIR-NA2 binding inhibitor or a DCIR inhibitor.

According to another aspect of the present disclosure, use of a DCIR-NA2 binding inhibitor or a DCIR inhibitor in the manufacture of a medicament for treating or preventing at least one disease selected from the group consisting of inflammatory bowel disease and intestinal tumor is provided.

According to another aspect of the present disclosure, a DCIR-NA2 binding inhibitor or a DCIR inhibitor for use in the treatment or prevention of at least one disease selected from the group consisting of inflammatory bowel disease and intestinal tumor is provided.

In these aspects, the at least one disease selected from the group consisting of inflammatory bowel disease and intestinal tumor, the DCIR-NA2 binding inhibitor and the DCIR inhibitor, and the scope of applications thereof are the same as those described above. Examples

Hereinafter, embodiments of the present disclosure will be described in detail by way of Examples. However, the embodiments of the present disclosure are not limited to these Examples.

### [Example 1]

### 1. Introduction

Inflammatory bowel disease (IBD) including Crohn's disease (CD) and ulcerative colitis (UC) is a chronic inflammatory disease of the intestinal tract that ultimately causes the development of colorectal tumors [1, 2]. Innate immune receptors such as C-type lectin receptors (CLRs) and Toll-like receptors (TLRs) expressed in the intestine are suggested to play important roles not only for the host defense against pathogens but also for the development of IBD by recognizing commensal microbiota and food components to induce reactive oxygen species (ROS), antimicrobial proteins (AMPs), cytokines, and chemokines [3, 4]. Thus, a fine balance between the commensal microbiota and mucosal immune system is needed to maintain homeostasis of the intestine [4].

In this context, the inventors previously showed that Dectin-1, a CLR that recognizes β-glucans, induces antimicrobial protein S100A8 in the intestine by recognizing β-glucans in foods, and suppresses growth of Treg-inducing bacteria *Lactobacillus murinus* (*L. murinus*) [5]. Thus, blocking Dectin-1 can suppress development of colitis by expanding *L. murinus* and increasing Treg cells in the intestine [6]. MGL1, another CLR expressed in colonic lamina propria (cLP) macrophages, also ameliorates chemical-induced colitis through induction of IL-10 by binding to *Streptococcus* and *Lactobacillus* species [7].

Dendritic cell immunoreceptor (DCIR; gene symbol Clec4a2) is another CLR family member, and predominantly expressed in myeloid-derived cells such as dendritic cells (DCs) and osteoclasts. Different from Dectin-1, which has an immunoreceptor tyrosine-based activation motif (ITAM), DCIR has an immunoreceptor tyrosine-based inhibitory motif (ITIM) in the cytoplasmic region to negatively control multiple signaling pathways by recruiting protein tyrosine phosphatase SHP-1 [7-12].

The inventors previously reported that aged Clec4a2^{-/-} mice spontaneously develop autoimmune sialadenitis and enthesitis [13, 14]. Clec4a2^{-/-} mice are highly susceptible to autoimmune diseases such as collagen-induced arthritis (CIA) and experimental autoimmune encephalomyelitis (EAE) [13, 15]. DC population is expanded and antigen presenting ability of DC is enhanced in Clec4a2^{-/-} mice, and this enhanced antigen presenting ability is suggested to cause autoimmunity and overresponses to antigen stimulation in Clec4a2^{-/-} mice [50, 13]. Further, DC differentiation from Clec4a2^{-/-} mouse bone marrow cells (BMCs) by granulocyte-macrophage colony-stimulating factor (GM-CSF), known as colony-stimulating factor 2 (CSF2), is enhanced due to excessive phosphorylation of signal transducer and activation of transcription-5 (Stat-5), suggesting that DCIR regulates GM-CSF signaling by suppressing phosphorylation of downstream Stat5 though recruitment of SHP-1 to ITIM [13, 16]. Thus, it seems likely that DCIR is also involved in intestinal inflammation and anti-tumor immunity in the intestine. However, the involvement of DCIR in the development of intestinal inflammation and tumor development remains to be elucidated.

In this study, the roles of DCIR in the development of dextran sodium sulfate (DSS)-induced colitis and azoxymethane (AOM)-DSS-induced colon tumor in mice were examined. DSS-induced colitis is an animal disease model for ulcerative colitis and Crohn's disease in humans. AOM-DSS-induced tumor is a model for colorectal cancer, in which polyps are induced in the colon by inducing mutations in protooncogenes such as K-ras and β-Catenin genes, and in which tumorigenesis is promoted by DSS-induced inflammation. As a result, it was found that Clec4a2^{-/-} mice were less susceptible to DSS-induced colitis compared with wild type (WT) mice, accompanied with reduced expression of proinflammatory cytokines and chemokines, and that commensal microbiota is not involved in these phenomena. Development of AOM-DSS-induced colon tumors was also reduced in Clec4a2^{-/-} mice compared with WT mice, accompanied with an increase in Csf2 expression in polyps. Furthermore, administration of an anti-asialo-biantennary N-glycan (NA2) antibody suppressed development of colitis and colorectal cancer. These results suggest that NA2, which is a DCIR ligand, may be utilized as a target for the treatment and prevention of diseases that develop by the action of DCIR.

### 2. Materials and Methods

### 2.1 Mice

Clec4a2^{-/-} mice were generated using homologous recombination techniques and backcrossed to BALB/cA (Japan SLC, Shizuoka, Japan) for 12 generations [1]. WT and Clec4a2^{-/-} male mice with similar ages were used. These mice were housed under specific pathogen-free conditions in environmentally controlled clean rooms at the Research Institute for Biomedical Sciences, Tokyo University of Science. All animal experiments were approved by the institutional animal use committees and were conducted according to the ethics guidelines for animal experiments and safety guidelines for gene manipulation experiments.

### 2.2 DSS-induced colitis and AOM-DSS-induced tumor

DSS-induced colitis was induced by the following procedure. Clec4a2^{-/-} mice and WT mice around 10 weeks old, in separated or co-housed groups (co-housed after being weaned), were treated with 4% DSS (36 to 50 kDa; MP Biomedicals, Illkirch, France) in drinking water for 7 days, and then were treated with normal water for recovery for 5 days.

Diarrhea and bloody stool were scored once every two days as follows.
[Diarrhea] 0: normal, 1: slightly loose stool, 2: loose stool, 3 slightly watery diarrhea, 4: watery diarrhea.
[Bloody stool] 0: normal, 2: slight bleeding, 3: moderate bleeding, 4: gross bleeding. Weight loss was scored as follows.
[Weight loss] 0: none, 1: loss of more than 0% to 5%, 2: loss of more than 5% to 10%, 3: loss of more than 10% to 15%, 4: loss of more than 15%.

Disease activity index was calculated as the average of these scores. Disease activity index was calculated as (the sum of scores of diarrhea, bleeding and weight loss)/3.

During the 4% DSS-induced colitis in Clec4a2^{-/-} mice and WT mice, PBS and rmGM-CSF (Petrotech, USA) (1 µg) were administered intraperitoneally on days 0, 1, 2, and 3.

WT mice were administered IgG2a isotype control (Bio X Cell, USA, clone: 2A3) and an anti-GM-CSF (Bio X Cell, USA, clone: MP1-22E9) neutralizing antibody (100 µg/mouse) intraperitoneally on days 0, 1, 2, 3, 4, 5, and 6.

Anti-NA2 antibody (clone: 633_1G3, 200 µg/mouse) and an isotype antibody (IgM, clone: MM-3, 200 µg /mouse) were administered to WT mice intraperitoneally on days 1, 2 and 5.

On day 12 after the DSS administration started, mice were sacrificed and histopathological analysis was carried out. Colon sections were washed with PBS and were fixed with 10% neutral buffered formalin and embedded in paraffin. After being cut in slices, tissue sections were stained with haematoxylin and eosin.

The anti-NA2 antibody was prepared by immunizing a galactosyltransferase-1 (GalT1)-deficient mouse with NA2 according to the following procedure. The NA2 used for immunization is as follows:
Product name: GT-11157
Company name: GlyTech, Inc.
Structure: NA2 glycan is bound to BSA via a maleimide bond, and 15 to 25 molecules of NA2 are bound to one molecule of BSA.

Immunization was performed as follows. NA2-BSA (1 mg/ml) was mixed with an adjuvant, Titer Max Gold (registered trademark, TiterMax), at a ratio of 1:1, and 400 µL per GalT1-deficient mouse was subcutaneously inoculated, followed by a boost inoculation of 200 µL four weeks later. Two weeks later, the mice were sacrificed, and the spleen cells were fused with myeloma (NS-1) cells to produce a hybridoma. A hybridoma monoclonal library was produced by limiting dilution, and an NA2-specific antibody-producing clone (633) was identified by the method described below. Further, to establish a single clone, cloning was performed again to establish the 633_1G3 clone. The CDR sequences of clones 633 and 633_1G3 were obtained (FIG. 10), and since 633 and 633_1G3 have the same sequence, they were recognized as monoclonal antibodies.

The 633_1G3 hybridoma was transplanted into a nude mouse pre-stimulated with pristane, and the mouse was sacrificed and the peritoneal fluid was collected. Since 633_1G3 is an IgM antibody, the antibody was purified using CaptoL (Sigma Aldrich), protein L-modified agarose beads.

Identification of anti-NA2 antibodies was performed by confirming the binding inhibitory ability of DCIR-Fc to NA2 by the following procedure. NA2-OVA (GlyTech, Inc., GT-11204) or OVA (10 µg / ml) was added to the bottom of a 96-well plate and allowed to bind to the plate by standing overnight at 4 °C. Blocking was performed with PBS containing 10% FCS, and Fc or DCIR-Fc (5 µg / ml) and culture medium from the anti-NA2 antibody-producing hybridoma were added. After the plate was allowed to stand at room temperature for 1 hour and washed, Fc or DCIR-Fc associated with the plate was detected by Peroxidase-conjugated anti-human Fc (Jackson Immunoresearch) (FIG. 9A). The graph shows mean ± standard deviation of triplicates. Unless otherwise specified, DCIR-Fc refers to mouse DCIR-Fc in Example 1.

Binding of the extracellular domain of the DCIR-human Fc fusion protein to NA2-OVA was significantly inhibited by the anti-NA2 antibody (633 clone; 633_1G3 was cloned again from this clone and has the same BCR VDJ sequence).

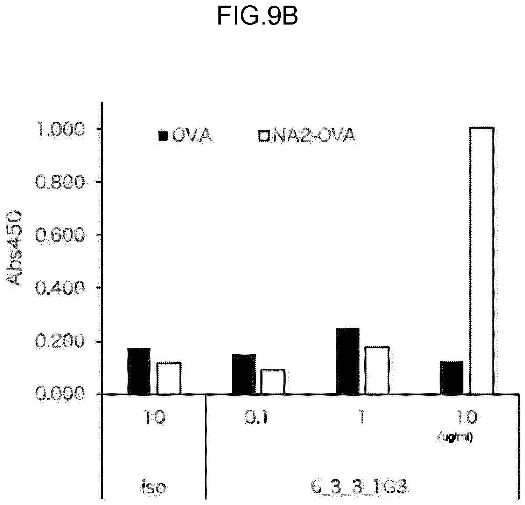

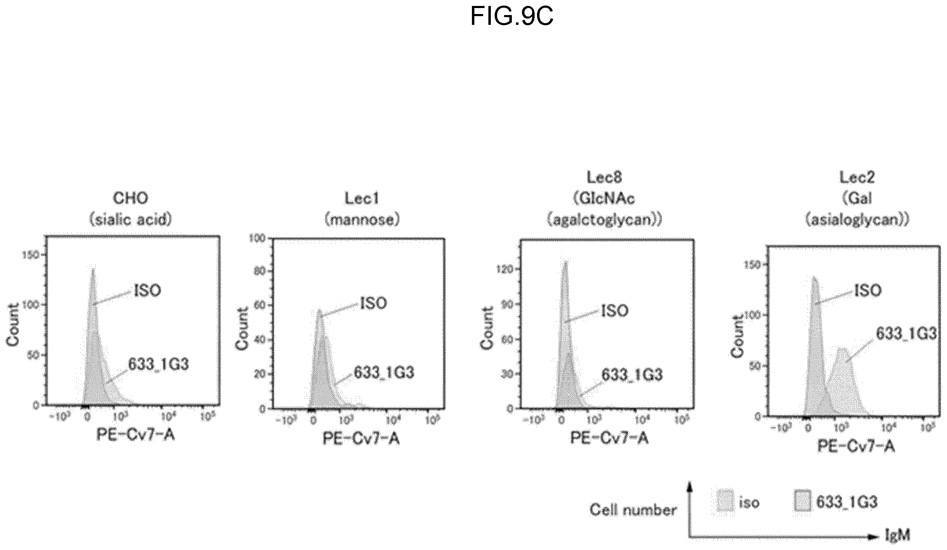

Binding ability of the anti-NA2 antibody to NA2 was confirmed by the following procedure: Using the 633_1 G3 antibody, binding ability to NA2 was confirmed by ELISA (FIG. 9B) and by staining of asialoglycan-expressing LEC2 cell line (FIG. 9C).

ELISA was performed by the following procedure. OVA or NA2-OVA (10 µg/ml) was coated on a 96-well plate and allowed to stand overnight at 4°C to coat the plate with the antigen. Thereafter, the plate was washed and blocked with 10% FC-containing PBS at room temperature for 1 hour. Subsequently, the plate was washed, and an isotype antibody (iso) (10 µg/ml (10)) or the 633_1G3 antibody (0.1 µg/ml (0.1), 1 µg/ml (1) or 5 µg/ml (5)) was added thereto, and the plate was allowed to stand at room temperature for 1 hour. After the plate is washed, IgM antibody was detected using Peroxidase-conjugated anti-Mouse IgM (Jackson Immunoresearch). The graph shows the average of the wells performed in triplicate. The result is representative of two or more independent experiments.

Staining of the asialoglycan-expressing LEC2 cell line was performed by the following procedure. Binding of the anti-NA antibody (633_1G3) to CHO cells and their glycosylation enzyme mutants Led (expressing high mannose-type glycan), Lec8 (expressing agalactoglycan), and Lec2 (expressing asialoglycan) was analyzed. An isotype antibody (iso) or the anti-NA antibody (633_1G3) was added to each cell (10 µg/ml), and the cells were left to stand on ice for 30 minutes. After washing, PE/Cy7-conjugated anti-mouse IgM antibody (Biolegend) was added and the cells were left to stand on ice for another 20 minutes. The cells were washed and analyzed with FACSCanto II.

RNA was purified from the 633_1G3 clone hybridoma cells, cDNA was prepared, and DNA sequence analysis of FR1 to FR4 was performed. The amino acid sequence of 633_1G3 and the analyzed DNA sequence are shown in FIG. 10. In FIG. 10, the upper row shows the sequence of the heavy chain (VH), and the lower row shows the sequence of the light chain (VL).

AOM-DSS-induced tumor was induced by the following procedure. 7 to 8 week old female mice, which were weaned at 4 weeks of age and then housed together or separated, were injected with AOM (1 mg/ml) according to body weight (10 mg/kg), and one week later, treated with 2% DSS for one week, followed by treatment with normal water. Three cycles of the foregoing treatment of DSS and water were followed by treatment with water for 8 weeks. Body weight was measured every three days. WT mice were intraperitoneally administered with the anti-NA2 antibody (clone: 633_1G3, 200 µg/mouse) and the isotype antibody (IgM, clone: MM-3, 200 µg/mouse).

### 2.3 Western blot analysis

On day 12 after the DSS and normal water treatment, total protein was extracted from mouse colon using NP40 lysis buffer (Invitrogen, USA) and was treated with phosphorylation inhibitor (Roche, Switzerland). Total protein from polyps (2 to 3 polyps in each group) and non-polyps (tissue around the polyps) was extracted in a similar manner to the foregoing. 10⁶ colon cells were treated rmGM-CSF (0, 1, 10 ng/ml) for 1 hour, and protein was harvested after the stimulation. An anti-mouse Stat5 (D2O6Y) rabbit antibody, an anti-mouse phospho-Stat5 (Try694) rabbit antibody (Cell Signaling Technology), and an anti-rabbit IgG (H+L) antibody (ThermoFisher, USA) were used for the blotting.

### 2.4 Quantitative real-time RT-PCR and RNA-sequencing (RNA-seq) method

cDNA of the flora is extracted from the feces of separately raised mice usgin a QIAmp DNA stool mini kit (QIAGEN, USA) . Total mRNA was extracted from the colon of the colitis model, or 2 to 3 polyps and non-polyps, using a GenElute mammalian RNA miniprep kit (Sigma-Aldrich, St. Louis, USA). After pooling the same amount of RNA from each sample, RNA-seq was performed. The isolated RNA was subj ected to reverse transcription using a high-capacity cDNA reverse transcription kit (Applied Biosystems, Foster City, USA). Messenger RNA expression of indicated genes and the expression of different bacteria were determined by real-time quantitative PCR using the SYBR premix Ex Taq (Takara Bio, Otsu, Japan) on an iCycler system (Bio-Rad, Hercules, USA). The relative value of each gene was calculated by the comparative Ct method and was normalized with Gapdh expression level. The relative value of each bacteria was calculated by the comparative Ct method and was normalized with 16S expression level. Tables 1A and 1B are lists of primers.

**[Table 1A]**

| Gene or bacterial species | Forward sequence | Reverse sequence |
|---|---|---|
| Gapdh | TTCACCACCATGGAGAAGGC (SEQ ID NO: 1) | GGCATGGACTGTGGTCATGA (SEQ ID NO: 2) |
| Il17a | TTTAACTCCCTTGGCGCAAAA (SEQ ID NO: 3) | CTTTCCCTCCGCATTGACAC (SEQ ID NO: 4) |
| Il17f | TGCTACTGTTGATGTTGGGAC (SEQ ID NO: 5) | AATGCCCTGGTTTTGGTTGAA (SEQ ID NO: 6) |
| Tnfa | GCCTCCCTCTCATCAGTTCT (SEQ ID NO: 7) | CACTTGGTGGTTTGCTACGA (SEQ ID NO: 8) |
| Stat5a | GGTCCCCTGTGAGCCCGCAAC (SEQ ID NO: 9) | TGACTGTGCGTGAGGGATCCACTGACT (SEQ ID NO: 10) |
| Il6 | GAGGATACCACTCCCAACAGACC (SEQ ID NO: 11) | AAGTGCATCATCGTTGTTCATACA (SEQ ID NO: 12) |
| Il1a | TCCAGGGCAGAGAGGGAGT (SEQ ID NO: 13) | GGAACTTTGGCCATCTTGATTT (SEQ ID NO: 14) |
| Il1b | CAACCAACAAGTGATATTCTCCATG (SEQ ID NO: 15) | GATCCACACTCTCCAGCTGCA (SEQ ID NO: 16) |
| Cxcl2 | AATGCCCTGGTTTTGGTTGAA (SEQ ID NO: 17) | AATGCCCTGGTTTTGGTTGAA (SEQ ID NO: 18) |
| Csf2 | GGCCTTGGAAGCATGTAGAGG (SEQ ID NO: 19) | GGAGAACTCGTTAGAGACGACTT (SEQ ID NO: 20) |
| Foxp3 | AGAAGCTGGGAGCTATGCAG (SEQ ID NO: 21) | TACTGGTGGCTACGATGCAG (SEQ ID NO: 22) |
| Tgfb | TGACGTCACTGGAGTTGTACGG (SEQ ID NO: 23) | GGTTCATGTCATGGATGGTGC (SEQ ID NO: 24) |
| Tbet | GGTGTCTGGGAAGCTGAGAG (SEQ ID NO: 25) | CCACATCCACAAACATCCTG (SEQ ID NO: 26) |
| Zo1 | GCCGCTAAGAGCCACAGCAA (SEQ ID NO: 27) | GCCCTCCTTTTAACACATCAGA (SEQ ID NO: 28) |
| Occludin | TTCCTCTGACCTTGAGTGTGG (SEQ ID NO: 29) | CTCTTGCCCTTTCCTGCTCTTT (SEQ ID NO: 30) |
| Claudin | GTCCTGGGAATCTCCTTGGC (SEQ ID NO: 31) | TCTGTGCCGTGACGATGTTG (SEQ ID NO: 32) |

**[Table 1B]**

| Gene or bacterial species | Forward sequence | Reverse sequence |
|---|---|---|
| 16s (8F&R357) | AGAGTTTGATCMTGGCTCAG (SEQ ID NO: 33) | CTGCTGCCTYCCGTA (SEQ ID NO: 34) |
| Tenericute | ATGTGTAGCGGTAAAATGCGTAA (SEQ ID NO: 35) | CMTACTTGCGTAGGTACTACT (SEQ ID NO: 36) |
| Firmicutes | TGAAACTYAAAGGAATTGACG (SEQ ID NO: 37) | ACCATGCACCACCTGTC (SEQ ID NO: 38) |
| ε-proteobacteria | TAGGCTTGACATTGATAGAATC (SEQ ID NO: 39) | CTTACGAAGGCAGTCTCCTTA (SEQ ID NO: 40) |
| Actinobacteria | TACGGCCGCAAGGCTA (SEQ ID NO: 41) | TCRTCCCCACCTTCCTCCG (SEQ ID NO: 42) |
| α-proteobacteria | CIAGTGTAGAGGTGAAATT (SEQ ID NO: 43) | CCCCGTCAATTCCTTTGAGTT (SEQ ID NO: 44) |
| Clostridium | CACCAAGGCGACGATCAGT (SEQ ID NO: 45) | GAGTTTGGGCCGTGTCTCA (SEQ ID NO: 46) |
| γ-proteobacteria | TCGTCAGCTCGTGTYGTGA (SEQ ID NO: 47) | CGTAAGGGCCATGATG (SEQ ID NO: 48) |
| Lactobacillus | TGGAAACAGRTGCTAATACCG (SEQ ID NO: 49) | GTCCATTGTGGAAGATTCCC (SEQ ID NO: 50) |

### 2.5 Preparation of colon or tumor cells

Mouse colon or 2 to 3 tumors (2 to 3mm) were washed with phosphate buffered saline (PBS) and cut into 2 mm slices, which were then shaken in Hanks Balanced Salt Solution (HBSS) containing 3 mM ethylenediaminetetraacetic acid (EDTA) at 37°C for 30 min. Then, the slices were suspended in RPMI (Life Technologies/Invitrogen, Carlsbad, CA, USA) with 10% fetal calf serum (FCS) (Life Technologies), 400 U/ml collagenase (C2139; Sigma-Aldrich), and 5 U/ml DNase 1 (Sigma-Aldrich), and shaken at 37°C for 120 min. The samples were vortexed for 30 seconds after final incubation, and filtered through sterile gauze to harvest a single cell suspension. Epithelial cell adhesion molecule (EpCAM), CD4, CD8, CD49b, and CD19 microbeads (BD, USA) were incubated for 20 minutes, washed twice with MACS (registered trademark) buffer, and then MACS (registered trademark) treatment was performed to sort negative cells. CD11b microbeads (BD, USA) were incubated for 20 min, washed twice by MACS (registered trademark) buffer, and then MACS (registered trademark) treatment was performed to sort negative cells. For flow cytometry, cells were washed twice with FACS Hanks buffer (HBSS containing 2% FCS and 0.1% sodium azide) and then were treated with 2.4G2 to block FcR binding. Cells were then surface-stained with the following 250x diluted fluorescence-conjugated antibodies: anti-mouse CD11b (M1/70), CD45 (30-F11), Ly6C (HK1.4), Ly6G (1A8), TCR-β (H57-597), CD3 (17A2), IL17A(TC11-18H10), Foxp3 (MF-14), IFN-γ (XMG1.2), GM-CSF (MP1-22E9), CD4 (GK1.5), CD8 (53-6.7), CD206 (C068C2), Nos2 (W16030C), RORyt (B2D), CD90.2 (30-H12), GATA3 (PC61), Gr1 (RB6-8C) and the like. All the antibodies were obtained from BioLegend (San Diego, CA, USA). 7-amino-actinomycin D (7-AAD) was added to all the samples before applying to distinguish dead cells. Cells were acquired with FACSCanto II (BD Biosciences) and data were analyzed using FlowJo 10.0 software (Tree Star, Ashland, OR, USA).

### 2.6 Statistical Analysis

Differences in parametric data were evaluated by Student's t-test. Statistics were computed with the PRISM7 software (GraphPad Software), and a P value of < 0.05 was considered to be statistically significant.

### 3. Results

### 3.1 Clec4a2^{-/-} mice are refractory to DSS-induced colitis independently of the changes in intestinal flora.

In order to elucidate the role of DCIR in the development of colitis, the expression of the DCIR gene in the inflammatory site of colitis was examined first. To measure the expression of DCIR in the inflammatory site of DSS-induced colitis, mice were administered water containing 4% DSS for 7 days to induce colitis. After that, the mice were raised with normal drinking water until day 12, and then sacrificed and analyzed. In the colon of mice that developed colitis, the expression of the Clec4a2 gene was enhanced compared to the colon of healthy mice (FIG. 1A). In addition, when the expression of the human DCIR gene was examined from the NCBI public GEO database, the expression of the DCIR gene was enhanced in the colon of Crohn's disease and ulcerative colitis compared to healthy people (FIG. 1A-2). Weight loss and colitis severity scores in Clec4a2^{-/-} mice were milder than those in WT mice both during the DSS administration and during the recovery period (FIG. 1B). Inflammation-induced colon shortening was attenuated in Clec4a2^{-/-} mice compared with WT mice (FIG. 1C). Inflammatory cell infiltration in the colon of Clec4a2^{-/-} mice was milder than that in WT mice, while no significant difference was observed in colonic epithelial cell damage between Clec4a2^{-/-} and WT mice (FIG. 1D).

Next, to examine the possible influence of commensal microbiota on this DCIR function, Clec4a2^{-/-} mice and WT mice were co-housed for 4 weeks after weening. Then, the mice were allowed to freely drink water containing 4% DSS under the cohabitation condition. As a result, Clec4a2^{-/-} mice showed milder symptoms caused by DSS, such as weight loss, diarrhea and bloody stool, shortening of the colon, and infiltration of inflammatory cells, than WT mice (FIGs. 1E to G). Further, nucleic acid was extracted from the feces of the mice before and after the 4-week cohousing, and the composition of representative bacterial species in the intestinal flora was measured by real-time PCR of 16SrRNA sequences. The results are shown in FIG. 1H and FIG. 1I, respectively. As a result, it was found that the compositions of representative bacterial species in the intestinal flora in Clec4a2^{-/-} mice and WT mice were different before the cohousing, and the composition became very similar after the cohousing. These results indicate that Clec4a2^{-/-} mice are refractory to DSS-induced colitis in a commensal microbiota-independent manner.

### 3.2 Inflammatory cell infiltration, but not regulatory T (Treg) cell infiltration, is reduced in Clec4a2^{-/-} mice after DSS treatment.

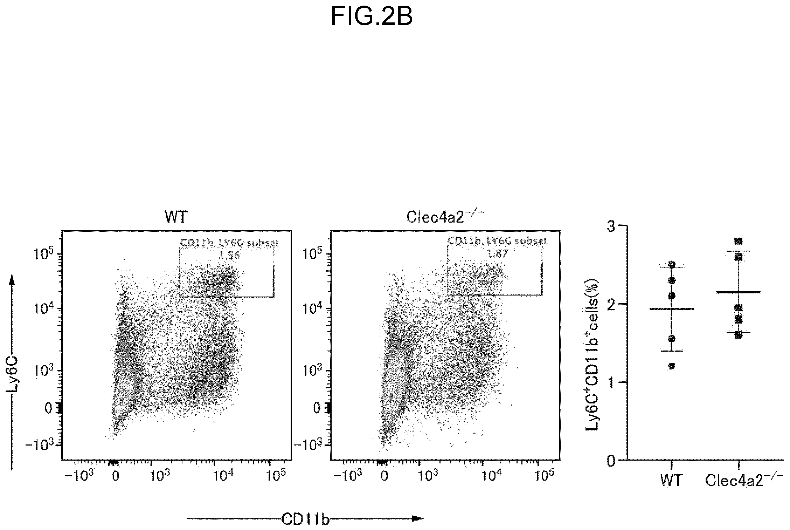

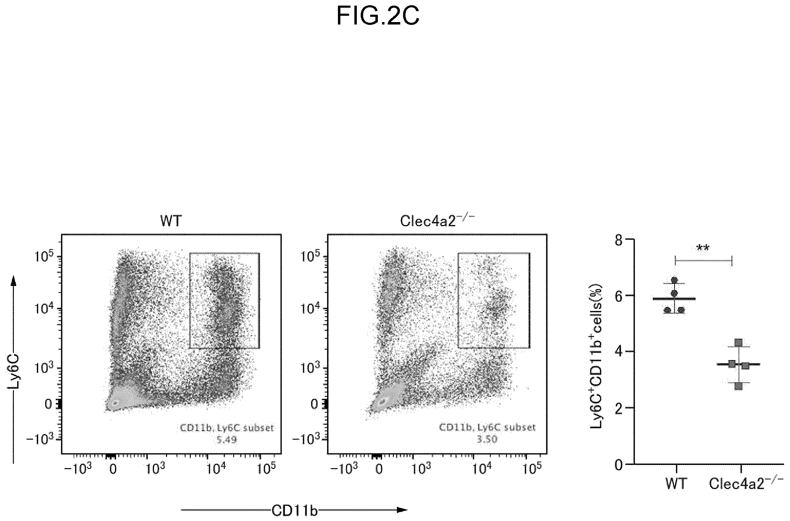

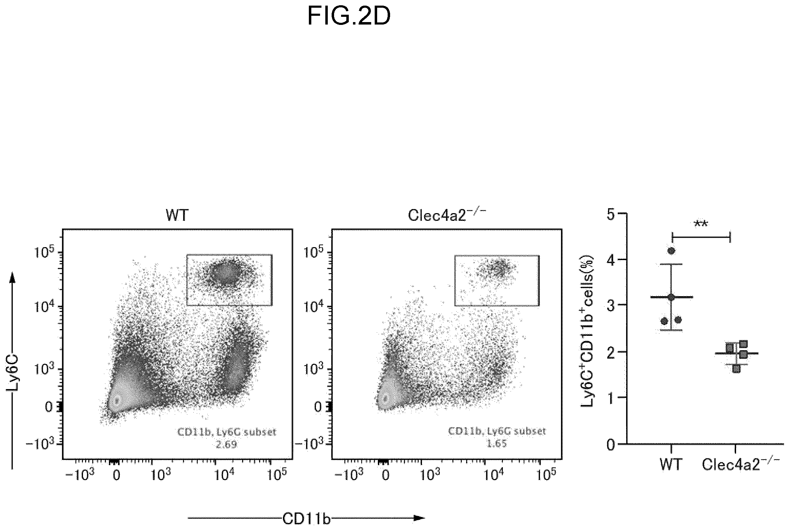

To know the mechanisms of amelioration of colitis in Clec4a2^{-/-} mice, proportions of infiltrating cells in the colonic lamina propria (cLP) were examined. Before the DSS treatment, the proportions of inflammatory cells such as Ly6C⁺ monocytes and Ly6G⁺ neutrophils were similar between Clec4a2^{-/-} and WT mice (FIGs. 2A and B). However, after the 4% DSS treatment, the proportions of these cells were significantly increased in WT mice, whereas the proportions of these cells were not increased in Clec4a2^{-/-} mice (FIGs. 2C and D). This suggests that the increase in these inflammatory cells causes inflammation.

Since Treg cells play an important role in homeostasis of the intestinal immune system and suppress the development of DSS-induced colitis [6], Treg cell population after the DSS-induced colitis was also examined. As a result, it was found that, on day 12 after the DSS treatment, the proportions of CD4⁺T cells, CD8⁺T cells, and CD4⁺Foxp3⁺T cells in cLP CD45⁺ cells were similar between WT and Clec4a2^{-/-} mice (FIG. 2E). The expression levels of Foxp3, Tgfb and Tbx21 in the colon were also similar between Clec4a2^{-/-} mice and WT mice (FIG. 2F). These results suggest that cLP Treg cell population as well as Th1 and Th2 cell populations are not affected by the DCIR deficiency in Clec4a2^{-/-} mice after the induction of DSS-induced colitis.

### 3.3 Expression of inflammatory cytokines and chemokines such as Il17a and Cxcl2 is reduced in Clec4a2^{-/-} mice.

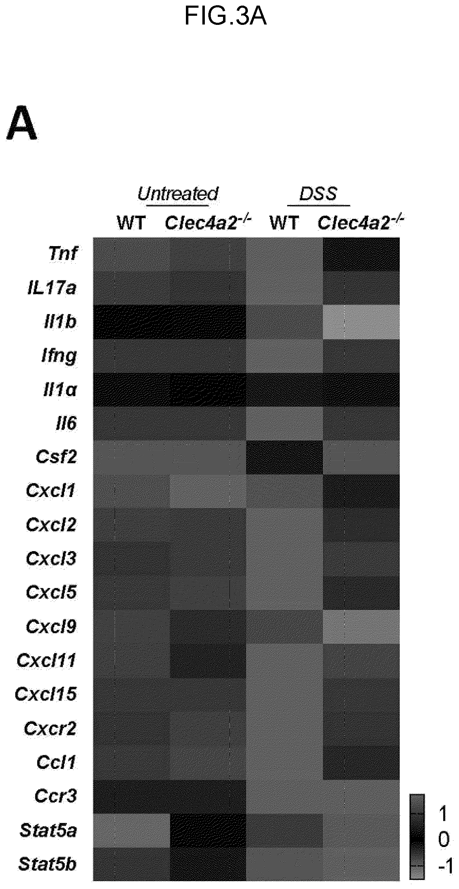

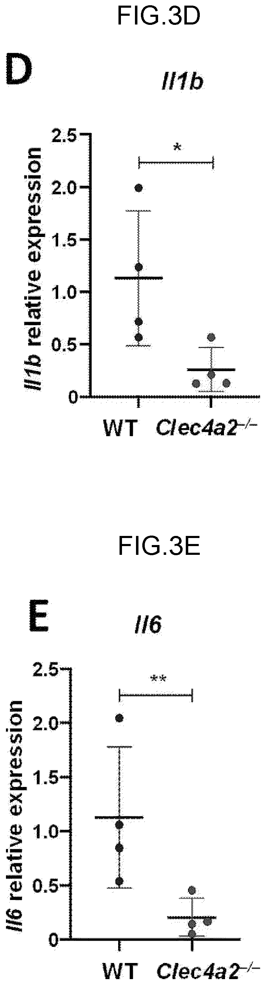

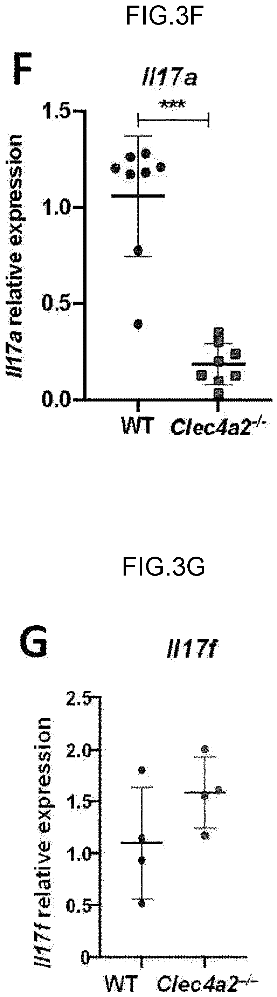

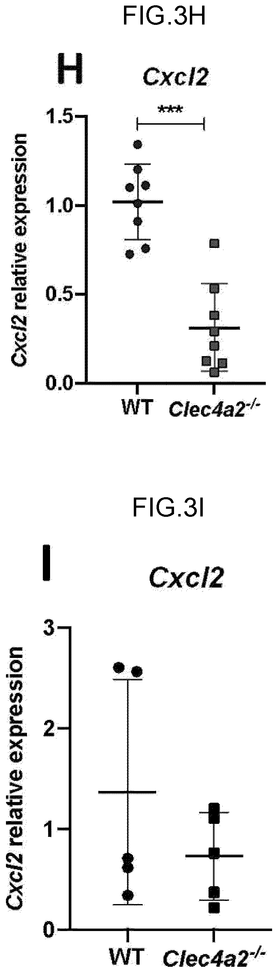

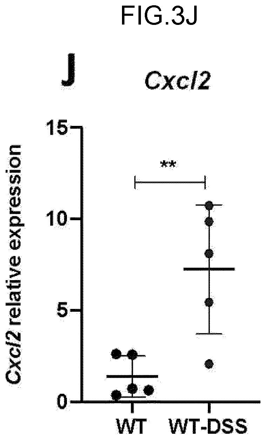

Next, gene expression in the colon on day 12 after the induction of DSS-induced colitis was examined by RNA-seq analysis (FIG. 3A). Compared with WT mice, the expression of inflammatory cytokines such as Tnf, Il6, Il1b, Il1a, Ifn-g was greatly reduced. Il17f was not reduced. The expression of Csf2 was enhanced in Clec4a2^{-/-} mice after the DSS treatment, whereas it was greatly decreased in WT mice. Before the DSS treatment, the expression of these genes was similar between WT and Clec4a2^{-/-} mice. Furthermore, the expression of chemokines and their receptors such as Cxcl2, Cxcl5, Cxcl9, Cxcl11, Cxcl15, Cxcr2, Cel1, and Ccr3 was decreased in Clec4a2^{-/-} mice compared to WT mice. It was also found that the expression of transcription factors such as Stat5a and Stat5b was increased in Clec4a2^{-/-} mice.

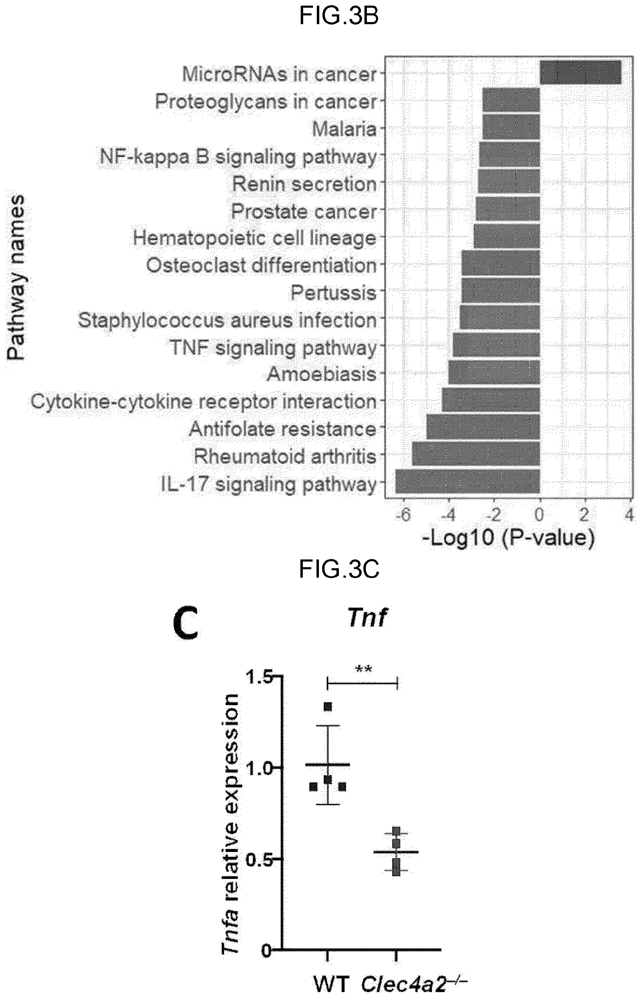

A pathway analysis of the RNA-seq data revealed that pathways related to inflammation and immune responses, including NF-κB, TNF and IL-17 signaling pathways, were downregulated in the colons of Clec4a2^{-/-} mice (FIG. 3B).

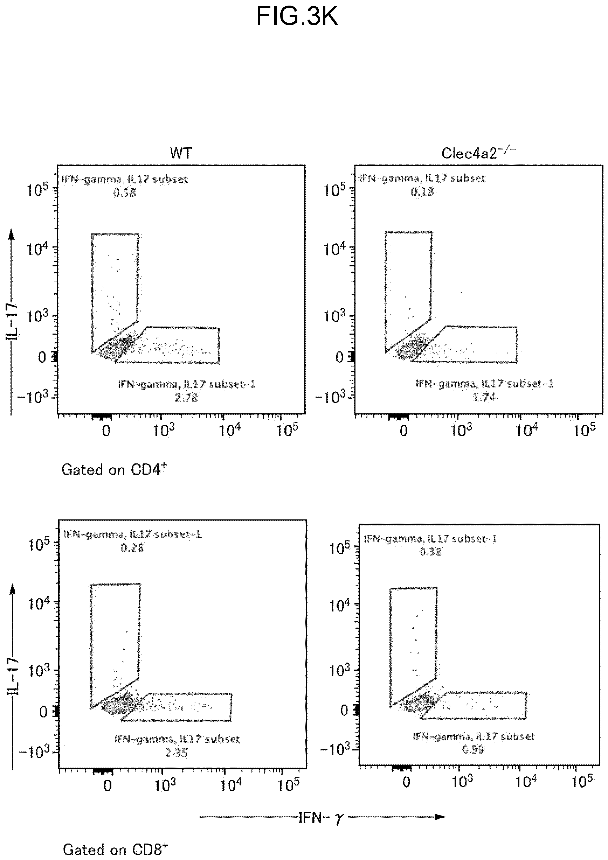

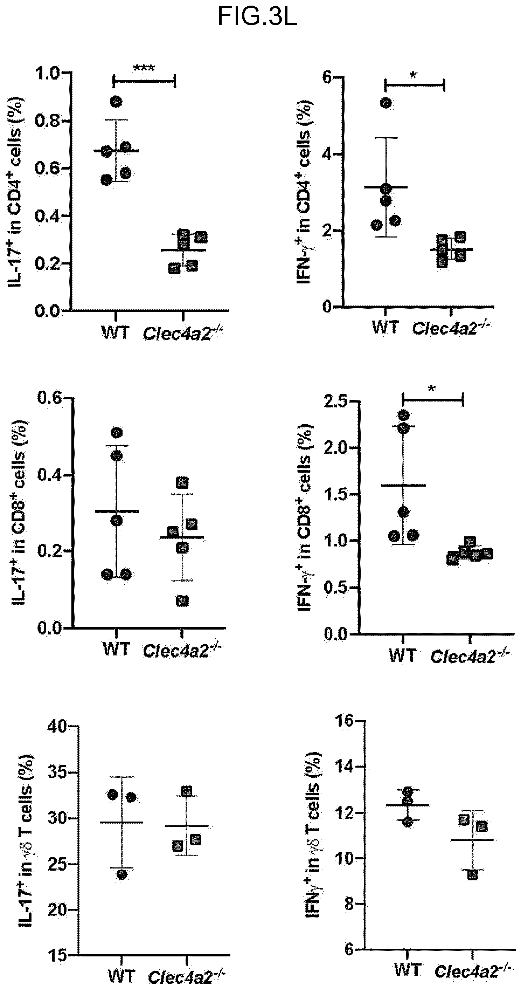

Then, the expression of cytokines and chemokines was confirmed by real time RT-PCR. As a result, it was confirmed that the expression of Tnf, Il1b, Il6, and Il17a was reduced in Clec4a2^{-/-} mice compared to those in WT mice (FIGs. 3C to F). FIGs. 3C to J are combined. The expression of Il17f was not reduced in Clec4a2^{-/-} mice (FIG. 3G). The expression of Cxcl2, which is a chemokine, was also greatly reduced in Clec4a2^{-/-} mice, whereas the expression was similar between Clec4a2and WT mice without DSS treatment (FIGs. 3H to J). Next, the cells expressing these cytokines were analyzed. Analysis of IL-17A-expressing cells in cLP CD4⁺ T cells showed that IL-17A from CD4⁺ T cells, but not from CD8⁺ T cells , was decreased in Clec4a2^{-/-} mice (Fig. 3K and 3L). The proportion of IFN-γ-producing cLP-T cells was also decreased in Clec4a2^{-/-} mice (FIGs. 3K and 3L).

### 3.4 DCIR suppresses GM-CSF expression in ILC3 by suppressing TLR-induced Illb expression.

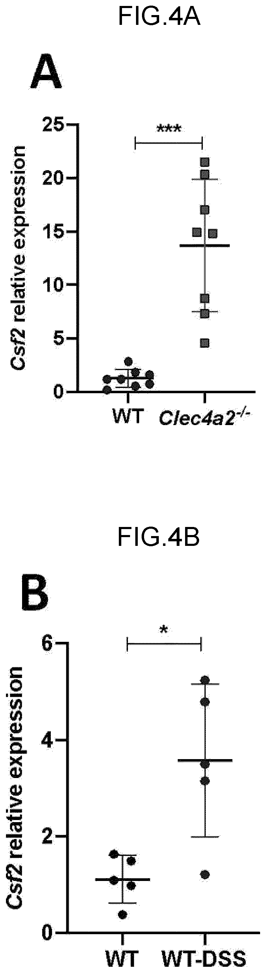

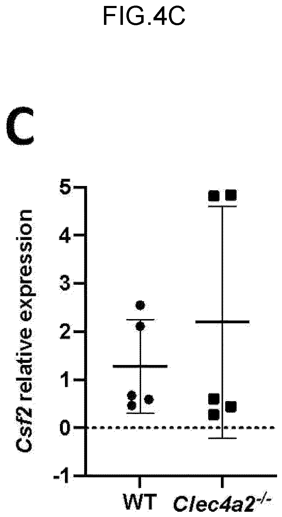

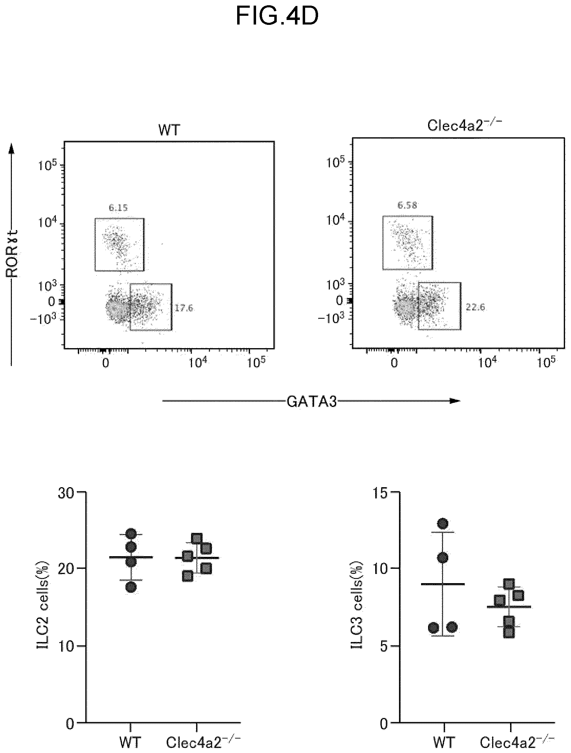

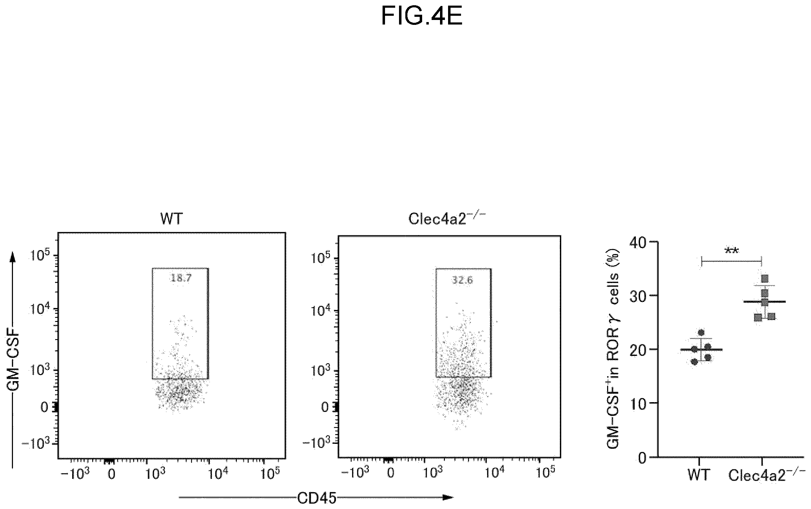

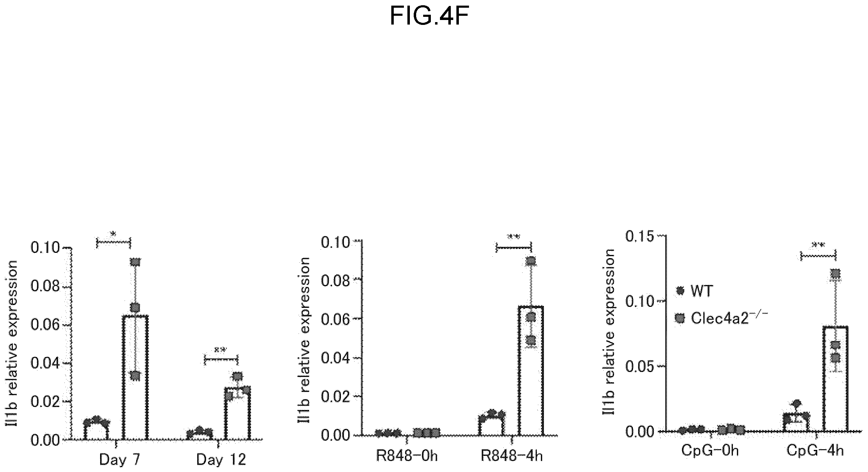

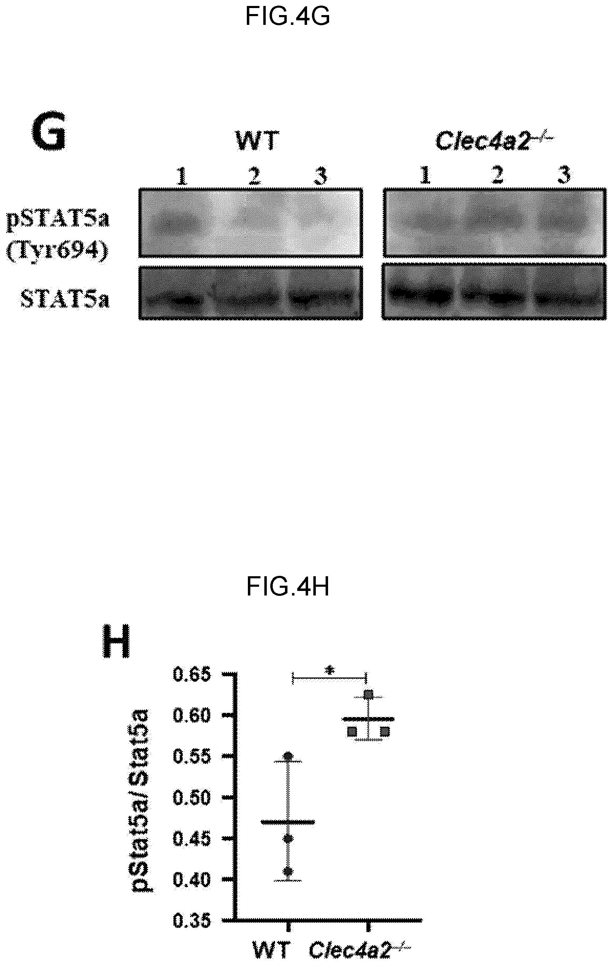

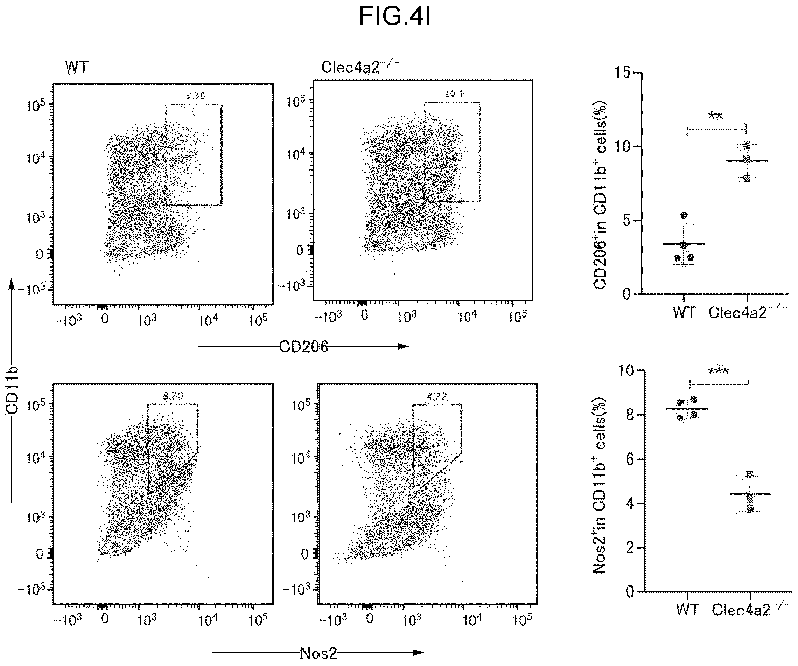

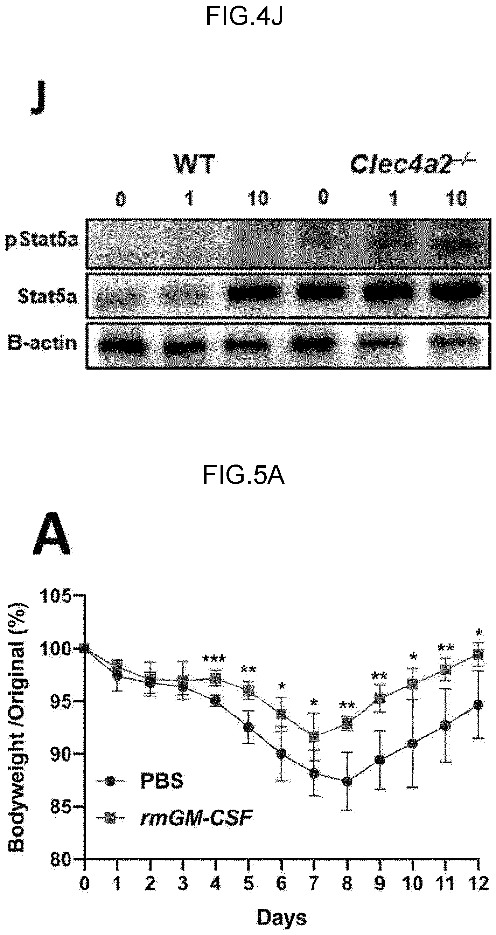

It was found that the expression of Csf2, which encodes GM-CSF, was increased in Clec4a2^{-/-} mice in which colitis was induced compared with WT mice (FIGs. 3A and 4A). The expression of this gene was only augmented after the DSS treatment (FIGs. 4B and 4C). GM-CSF is mainly produced by ILC3 in the colon after DSS treatment [44, 45]. The proportions of GATA⁺ ILC2 cells and RORγt⁺ ILC3 cells were not changed in cLP between WT and Clec4a2^{-/-} mice (FIG. 4D). Compared with WT mice, the proportion of GM-CSF⁺ cells in RORγt⁺ ILC3 cells was increased in the cLP of Clec4a2^{-/-} mice (FIG. 4E). ILC3-derived GM-CSF production was dependent on the ability of macrophages to sense microbial signals and produce Il-1β [45]. DCIR is a negative regulator of innate immune response through TLR8 and TLR9. In macrophages, activation of DCIR inhibits TLR9-mediated Il1b production via phosphorylation of Erk1/2, JNK1/2 and p38 [46-47]. It was found that, compared with WT mice, Il1b relative expression was increased in colonic CD11b⁺ cells of Clec4a2^{-/-} mice after R848 and CpG-ODN stimulation (FIG. 4F). It was found that, although the relative expression of Il1b was decreased in the entire colon, the expression of Il1b was increased in colonic CD11b⁺ cells of Clec4a2^{-/-}mice 7 and 12 days after DSS administration (FIG. 4F). These data suggests that DCIR regulates the production of GM-CSF by ILC3 cells though microbial-mediated TLR signaling pathway. Furthermore, the inventors' previous report showed that GM-CSF signaling is mediated by phosphorylation of Stat5 in bone marrow cells [13]. Consistent with the increase in the GM-CSF expression in Clec4a2^{-/-} mice, Stat5 phosphorylation was enhanced in the colons of the Clec4a2^{-/-} mice (FIGs. 4G and 4H). Treatment of colon cells with recombinant mouse GM-CSF enhanced Stat5 phosphorylation in Clec4a2^{-/-} mice (FIG. 4J). Further, it was found that M2 macrophages were increased and M1 macrophages were decreased in cLP of Clec4a2^{-/-}mice compared to WT mice (FIG. 4I).

### 3.5 GM-CSF suppresses the development of DSS-induced colitis.

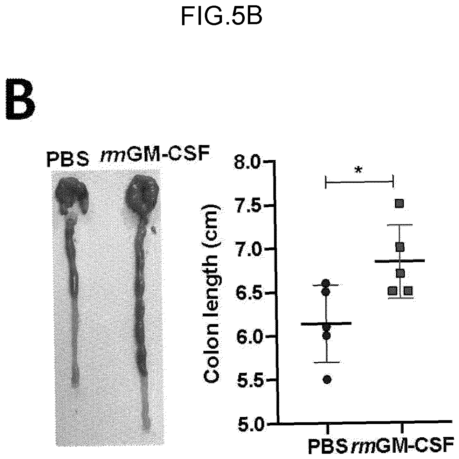

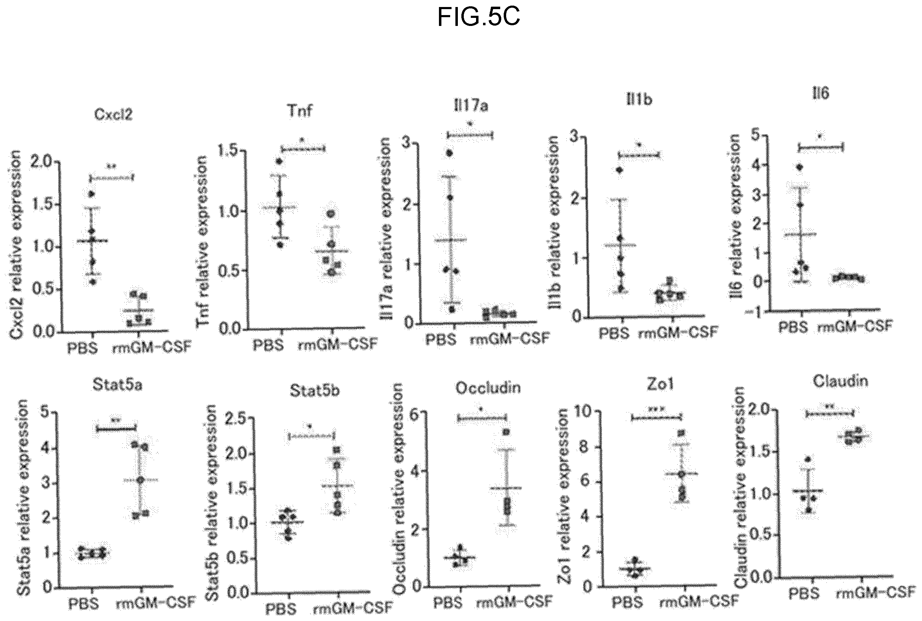

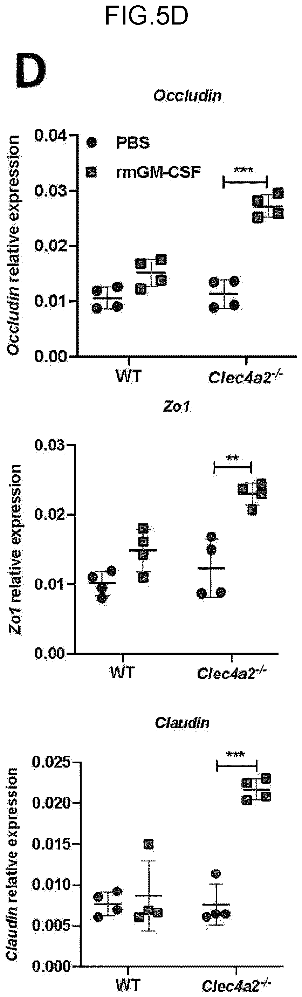

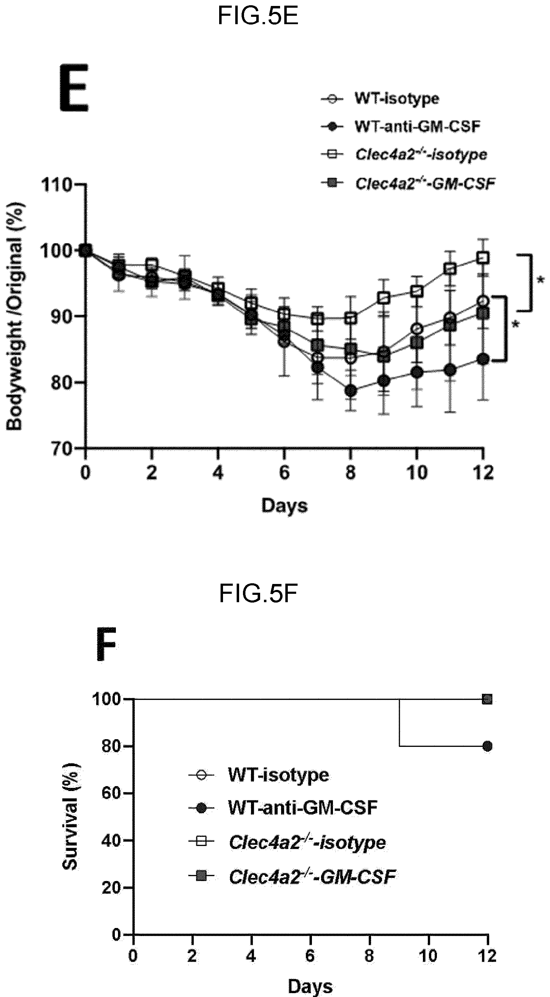

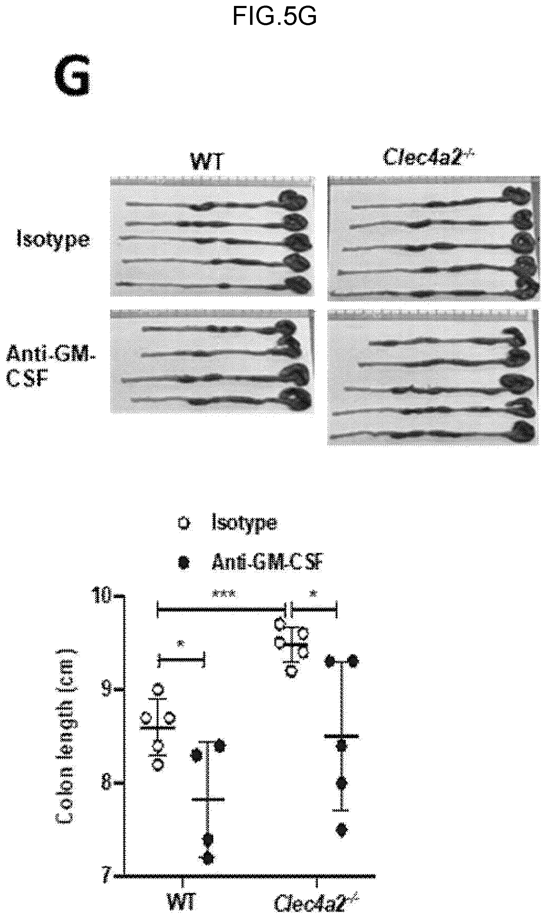

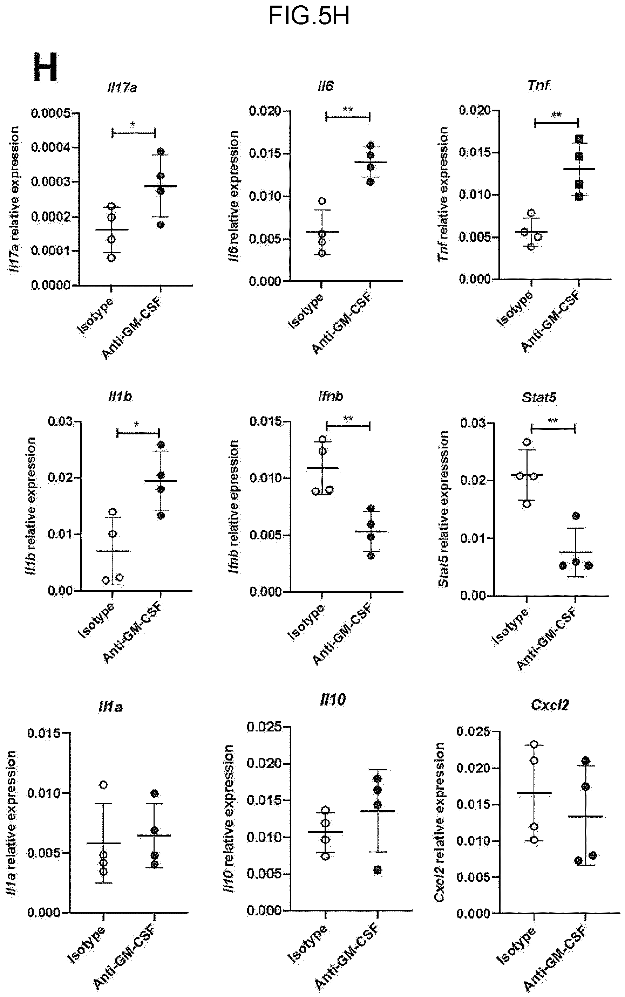

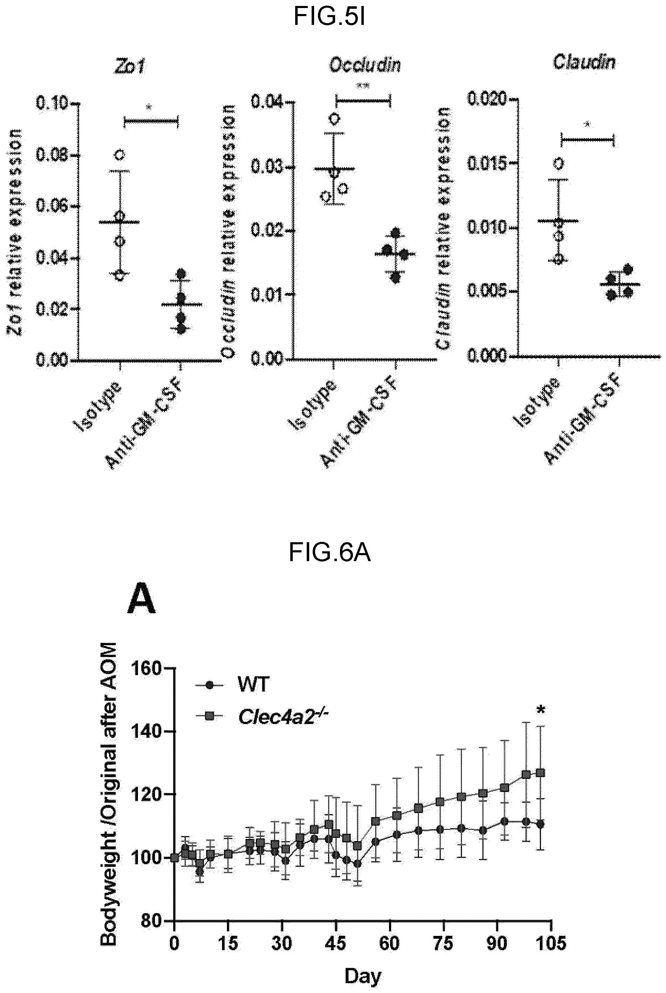

Next, the role of GM-CSF in the development of DSS-induced colitis was examined. WT mice were administered recombinant mouse GM-CSF during the DSS treatment and examined for the development of colitis. As a result, it was found that the GM-CSF administration effectively suppressed the development of DSS-induced colitis, accompanied with enhanced expression of Stat5 and decreased expression of Il17a, Cxcl2, Il6 and Il1b in the colon (FIGs. 5A to C). The expression of tight junctional components, such as occludin, claudin and zo1, was also increased after the GM-CSF administration (FIG. 5C). Furthermore, GM-CSF treatment enhanced occludin, claudin and zo1 expression more efficiently in colon cells from Clec4a2^{-/-} mice compared with cells from WT mice (FIG. 5D). In contrast, administration of an anti-GM-CSF neutralizing antibody aggravated the symptoms of DSS-induced colitis both in WT and Clec4a2^{-/-} mice (FIGs. 5E to G). The expression of Il17a, Il6, and Il1b was increased, while the expression of StatS was decreased, in the colon after treatment with the anti-GM-CSF antibody compared with an isotype control Ig, although Cxcl2 expression was not affected (FIG. 5H). The expression of Occludin, claudin and zo1 was decreased after the administration of the anti-GM-CSF antibody (FIG. 5I). These findings suggest that GM-CSF plays a crucial role in the regulation of colitis development.

### 3.6 Development of colorectal tumor by AOM-DSS is suppressed in Clec4a2^{-/-} mice in a commensal bacterium-independent manner.

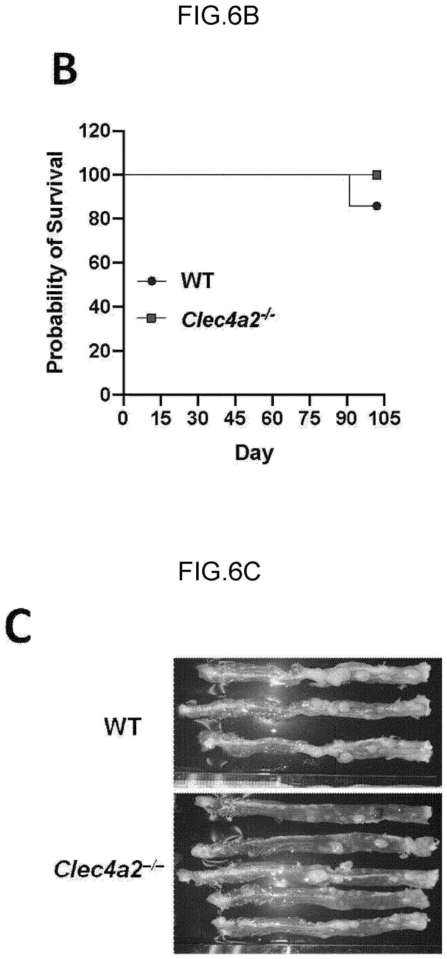

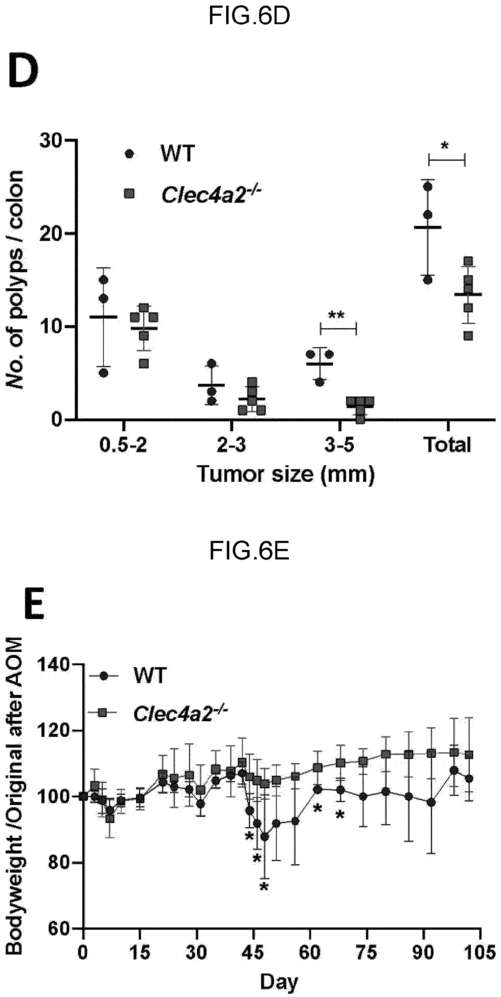

Since DCIR plays an important role in the development of colitis, the effect of Clec4a2-deficiency on tumorigenesis in the colon was further examined. WT and Clec4a2^{-/-}mice were treated with AOM, and after 7 days, these mice were administered 2% DSS-containing water for 1 week and normal water for 2 weeks. After 3 cycles of the DSS-normal water treatment, the mice were raised with normal water for another 6 weeks. As a result, it was found that weight loss is milder in Clec4a2^{-/-} mice as compared to WT mice, while no significant difference in the survival rate was observed (FIGs. 6A and 6B). Development of tumors in the colon was significantly reduced in the Clec4a2^{-/-} mice compared with WT mice (FIGs. 6C and 6D).

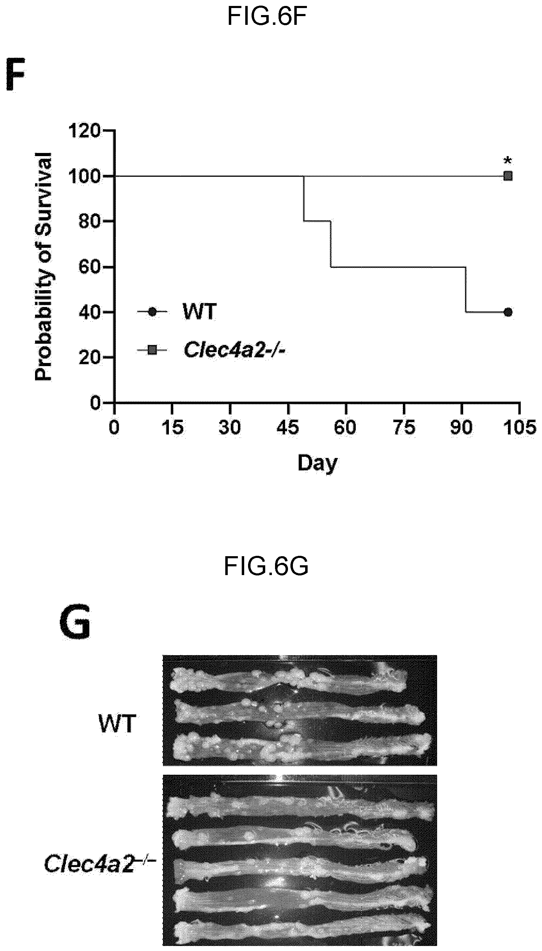

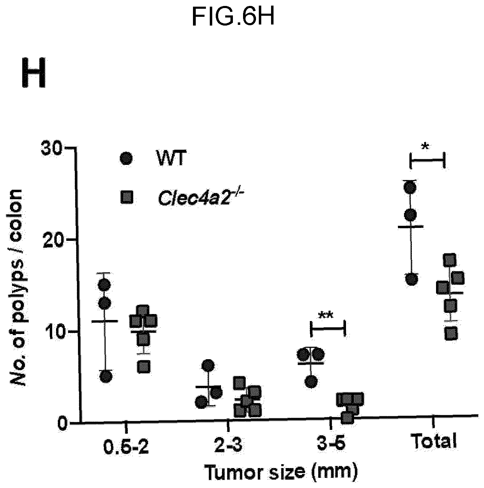

To examine the effect of commensal microbiota, Clec4a2^{-/-} mice were firstly cohoused with WT mice 4 weeks after weaning, and tumors were induced under the co-housing conditions. Similar to the separately housed group, weight loss was milder and the survival rate was significantly higher in Clec4a2^{-/-} mice as compared to WT mice (FIGs. 6E and 6F). Further, tumor development was suppressed in Clec4a2^{-/-} mice compared with WT mice even under the cohousing condition, suggesting that commensal microbiota is not involved in the suppression of tumorigenesis in Clec4a2^{-/-} mice (FIGs. 6G and 6H).

### 3.7 Infiltration of MDSCs and expression of inflammatory cytokines are reduced in Clec4a2^{-/-} mice associated with the reduction of Cxcl2 expression in polyps

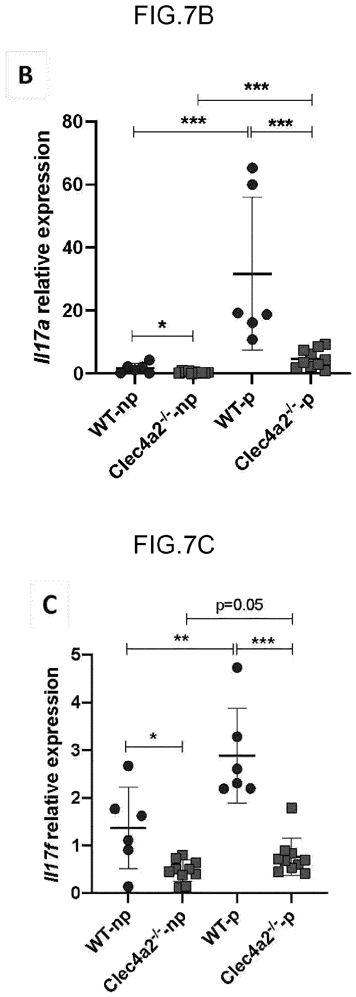

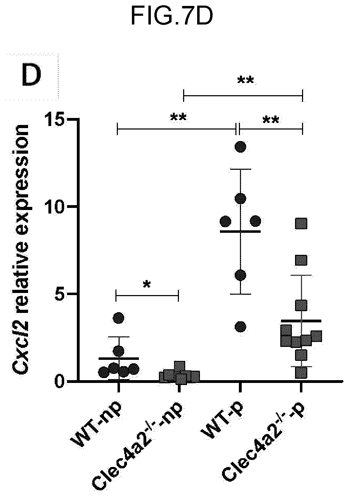

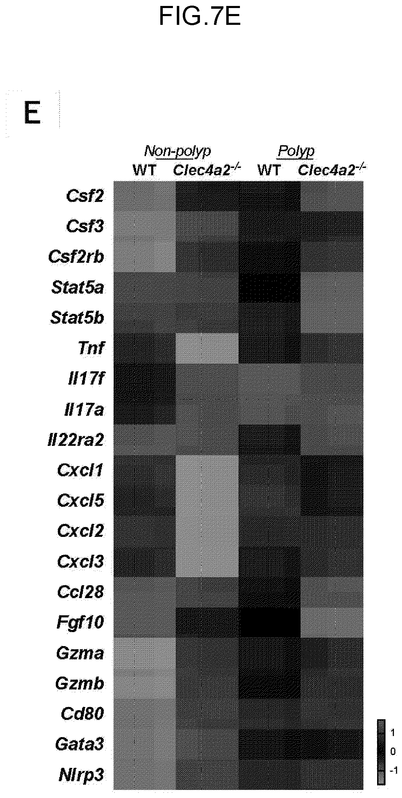

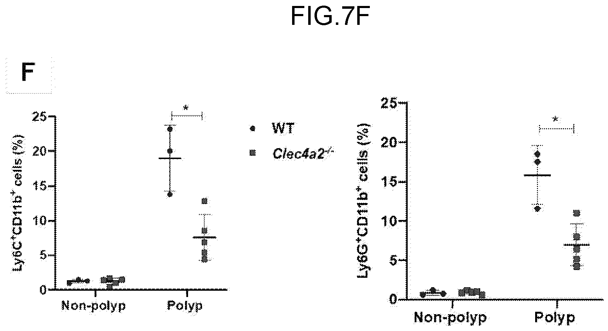

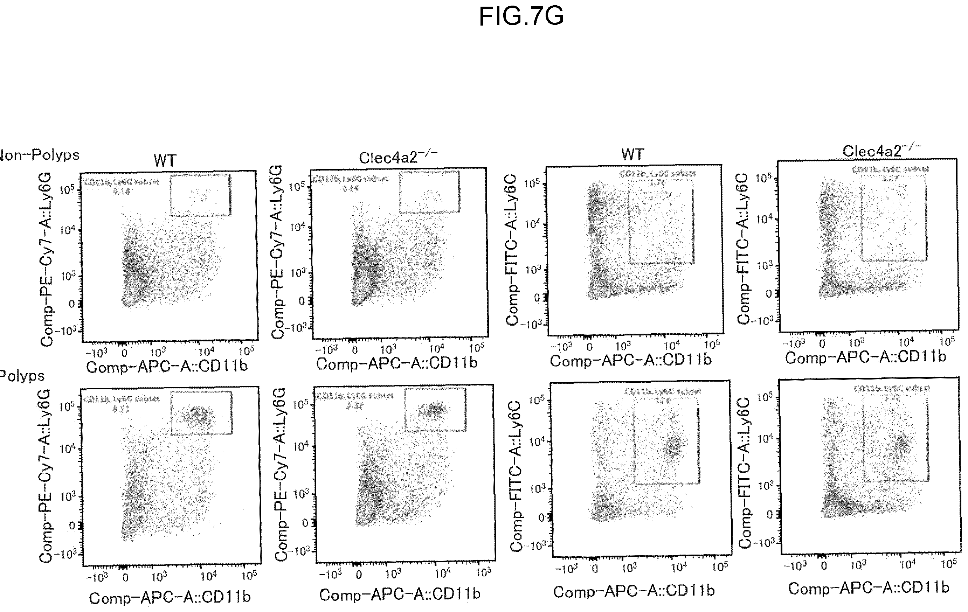

Next, gene expression in polyps and tissues around polyps was analyzed using real time RT-PCR and RNA-seq analysis. As a result, it was found that the expression of Il17a was drastically decreased in the polyps and non-polyp tissues in Clec4a2^{-/-} mice compared with WT mice (FIGs. 7B and 7E). Il17f expression was also decreased, although to the less extent compared to Il17a. These expression levels were also confirmed by qPCR (FIGs. 7C and 7E). The mRNA levels of chemokines including Cxcl2 were decreased in polyps and non-polyp tissues of Clec4a2^{-/-} mice (FIG. 7E). Reduced Cxcl2 expression was also confirmed by RT-PCR (FIG. 7D). Consistent with this observation, it was found that infiltration of CXCR2-expressing monocytic (CD11b+Ly6C+) and granulocytic (CD11b+Ly6G+) MDSCs in polyps was reduced in Clec4a2^{-/-} mice compared with WT mice after AOM-DSS treatment (FIGs. 7F and 7G). The expression of DCIR (Clec4a2) in polyps was significantly increased compared to surrounding normal tissues. The same was also observed in human colon cancer. The expression was increased in human inflammatory bowel disease similarly to mice. These results strongly suggest that DCIR is involved in the development of colitis and intestinal tumor in humans similarly to mice. Further, it was also found that Gzmb expression was decreased in polyps and non-polyp tissues of Clec4a2^{-/-} mice compared with WT mice (FIG. 7E). These results indicates that the expression of various cytokines and chemokines including Il17a and Cxcl2, which are important for inducing inflammation, is reduced in Clec4a2^{-/-} mice, which is associated with reduced recruitment of MDSCs to the polyps.

### 3.8 It was found that the GM-SCF-Stat5 axis is activated in Clec4a2^{-/-} mice to suppress development of colorectal cancer.

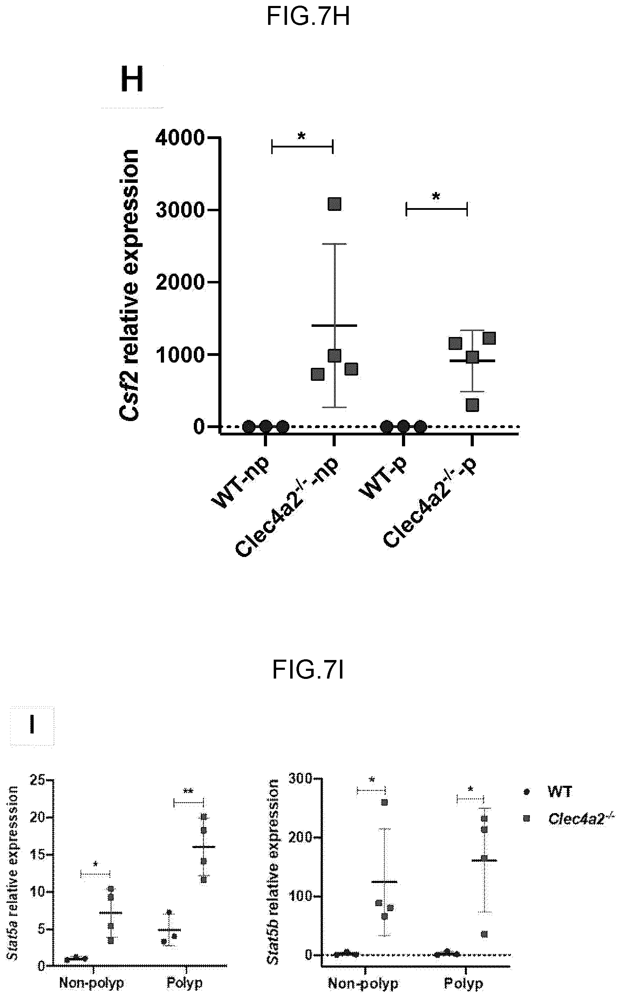

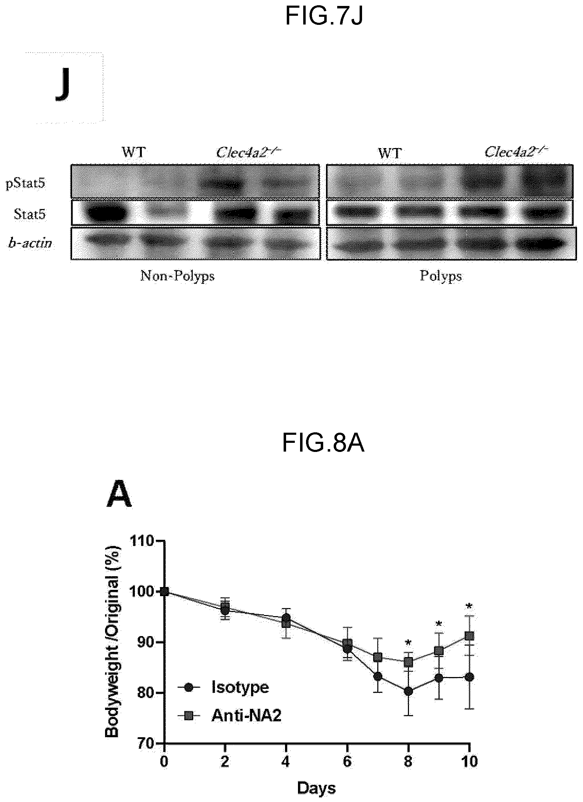

Further, it was found that the expression of Csf2 was 1000 times higher in polyps and non-polyp tissues of Clec4a2^{-/-} mice as compared to WT mice (FIG. 7H). The expression of GM-CSF signaling molecule StatS was also greatly increased in Clec4a2^{-/-} mice (FIG. 7I). Next, the activation status of this molecule was analyzed. As a result, it was found that phosphorylation of Stat5 was increased in polyps and non-polyp tissues of Clec4a2^{-/-} mice (FIG. 7J), suggesting that the GM-CSF-Stat5 axis is activated in the intestine of Clec4a2^{-/-} mice after induction of colorectal tumors.

### 3.9 An anti-NA2 antibody can suppress the development of colitis.

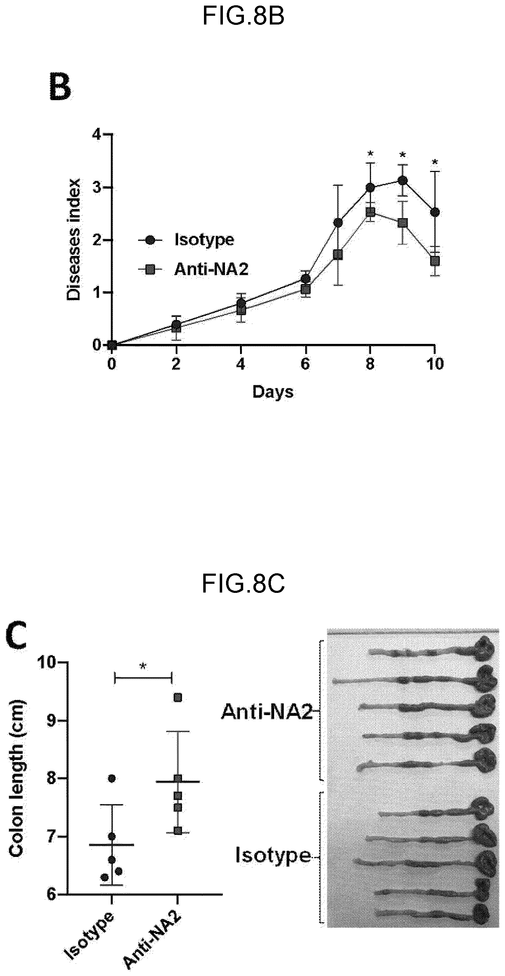

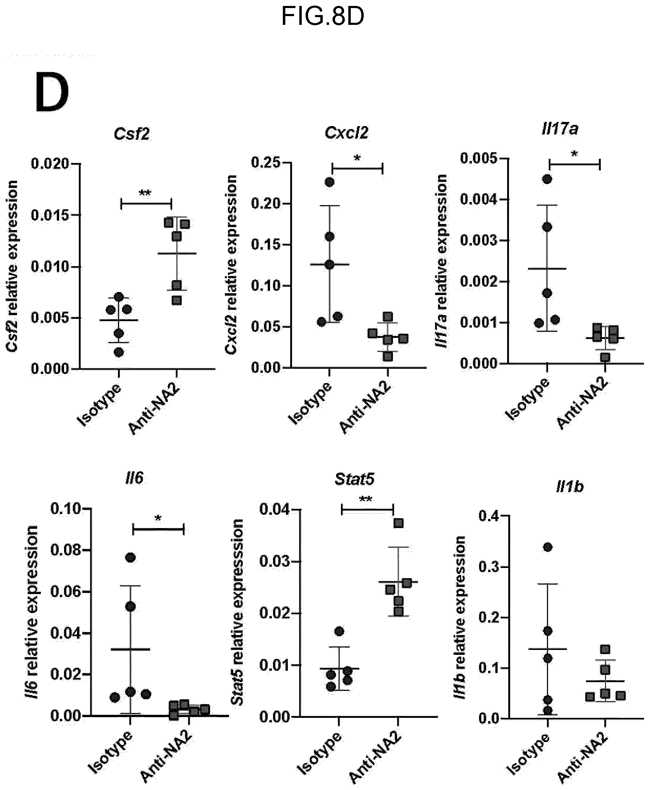

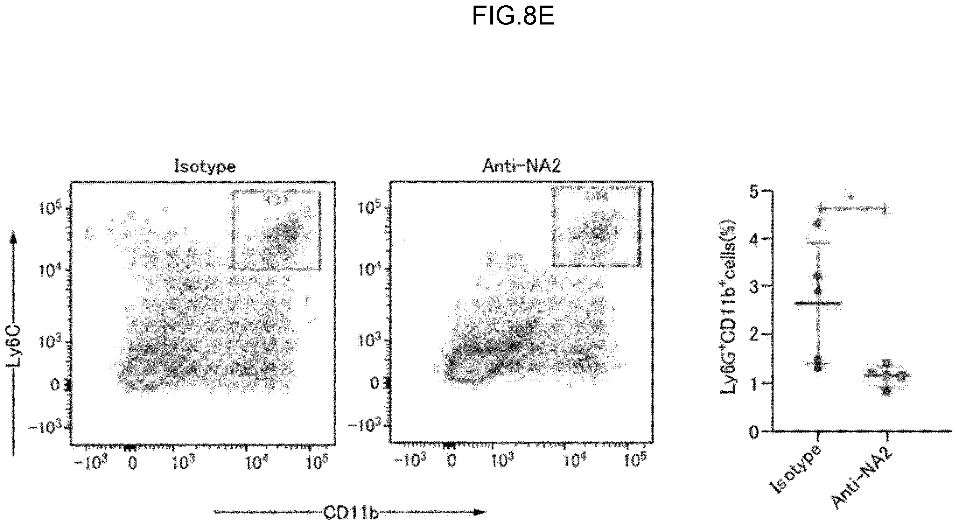

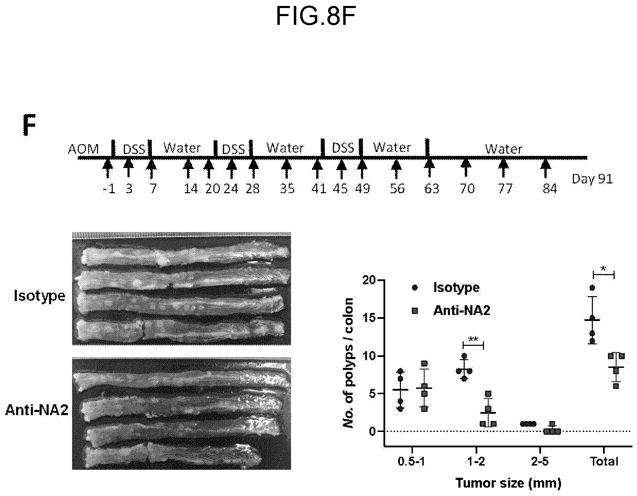

We showed that asialo-biantenary N-glycan (NA2) is a ligand for DCIR (Kaifu et al.), and generated an anitbody against NA2 by immunizing galactosyl transferase-1-deficient mice with NA2 [50]. Then, the effect of the anti-NA2 antibody on the development of DSS-induced colitis was examined. As a result, it was found that the development of DSS-induced colitis was suppressed in the anti-NA2 antibody-administration group compared with the isotype Ig-administration group. The anti-NA2 antibody-administration group showed less weight loss (FIG. 8A), lower disease severity (FIG. 8B) and longer colon length (FIG. 8C). Further, the administration of the antio-NA2 antibody suppressed the expression of Cxcl2, Il17a, and Il6, and enhanced Csf2 expression in the colon (FIG. 8D). Infiltration of Ly6G⁺ neutrophils into cLP was significantly reduced in the anti-NA2 antibody-treatment group compared with the isotype Ig-treatment group after 4% DSS treatment (FIG. 8E). Development of AOM-DSS induced polyps in the colon was significantly reduced in the anti-NA2 antibody treatment group compared with the Ig-treatment group (FIG. 8F). These observations show that an antibody that inhibits the interaction between NA2 and DCIR can suppress the development of DSS-induced colitis and colorectal tumor.

### 4. Discussion

In this study, the inventors have revealed that DCIR deficiency suppresses development of DSS-induced colitis and AOM-DSS-induced colorectal tumors. After oral administration of DSS, Clec4a2^{-/-} mice showed less weight loss, milder colitis severity and attenuated proinflammatory cell infiltration in the colon compared with WT mice. Further, Clec4a2^{-/-} mice developed less colonic tumors after AOM-DSS administration. Furthermore, an antibody against NA2, which is a DCIR ligand, was able to ameliorate colitis and tumor development induced by AOM-DSS. These results suggest that inhibition of DCIR signaling can prevent the development of colitis and colon tumor.

Commensal microbiota is suggested to play an important role for the development of colitis in mice and humans [17, 18]. However, cohousing Clec4a2^{-/-} mice with WT mice did not affect the sensitivity of Clec4a2^{-/-} mice to colitis, suggesting that intestinal microbiota is not involved in this phenotype. Tumor development after AOM-DSS treatment was also not affected by cohousing, indicating that the effect of DCIR-deficiency on tumorigenicity is also not mediated by microbiota.

The finding that Clec4a2^{-/-} mice are refractory to the development of DSS-induced colitis and AOM-DSS-induced tumor makes a clear contrast to the observations that the development of autoimmune diseases such as CIA and EAE is exacerbated in this mutant mouse [13, 15]. Development of autoimmune diseases is enhanced in Clec4a2^{-/-} mice because antigen-presenting ability is excessively enhanced in this mouse strain due to excessive expansion and enhanced maturation of DCs [13]. Thus, suppression of colitis development and colorectal tumors in Clec4a2^{-/-} mice suggests that T cell-mediated autoimmunity, which is dependent on the antigen presentation by DCs, is not involved in these phenotypes, but that innate immune responses that are independent of antigen presentation are involved therein. In support of this idea, Th1, Th2, and Treg cell populations were not changed in Clec4a2^{-/-} mice after the induction of DSS-induced colitis.

It was found that the expression of Csf2 is increased in Clec4a2^{-/-} mice as compared to WT mice after the DSS treatment. ILC3 cells are major GM-CSF producer cells after DSS treatment [44, 45]. Further, the proportion of GM-CSF⁺ cells in ILC3 cells is increased in Clec4a2^{-/-} mice as compared to WT mice after the DSS treatment. II,C3-driven GM-CSF production was dependent on the production of Il-1β by macrophages upon sensing TLR-mediated microbial signals, and GM-CSF^{-/-} altered mononuclear phagocyte effector functions and reduced Treg population and impaired oral tolerance [45]. Our data showed that the expression of Il1b is increased in CD11b⁺ cells of Clec4a2^{-/-} mice after stimulation with TLR8 and TLR9 agonists or DSS-induced colitis. This is consistent with previous reports that DCIR suppresses TLR8- and TLR9-mediated IL-1β production by suppressing the phosphorylation of Erk1/2, JNK1/2, and p38 in macrophages [46-47]. GM-CSF signaling is transduced to bone marrow cells through phosphorylation of Stat5 [13]. GM-CSF-STAT5 signaling is essential for the intestinal homeostatic response to intestinal injury, and knockout of StatS in intestinal epithelial cells increases NF-κB signaling and tight junction permeability [48]. StatS-deficient mice exhibit increased susceptibility to experimental colitis [20, 49]. Consistent with the increase in the GM-CSF expression in Clec4a2^{-/-} mice, StatS phosphorylation was enhanced in the colon of Clec4a2^{-/-} mice. In addition, it was shown that rmGM-CSF suppresses development of colitis, while an anti-GM-CSF aggravates colitis. This is consistent with the previous observations that GM-CSF deficiency aggravates development of colitis, and that the administration of GM-CSF improves colitis in mice [19-21] and in humans [22, 23]. Thus, it is concluded that overproduction of GM-CSF in the colon is responsible for the suppression of colitis in Clec4a2^{-/-} mice, suggesting a protective function of GM-CSF/Stat5 signaling in colitis.

Interestingly, it was found that the expression of junctional complex proteins Occludin, zo1, and Claudin was increased in Clec4a2 mice. Enterocyte Stat5 signaling protects against tight junction barrier dysfunction and promotes wound healing of the intestinal mucosa [48]. The expression of these genes was enhanced by mrGM-CSF treatment and decreased by anti-GM-CSF treatment, suggesting that the GM-CSF/Stat5 signaling pathway induces these tight junction proteins. As these proteins are important structural components of the tight junction, GM-CSF may play an important role to strengthen the epithelial barrier function of the intestine, in addition to the growth promotion and survival of the epithelial cells [23].

In contrast, the expression of inflammatory cytokine genes, such as Tnf, 111, and Il6, was suppressed in Clec4a2^{-/-} mice, suggesting that the amelioration of colitis is caused by the suppression of these inflammatory cytokine expression. It was shown that the expression of Il1b, Il6 Tnf, and Cxcl2 was suppressed by rmGM-CSF treatment, and enhanced by anti-GM-CSF treatment, indicating that the expression of these inflammatory cytokines is regulated by GM-CSF. Consistent with the inventors' results, it was reported that GM-CSF treatment downregulates the expression of proinflammatory cytokines such as Tnf, Il1a and Il1b [21]. Furthermore, it was found Il17a expression was also significantly suppressed in Clec4a2^{-/-} mice. This is because StatS, which suppresses Il17a expression by allowing Stat 3 to compete for the binding to the Il17a enhancer [24, 25], is excessively activated in Clec4a2^{-/-} mice.

The roles of IL-17A in the development of colitis is controversial. IL-17A is a proinflammatory cytokine and plays important roles in the development of autoimmune diseases such as EAE or CIA [26, 27]. IL-17A induces inflammation by recruiting neutrophils through induction of a chemokine CXCL2 [28, 29].

It was observed that the expression of Cxcl2 was decreased in Clec4a2^{-/-} mice, which was coordinated with the decreased Il17a expression. Increased expression of Il17a is observed in IBD [30], the expression of CXCL2 is correlated with the clinical activity of human UC [31], and excessive recruitment and accumulation of activated neutrophils are observed in association with mucosal injury in the intestine of IBD patients [32]. Further, blockade of its receptor CXCR2 can suppress DSS colitis in mice [33-35]. These observations may suggest that the reduced expression of IL-17A contributes to the reduction of colitis by reducing recruitment of neutrophils in Clec4a2^{-/-} mice. However, it was reported that treatment with an anti-IL-17A neutralizing antibody is ineffective and rather exacerbates the disease in IBD patients [36]. Development of colitis is also aggravated in Il17a^{-/-} mice in experimental colitis models, making a contrast to Il17f^{-/-} mice that are resistant to induced colitis [37-39]. These observations suggest that IL-17A plays rather protective roles in the development of colitis. In line with this notion, IL-17A was reported to play an important role in maintaining barrier integrity of the intestinal epithelium by regulating cellular localization of a tight junction protein Occludin in colonic epithelial cells [40]. Thus, even if the reduced expression of IL-17A in Clec4a2^{-/-} mice is involved in the suppression of colitis in these mice, it is unlikely that this is the main reason.

As a result, it was found that the development of colorectal tumor induced by AOM-DSS is suppressed in Clec4a2^{-/-} mice, similarly to the development of DSS-induced colitis. The expression of Csf2 was greatly enhanced in both polyps and non-polyp areas in the mutant mice compared with WT mice. Similar to the mice that developed colitis by DSS, the expression of Il17a, Il1b, Tnf, and Cxcl2 was reduced in Clec4a2^{-/-} mice. Since inflammation promotes tumorigenicity, augmented inflammation in Clec4a2^{-/-} mouse intestine may enhance tumorigenesis. In this context, IL-17A is known to enhance tumorigenesis by promoting angiogenesis [41]. IL-1 is also known to promote tumorigenesis [42]. Loss of CXCR2 dramatically suppresses colonic chronic inflammation and colitis-associated tumorigenesis through suppressing infiltration of MDSCs into colonic mucosa and tumors [34, 43]. Thus, reduced inflammatory cytokine production and MDSC infiltration explain why AOM-DSS-induced tumor development is suppressed in Clec4a2^{-/-} mice.

We showed that inhibition of the interaction between DCIR and its ligand NA2 using an anti-NA2 antibody can suppress the development of DSS-induced colitis and AOM-DSS-induced tumors. After anti-NA2 treatment, Csf2 expression was increased, and the expression of Il17a, IL6, IL1b, and Cxcl2 was reduced, which was consistent with the observations in Clec4a2^{-/-} mice. These results indicate that the NA2-DCIR axis plays an important role to regulate immune responses induced by innate immune receptors such as TLR8 and TLR9 in the intestine. Under physiological conditions, DCIR regulates excess differentiation and activation of DCs to prevent autoimmunity. However, in the intestine, DCIR signaling regulates innate immune signaling and suppresses induction of GM-CSF, which makes intestine vulnerable to inflammation and tumor development. Thus, inhibition of DCIR activation can suppress inflammation and tumor development in the intestine through innate immune mechanisms. In addition, since the structure of NA2 is the same in humans and mice, the foregoing findings are expected to be applicable to humans. These findings may provide us with a clue to develop novel therapeutics to treat colitis and colorectal tumors.

### [Example 2]

### 1. Introduction

An anti-DCIR antibody inhibits the binding between NA2 and DCIR, which may be applied to the treatment of inflammatory bowel disease and intestinal tumor. Therefore, an anti-DCIR antibody was prepared and evaluated for the binding property to DCIR and the ability to inhibit NA2-DCIR binding.

### 2. Material and methods

### 2.1 Preparation of the anti-DCIR antibody

An anti-DCIR antibody was established by the following procedure. A DCIR-expressing cell line was mixed with immunoadjuvant (Titer Max), DCIR-deficient mice were immunized twice, and the mice were sacrificed. Spleen cells were collected and fused with myeloma cells to prepare hybridoma cells, and a monoclonal library was prepared by limiting dilution. A DCIR-specific antibody was then identified based on the binding activity to DCIR-expressing cells and DCIR protein. The established monoclonal antibody-producing hybridoma was adapted to serum-free medium, and finally, the antibody was purified from the cell culture medium using Protein-L-associated beads.

RNA was purified from the monoclonal antibody-producing hybridoma cells, cDNA was prepared, and the DNA sequence of FR1 to FR4 was analyzed. The base sequence and the amino acid sequence of the produced anti-DCIR antagonist antibody are shown in FIGs. 14A and 14B.

### 2.2 Binding of the anti-DCIR antibody to mouse and human DCIRs

The anti-DCIR antibody or an isotype antibody (10 µg/ml) was added to the bottom of a 96-well plate and allowed to bind to the plate by standing overnight at 4°C. Blocking was performed with PBS containing 10% (w/v) FCS, and Fc, mouse DCIR-Fc, or human DCIR-Fc (5 µg/ml) was added. After allowing the plate to stand at room temperature for 1 hour and washing the plate, Fc, mouse DCIR-Fc or human DCIR-Fc associated with the plate was detected using Peroxidase-conjugated anti-human Fc (Jackson Immunoresearch) (FIG. 11).

### 2.3 Inhibition of binding between NA2-expressing cells (Lec2) and DCIR by the anti-DCIR antibody

DCIR-Fc, and an isotype antibody (5 µg/ml) or the anti-DCIR antibody (0 µg/ml, 1 µg/ml, 10 µg/ml) were added to Lec2 cells, which express NA2, and were suspended and allowed to stand on ice for 30 minutes. The cells were washed, and the binding of the DCIR-Fc protein to Lec2 cells was detected using a fluorescently labeled anti-human Fc antibody.

### 3. Results and Discussion

The produced anti-DCIR antibody showed a binding activity to the mouse and human DCIRs (FIG. 12). It was also confirmed that the prepared anti-DCIR antibody has the ability to inhibit the binding between the NA2-expressing cells (Lec2) and DCIR (FIG. 13). This antibody shows binding to human DCIR antibody and inhibits the binding between DCIR and NA2, suggesting the possibility that it may be effectively applied to new pharmaceuticals.

### [Example 3]

### Confirmation of the antagonist activity of anti-DCIR antibody

Bone marrow cells from wild type or DCIR-deficient mice were cultured with M-CSF (20 ng/mL) for 2 days, followed by M-CSF (20 ng/mL) and RANKL (100 ng/mL) for 4 days. An isotype or recombinant anti-DCIR antagonist antibody (5A3D8_recombinant) was added at 1 µg/mL at the start of the culture and at the time of changing the culture medium. After TRAP staining, TRAP-positive multinucleated cells with a diameter of 100 to 200 µm, and with a diameter of 200 µm or more, were counted as osteoclasts. The graph shows the average and standard deviation of the number of osteoclasts in four replicate wells, and the student's t-test was performed. *: p<0.05, iso vs anti-DCIR antibody. Data from one experiment performed are shown (FIGs. 15A, B).

As a result, the anti-DCIR antibody promoted osteoclast differentiation. In contrast to the anti-DCIR agonist antibody, this antibody promoted osteoclast differentiation, indicating that this antibody is not an agonist but an antagonist antibody.

### References

1. Maloy KJ, Powrie F. Intestinal homeostasis and its breakdown in inflammatory bowel disease. Nature 474, 298-306 (2011).
2. Kmiec Z, Cyman M, Slebioda TJ. Cells of the innate and adaptive immunity and their interactions in inflammatory bowel disease. Adv Med Sci. 62, 1-16 (2017).
3. Borregaard N. Neutrophils, from marrow to microbes. Immunity 33, 657-670 (2010).
4. Tang C et al. Myeloid C-type lectin receptors in skin/mucoepithelial diseases and tumors. J Leukoc Biol. 106, 903-917 (2019).
5. Shinobu Saijo et al. Dectin-1 is required for host defense against Pneumocystis carinii but not against Candida albicans. Nat Immunol. 8, 39-46(2007).
6. Ce Tang et al. Inhibition of Dectin-1 Signaling Ameliorates Colitis by Inducing Lactobacillus-Mediated Regulatory T Cell Expansion in the Intestine. Cell Host & Microbe 18, 183-197 (2015).
7. Saba K et al. A C-type lectin MGL1/CD301a plays an anti-inflammatory role in murine experimental colitis. Am J Pathol. 174, 144-152 (2009).
8. Huang, X. et al. Cloning and characterization of a novel ITIM containing lectin-like immunoreceptor LLIR and its two transmembrane region deletion variants. Biochem. Biophys. Res. Commun. 281, 131-140 (2001).
9. Kanazawa, N. et al. DCIR acts as an inhibitory receptor depending on its. immunoreceptor tyrosine-based inhibitory motif. J. Invest. Dermatol. 118, 261-266 (2002).
10. Bates, E.E. et al. APCs express DCIR, a novel C-type lectin surface receptor containing an immunoreceptor tyrosine-based inhibitory motif. J. Immunol. 163, 1973-1983 (1999).
11. Kaifu T et al. Dendritic Cell Immunoreceptor (DCIR): An ITIM-Harboring C-Type Lectin Receptor. C-Type Lectin Receptors in Immunity. 103-113 (2016).
12. Daigle, I. et al. Death receptors bind SHP-1 and block cytokine-induced anti-apoptotic signaling in neutrophils. Nat. Med. 8, 61-67 (2002).
13. Fujikado N et al. D et al. Dcir deficiency causes development of autoimmune diseases in mice due to excess expansion of dendritic cells. Nat. Med. 14, 176-180 (2008).
14. Maruhashi T. et al. DCIR Maintains Bone Homeostasis by Regulating IFN-g Production in T Cells. J. Immunol. 194, 5681-5691 (2015).
15. Akimasa Seno et al. Exacerbation of experimental autoimmune encephalomyelitis in mice deficient for DCIR, an inhibitory C-type lectin receptor. Exp. Anim. 64, 109-119 (2015).
16. M. Dougan et al. GM-CSF, IL-3, and IL-5 family of cytokines: Regulators of inflammation. Immunity 50, 796-811 (2019).
17. Mayne CG et al. Induced and natural regulatory T cells in the development of inflammatory bowel disease. Inflamm Bowel Dis. 19, 1772-1788 (2013).
18. Maul J et al. Peripheral and intestinal regulatory CD4+ CD25(high) T cells in inflammatory bowel disease. Gastroenterology 128, 1868-1878 (2005)
19. Xu Y et al. The role of granulocyte macrophage-colony stimulating factor in acute intestinal inflammation. Cell Res. 18, 1220-1229 (2008).
20. Han et al. Loss of GM-CSF signalling in non-haematopoietic cells increases NSAID ileal injury. Gut 59, 1066-1078 (2010).
21. Sainathan SK et al. Granulocyte macrophage colony-stimulating factor ameliorates DSS induced experimental colitis. Inflamm Bowel Dis. 14, 88-99 (2008).
22. J. R. Korzenik et al. Sargramostim in Crohn's Disease Study Group, Sargramostim for active Crohn's disease. N. Engl. J. Med. 352, 2193-22-1 (2005).
23. Daebritz J et al. Granulocyte macrophage colony-stimulating factor and the intestinal innate immune cell homeostasis in Crohn's disease. Am J Physiol Gstrointest Liver Physiol. 306, 455-465 (2014).
24. Yang et al. Opposing regulation of the locus encoding IL-17 through direct, reciprocal actions of STAT3 and STATS. Nature immunology 12, 247-254 (2011).
25. Arian Laurence et al. Interleukin-2 Signaling via STATS Constrains T Helper 17 Cell Generation. Immunity. 26, 371-381 (2007).
26. Komiyama Y et al. IL-17 Plays an Important Role in the Development of Experimental Autoimmune Encephalomyelitis. J Immunol. 177, 566-573 (2006).
27. Nakae S et al. IL-17 production from activated T cells is required for the spontaneous development of destructive arthritis in mice deficient in IL-1 receptor antagonist. PNAS. 100, 5986-5990 (2003).
28. Iwakura Y et al. Functional specialization of interleukin-17 family members. Immunity 34, 149-162 (2011).
29. Ye P et al. Requirement of interleukin 17 receptor signaling for lung CXC chemokine and granulocyte colony-stimulating factor expression, neutrophil recruitment, and host defense. J. Exp. Med. 194, 519-527 (2001).
30. Fujino S et al. Increased expression of interleukin 17 in inflammatory bowel disease. Gut 52, 65-70 (2013).
31. Paulo et al. Conserved transcriptomic profile between mouse and human colitis allows unsupervised patient stratification. Nature Communications 10, 2892 (2019).
32. Mieke et al. Neutrophil chemoattractant receptors in health and disease: double-edged swords. Cellular & Molecular Immunology 17, 433-450(2020).
33. Buanne P et al. Crucial pathophysiological role of CXCR2 in experimental ulcerative colitis in mice. J Leukoc Biol. 85, 1239-1246 (2007).
34. Fournier BM et al. The role of neutrophils during intestinal inflammation. Mucosal Immunol. 5, 354-366 (2012).
35. Koji et al. Microbiome-mediated neutrophil recruitment via CXCR2 and protection from amebic colitis. Plos Pathogens 13, e1006513 (2017).
36. Hueber W et al. Secukinumab, a human anti-IL-17A monoclonal antibody, for moderate to severe Crohn's disease: unexpected results of a randomised, double-blind placebo-controlled trial., Secukinumab in Crohn's Disease Study Group. Gut 61, 1693-1700 (2012).
37. Tang C et al. Suppression of IL-17F, but not of IL-17A, provides protection against colitis by inducing T reg cells through modification of the intestinal microbiota. Nat. Immunol. 19, 755-765 (2018).
38. O'Connor W et al. A protective function for interleukin 17A in T cell-mediated intestinal inflammation. Nat Immunol. 10, 603-609 2009.
39. Yang et al. Regulation of inflammatory responses by IL-17F. J Exp Med. 205, 1063-1075 (2008).
40. Lee et al. Interleukin-23-Independent IL-17 Production Regulates Intestinal Epithelial Permeability. Immunity 43, 727-738 (2015).
41. Zhao et al. The role of interleukin-17 in tumor development and progression. J Exp Med. 217, e20190297 (2020).
42. Krelin et al. Interleukin-1β-Driven Inflammation Promotes the Development and Invasiveness of Chemical Carcinogen-Induced Tumors. Cancer Res. 67, 1062-1071(2007).
43. H. Katoh et al. CXCR2-expressing myeloid-derived suppressor cells are essential to promote colitis-associated tumorigenesis. Cancer Cell 24, 631-644 (2013).
44. Tomas C.D et al. GM-CSF Calibrates Macrophage Defense and Wound Healing Programs during Intestinal Infection and Inflammation. Cell Report. 32, 107857 (2020).
45. Arthur M. et al. Microbiota-Dependent Crosstalk Between Macrophages and ILC3 Promotes Intestinal Homeostasis. Science 343, 1249288 (2014).
46. Zhao et al. DCIR negatively regulates CpG-ODN-induced IL-1β and IL-6 production. Mol Immunol. 68, 641-647 (2015).
47. Meyer-Wentrup, F. et al. DCIR is endocytosed into human dendritic cells and inhibits TLR8-mediated cytokine production. J. Leukoc. Biol. 85, 518-525 (2009).
48. Shila G. et al. Enterocyte STATS promotes mucosal wound healing via suppression of myosin light chain kinase-mediated loss of barrier function and inflammation. EMBO Mol Med. 4, 109-124 (2011).
49. Han et al. Signal Transducer and Activator of Transcription 5b Promotes Mucosal Tolerance in Pediatric Crohn's Disease and Murine Colitis. Am J Pathol. 169, 1999-2013 (2006).
50. Kaifu, T. *, Yabe, R. *, Maruhashi, T. *, Chung, S.-H.*, Tateno, H., Fujikado, N., Hirabayashi, J., and Iwakura, Y. DCIR and its ligand asialo-biantennary N-glycan regulate DC function and osteoclastogenesis. J. Exp. Med., 218, e20210435 (2021).

The disclosure of Japanese Patent Application No. 2022-008129 is incorporated herein by reference in its entirety. All literatures, patent applications, and technical standards described in the present disclosure are incorporated herein by reference to the same extent as a case in which the literatures, the patent applications, and the technical standards were specifically and individually indicated to be incorporated by reference.

## Claims

1. A pharmaceutical composition for treating or preventing at least one disease selected from the group consisting of inflammatory bowel disease and intestinal tumor, the pharmaceutical composition comprising a dendritic cell immunoreceptor inhibitor or a binding inhibitor between a dendritic cell immunoreceptor and an asialo-biantennary N-glycan, and a pharmaceutically acceptable carrier.

2. The pharmaceutical composition according to claim 1, wherein the dendritic cell immunoreceptor inhibitor or the binding inhibitor between a dendritic cell immunoreceptor and an asialo-biantennary N-glycan is an antibody.

3. The pharmaceutical composition according to claim 1 or 2, wherein the binding inhibitor between a dendritic cell immunoreceptor and an asialo-biantennary N-glycan is an anti-asialo-biantennary N-glycan antibody.

4. The pharmaceutical composition according to claim 1 or 2, wherein the dendritic cell immunoreceptor inhibitor or the binding inhibitor between a dendritic cell immunoreceptor and an asialo-biantennary N-glycan is an anti-dendritic cell immunoreceptor antibody.

5. The pharmaceutical composition according to claim 2, wherein the antibody is a mouse antibody, a chimeric antibody, a humanized antibody, or a human antibody.

6. The pharmaceutical composition according to claim 2, wherein the antibody is a humanized antibody.

7. The pharmaceutical composition according to claim 2, wherein the antibody is a full-length antibody, or a low-molecular antibody selected from the group consisting of Fab, Fab', F (ab')2, scFv, and diabody.

8. The pharmaceutical composition according to any one of claims 1 to 7, wherein the intestinal tumor comprises at least one selected from the group consisting of colorectal cancer, rectal cancer, and colon cancer.

9. The pharmaceutical composition according to any one of claims 1 to 8, wherein the inflammatory bowel disease comprises at least one selected from the group consisting of Crohn's disease and ulcerative colitis.
